# EUROPEAN PATENT APPLICATION

(11) **EP 4 295 844 A2**
(43) Date of publication of application: **27.12.2023**
(21) Application number: 22750091.5
(22) Date of filing: 08.02.2022
(51) Int. Cl.: A61K 31/437, A61K 31/18, A61P 35/04, G01N 33/68

(54) **PHARMACEUTICAL COMPOSITION FOR INHIBITING CANCER METASTASIS**

(30) Priority: 08.02.2021 KR 20210017485
(71) Applicant: Industry-Academic Cooperation Foundation, Yonsei University, Seoul 03722 (KR)
(72) Inventor: KIM, Hyun Seok, Goyang-si Gyeonggi-do 10361 (KR); HAN, Jang Hee, Seoul 04129 (KR)
(74) Representative: Müller, Christian Stefan Gerd
(86) International application number: PCT/KR2022/001890
(87) International publication number: WO 2022/169342

(57) **Abstract**

The present invention relates to a pharmaceutical composition capable of effectively inhibiting the metastasis of cancer, particularly cancer in which autophagy is activated.

## Description

### TECHNICAL FIELD

The present invention relates to a pharmaceutical composition for inhibiting metastasis of cancer.

### BACKGROUND OF THE INVENTION

Lung cancer is the second most common cancer among both sexes, and accounts for 15% of all cancers. According to a report by the American Cancer Society in 2011, more than 220,000 cases of lung cancer are diagnosed annually, and about 70% of such cases result in death, accounting for 27% of all cancer deaths. Among lung cancer types, non-small lung cancer is a type of carcinoma and refers to all epithelial lung cancers except small lung cancer, which accounts for about 85% to 90% of all lung cancers. Non-small lung cancer is relatively less sensitive to chemotherapy than small lung cancer, and cancer stages are divided based on the TNM classification: the size of the tumor, degree of cancer spread to regional lymph nodes, and the presence or absence of cancer metastasis. In the treatment of non-small lung cancer, surgery is usually performed with adjuvant chemotherapy involving cisplatin containing platinum because early non-metastatic non-small lung cancer has very low sensitivity to chemotherapy and radiation. On the other hand, in the case of metastatic non-small lung cancer past the early stage, various chemotherapy and radiation treatments are used. Symptoms of non-small lung cancer include persistent coughs, chest pain, weight loss, nail damage, joint pain, and shortness of breath, but there are few symptoms in the early stages because non-small lung cancer usually progresses slowly. Therefore, early detection and treatment of non-small lung cancer is difficult, and it is highly likely to be detected after metastasis throughout the body, such as to the bone, liver, small intestine and brain. Despite the high incidence and mortality rates, no drug or treatment method capable of overcoming non-small lung cancer has yet been developed, and therefore the need for it has emerged. Non-small lung cancer is divided into several subtypes according to the size, shape, and chemical composition of the cancer cells and representative examples include adenocarcinoma, squamous cell carcinoma, and large cell carcinoma. Adenocarcinoma is the most frequently occurring type of lung cancer, accounting for more than 40% of all lung cancers, is found in the outer region of the lung, and tends to progress more slowly than other lung cancers, but shows a high metastatic tendency in the early stages and a high radiation resistance. Squamous cell carcinoma is a type of non-small lung cancer that accounts for 25-30% of all lung cancer, and begins in the early version of cells constituting the airway, and shows a high incidence rate mainly in smokers. In addition, large cell carcinoma, which accounts for about 10-15% of all lung cancers, can develop in any part of the lung, and its progression is as fast as that of small cell lung cancer, so its treatment is still emerging as a challenge.

One of the most important biological properties of cancer is its ability to metastasize, which is considered one of the biggest obstacles to cancer treatment. In fact, about 60% of all solid tumor patients already have cancer that has clinically metastasized, albeit microscopically, at the time of diagnosis of the primary tumor, and it is already well known that metastasis is an important cause of death for most cancer patients.

In the process of metastasis, angiogenesis is a phenomenon that is accompanied by the invasion of local tissues by cancer, which involves tumor angiogenic factors, and since new blood vessels created by tumors are often defective, they are easily invaded by cancer cells. In addition, in the process of invasion and metastasis of cancer, numerous enzymes, growth factors, autocrine motility factors, and cancer genes necessary for dissolving the stroma of normal tissues, such as receptors on the surface of cancer cells, such as laminin receptors required for adhesion to tissue matrix and basement membrane, type IV collagenase, plasminogen activator, cathepsin D, and the like need to be expressed.

Currently, expectations for the development substances with cancer metastasis inhibitory action are very high, but there are very few examples of substances actually developed for the purpose of suppressing cancer metastasis. Sulfated polysaccharides, N-diazoacetylglycine derivatives, noiraminitase, and fibronectin degrading enzyme (FNS) have been reported to have such inhibitory effects, but there is no report of their practical uses yet, and they themselves have not reported an inhibitory effect on cancer metastasis. Therefore, if a method capable of effectively inhibiting cancer metastasis is developed, it is possible to develop a useful treatment method capable of effectively controlling death due to cancer metastasis.

### DISCLOSURE OF THE INVENTION

### TECHNICAL PROBLEM

An object of the present invention is to provide a pharmaceutical composition capable of inhibiting metastasis in cancer, preferably cancer in which autophagy is activated.

Another object of the present invention is to provide a method for screening metastasis inhibitors of cancer, preferably cancer in which autophagy is activated.

Another object of the present invention is to provide a method for providing information for predicting the therapeutic response of a metastasis inhibitor of cancer, preferably cancer in which autophagy is activated.

However, the technical problem to be achieved by the present invention is not limited to the above-mentioned problems, and other problems not mentioned will be clearly understood by those skilled in the art from the following detailed description.

### TECHNICAL SOLUTION

According to one embodiment of the present invention, the present invention relates to a pharmaceutical composition for inhibiting metastasis of cancer comprising at least one inhibitor of the activity or expression of a protein selected from calcium/calmodulin-dependent protein kinase kinase 2 (CAMKK2) and ATP citrate lyase (ACLY), or an expression inhibitor of the gene encoding the protein as an active ingredient.

In the present invention, the CAMKK2 protein may consist of amino acids represented by SEQ ID NO: 1, but is not limited thereto.

In the present invention, the ACLY protein may consist of amino acids represented by SEQ ID NO: 2, but is not limited thereto.

In the present invention the CAMKK2 or ACLY protein activity or expression inhibitor may comprise any one or more selected from the group consisting of compounds, peptides, peptide mimetics, aptamers, antibodies, and natural products that specifically bind to the protein.

The "peptide mimetics" as used herein refers to peptides or non-peptides that inhibit the protein. It can be created by using the β-turn dipeptide core (Nagai et al. Tetrahedron Lett 26:647, 1985), keto-methylene pseudopeptides, azepines, benzodiazepines, β-aminoalcohols and substituted gamma-lactam rings as major residues of non-hydrolyzable peptide analogs.

The "aptamer" as used herein refers to a single-stranded nucleic acid (DNA, RNA, or modified nucleic acid) that has a stable tertiary structure as it is and can bind to target molecules with high affinity and specificity. Since the first development of aptamer discovery technology called SELEX (Systematic Evolution of Ligands by Exponential Enrichment) (Ellington, AD and Szostak, JW., Nature 346:818-822, 1990), many aptamers that can bind to various target molecules, such as small-molecular organic substances, peptides and membrane proteins, have been continuously discovered. Aptamers are comparable to single antibodies because of their inherent high affinity (usually pM level) and ability to specifically bind to target molecules, and in particular, they have high potential as alternative antibodies to the extent that they are called "chemical antibodies".

The "antibody" as used herein can be either manufactured through protein injection or purchased commercially. In addition, the antibody comprises polyclonal antibodies, monoclonal antibodies and epitope-binding fragments, and the like. Here, the polyclonal antibody may be produced by a conventional method of injecting a protein into an animal and collecting blood from the animal to obtain antibody-containing serum. Such polyclonal antibodies can be purified by any method known in the art, and can be made from any animal species host, such as goat, rabbit, sheep, monkey, horse, pig, cow, dog, and the like. In addition, the monoclonal antibody can be produced using any technique that provides the production of antibody molecules through the cultivation of continuous cell lines. Such technologies include, but are not limited to, hybridoma technology, human B-cell line hybridoma technology, and EBV-hybridoma technology.

In addition, in the present invention, an antibody fragment comprising a specific binding site for the protein can be prepared. For example, but not limited to, F(ab')2 fragments can be prepared by pepsin digestion of antibody molecules, and Fab fragments can be prepared by reducing disulfide bridges of F(ab')2 fragments. Alternatively, by miniaturizing the Fab expression library, monoclonal Fab fragments with the desired specificity can be quickly and conveniently identified.

In the present invention, the antibody may be bound to a solid substrate to facilitate subsequent steps such as washing or separation of complexes. Solid substrates include, for example, synthetic resins, nitrocellulose, glass substrates, metal substrates, glass fibers, microspheres and microbeads. In addition, the synthetic resin includes polyester, polyvinyl chloride, polystyrene, polypropylene, PVDF, and nylon.

As an example of the present invention, the activity or expression inhibitor of the CAMKK2 protein may be STO-609, SGC-CAMKK2-1, KN62, KN93, AlP, ACS-I, and berbamine, but is not limited thereto.

As an example of the present invention, the activity or expression inhibitor of the ACLY protein may be BMS303141, NDI-091143, SB-204990, ETC-1002, radicicol, (-)-hydroxycitric acid and 2-chloro-1,3,8-trihydroxy-6-methylanthrone, and the like, but are not limited thereto.

In the present invention, expression inhibitors of the gene encoding CAMKK2 or ACLY may comprise any one or more selected from the group consisting of antisense nucleotides, short interfering RNA (siRNA), short hairpin RNA (shRNA) and ribozymes that complementarily bind to the gene.

The "antisense nucleotide" as used herein, as defined in Watson-Crick base pairing, binds (hybridizes) to a complementary nucleotide sequence of DNA, immature-mRNA or mature mRNA to interfere with the flow of genetic information from DNA to protein. The nature of specificity of antisense nucleotides to their target sequences makes them exceptionally multifunctional. Since antisense nucleotides are long chains of monomeric units, they can be easily synthesized against the target RNA sequence. Recently, many studies have demonstrated the usefulness of antisense nucleotides as a biochemical means to study target proteins. Since many recent advances have been made in the field of oligonucleotide chemistry and nucleotide synthesis exhibiting improved cell line adsorption, target binding affinity and nuclease resistance, the use of antisense nucleotides can be considered as a new type of inhibitor.

The "siRNA" and "shRNA" as used herein refer to nucleic acid molecules capable of mediating RNA interference or gene silencing, and can inhibit the expression of a target gene, thereby providing an efficient gene knockdown method or gene therapy. shRNA is a hairpin structure formed by binding between complementary sequences within a single-stranded oligonucleotide, and *in vivo,* the shRNA is cleaved by dicer into small RNA fragments of 21 to 25 nucleotides in size to become double-stranded oligonucleotide siRNA, and can specifically bind to mRNA with a complementary sequence and suppress its expression. Therefore, which means of shRNA and siRNA to use can be determined by a person skilled in the art, and if the mRNA sequences they target are the same, similar expression reduction effects can be expected. For the purpose of the present invention, by specifically acting on the gene encoding the CAMKK2 or ACLY protein to cleave these genes (eg. mRNA molecules) to induce RNA interference (RNAi), the CAMKK2 or ACLY protein can be suppressed. siRNAs can be synthesized chemically or enzymatically. The method for preparing siRNA is not particularly limited, and methods known in the art may be used. Methods include, for example, a method for chemically synthesizing siRNA directly, a method for synthesizing siRNA using in vitro transcription, a method for cutting long double-stranded RNA synthesized by in vitro transcription using an enzyme, expression method through intracellular delivery of shRNA expression plasmid or viral vector, and expression method through intracellular delivery of PCR (polymerase chain reaction)-induced siRNA expression cassette, but is not limited thereto.

The "ribozyme" as used herein refers to an RNA molecule having a catalytic activity. Ribozymes with various activities are known, and ribozymes of the CAMKK2 or ACLY gene comprise known or artificially produced ribozymes, and ribozymes having target-specific RNA cleavage activity can be selectively prepared by known standard techniques.

In the present invention, the cancer is a cancer in which autophagy, preferably macroautophagy, is activated, and may comprise at least one of the KRAS gene and the LKB1 gene, preferably the KRAS gene, and more preferably the KRAS gene and mutations in both the LKB1 gene.

In the present invention, acetylation of Snail (zinc finger protein SNAI1) transcription factor increases in cancers in which the autophagy is activated, and as a result, Snail is stabilized, so that cancer cells are converted via epithelial-mesenchymal transition (EMT) and cancer metastasis is promoted. More specifically, upon activation of autophagy, CAMKK2 promotes the supply of citrate necessary for acetyl-CoA synthesis, and ACLY catalyzes the synthesis of acetyl-CoA from the supplied citrate. The increased acetyl-CoA binds to Snail by the CBP/p300 enzyme, resulting in increased acetylation of Snail. Therefore, in the present invention, by using an activity or expression inhibitor of the CAMKK2 or ACLY protein or an expression inhibitor of the gene encoding the protein, the deacetylation of the Snail is increased and the Snail is destabilized, thereby preventing conversion to the EMT subtype and inhibiting cancer metastasis.

The "autophagy" as used herein refers to a destructive mechanism that naturally degrades unnecessary or nonfunctional cellular components in the regulatory process, and while, in the early stages of cancer, mutations, cell damage, and various stresses are relatively low so that autophagy removes carcinogenic factors and contributes as a tumor suppressor, in the late stages of cancer progression, already formulated tumors become malignant and in the process of becoming a cancer with the ability to metastasize, rapid cell division, cell growth, and angiogenesis reactions are maximized, requiring a large amount of energy supply, during which autophagy contributes greatly as a tumor[ promoter by helping cancer cells acquire building blocks for synthesizing ATP and biomolecules (Kimmelman, Alec C., and Eileen White. Cell metabolism 25.5 (2017): 1037-1043).

The "macroautophagy" as used herein refers to a type of autophagy, and cells form double-membrane vesicles, that is, "autophagosomes", around the cytoplasm. These autophagosomes eventually fuse with lysosomes to degrade substances contained therein, such as unnecessary or damaged organelles, cytoplasmic proteins, and invasive microorganisms. These decomposed substances are released back into the cytoplasm to produce energy or protect cells to maintain cell viability even under stressful conditions. On the other hand, when these autophagosomes mature, they fuse with lysosomes to form autophagolysosomes and decompose the components captured in an acidic environment mediated by acid hydrolases.

The "mutation" as used herein refers to a permanent change in the nucleotide sequence of an existing gene, and may include, without limitation, changes in chromosome structure or number, or changes in one or several nucleotides. As a specific example, the mutation may be one or more selected from deletion, duplication, inversion, translocation, base substitution, insertion, and fusion, but is not limited thereto.

In the present invention, the cancer may be breast cancer, ovarian cancer, colon cancer, stomach cancer, liver cancer, pancreatic cancer, cervical cancer, thyroid cancer, parathyroid cancer, lung cancer, non-small cell lung cancer, prostate cancer, gallbladder cancer, biliary tract cancer, non-Hodgkin's lymphoma, Hodgkin's lymphoma, blood cancer, bladder cancer, kidney cancer, melanoma, colon cancer, bone cancer, skin cancer, head cancer, uterine cancer, rectal cancer, brain tumor, perianal cancer, fallopian tube carcinoma, endometrial carcinoma, vaginal cancer, vulvar carcinoma, esophageal cancer, small intestine cancer, endocrine adenocarcinoma, adrenal cancer, soft tissue sarcoma, urethral cancer, penile cancer, ureteral cancer, renal cell carcinoma, renal pelvic carcinoma, central nervous system (CNS) tumor, primary CNS lymphoma, spinal cord tumor, brainstem glioma or pituitary adenoma, and may preferably be, lung cancer or pancreatic cancer, but is not limited thereto.

The "metastasis" as used herein refers to a state in which a malignant tumor has spread to other tissues away from an organ where it has developed. As a malignant tumor that started in one organ progresses, it spreads from that organ, which is the primary site where it first developed, to other tissues, and the spreading from the primary site to other tissues can be referred to as metastasis. Metastasis can be said to be a phenomenon accompanying the progression of malignant tumors, and metastasis can occur while acquiring new genetic traits as malignant tumor cells proliferate and cancer progresses. Metastasis can occur when tumor cells that have acquired new genetic traits invade blood vessels and lymph glands, circulate along the blood and lymph, and settle and proliferate in other tissues. Depending on the tissue in which metastasis occurs, various cancer diseases such as liver cancer, kidney cancer, lung cancer, stomach cancer, colon cancer, rectal cancer, and pancreatic cancer can be induced. The composition of the present invention can prevent and treat the spread of cancer by inhibiting metastasis.

The pharmaceutical composition of the present invention may be in the form of capsules, tablets, granules, injections, ointments, powders or beverages, and the pharmaceutical composition may be intended for humans.

The pharmaceutical composition of the present invention is not limited, but may be formulated in the form of oral formulations such as powders, granules, capsules, tablets, aqueous suspensions, external preparations, suppositories and sterile injection solutions according to conventional methods. The pharmaceutical composition of the present invention may comprise a pharmaceutically acceptable carrier. Pharmaceutically acceptable carriers may include binders, lubricants, disintegrants, excipients, solubilizers, dispersants, stabilizers, suspending agents, pigments, flavors, and the like for oral administration, may be mixed with buffers, preservatives, analgesics, solubilizers, isotonic agents, stabilizers and the like in the case of injections, and may include bases, excipients, lubricants, preservatives and the like for topical administration. Formulations of the pharmaceutical composition of the present invention may be variously prepared by mixing with the pharmaceutically acceptable carrier as described above. For example, for oral administration, it can be prepared in the form of tablets, troches, capsules, elixirs, suspensions, syrups, wafer and the like, and in the case of injections, it can be prepared in unit dosage ampoules or multiple dosage forms. In addition, it may be formulated into solutions, suspensions, tablets, capsules, sustained-release preparations, and the like.

On the other hand, examples of carriers, excipients and diluents suitable for formulation include lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinylpyrrolidone, Water, methylhydroxybenzoate, propylhydroxybenzoate, talc, magnesium stearate or mineral oil. In addition, fillers, anti-agglomerating agents, lubricants, wetting agents, flavoring agents, emulsifiers, preservatives, and the like may be further comprised.

The route of administration of the pharmaceutical composition of the present invention comprise, but are not limited to, oral, intravenous, intramuscular, intraarterial, intramedullary, intrathecal, intracardiac, transdermal, subcutaneous, intraperitoneal, intranasal, intestinal, topical, sublingual or rectal. Oral or parenteral administration is preferred.

The parenteral methods of the present invention comprise subcutaneous, intradermal, intravenous, intramuscular, intraarticular, intrasynovial, intrasternal, intrathecal, intralesional and intracranial injection or infusion techniques. The pharmaceutical composition of the present invention may also be administered in the form of a suppository for rectal administration.

The pharmaceutical composition of the present invention can vary widely depending on various factors including the activity of the specific compound used, age, body weight, general health, sex, diet, administration time, route of administration, excretion rate, drug combination and severity of the specific disease to be prevented or treated, and the dosage of the pharmaceutical composition varies depending on the patient's condition, body weight, disease severity, drug type, administration route and period, but may be appropriately selected by those skilled in the art, and can be administered at 0.0001 to 50 mg/kg or 0.001 to 50 mg/kg per day. Administration may be administered once a day, or may be administered in several divided doses. The above dosage does not limit the scope of the present invention in any way. The pharmaceutical composition according to the present invention may be formulated into a pill, dragee, capsule, liquid, gel, syrup, slurry, or suspension.

According to another embodiment of the present invention, it relates to a method for screening cancer metastasis inhibitors comprising the step of processing a candidate substance with respect to the separated biological sample; and
the step of measuring the activity or expression level of at least one protein of CAMKK2 and ACLY in the biological sample after treatment with the candidate substance or measuring the expression level of a gene encoding the protein.

The "screening" as used herein refers to the selection of a substance having a specific target property from a candidate group composed of various substances by a specific manipulation or evaluation method.

In the present invention, the isolated biological sample may be a biological sample isolated from a subject with or without cancer. Specifically, the biological sample may comprise whole blood, leukocytes, peripheral blood mononuclear cells, buffy coat, plasma, serum, sputum, tears, mucus, nasal washes, nasal aspirate, breath, urine, semen, saliva, peritoneal washings, pelvic fluids, cystic fluid, meningeal fluid, amniotic fluid, glandular fluid, pancreatic fluid, lymph fluid, pleural fluid, nipple aspirate, bronchial aspirate, synovial fluid, joint aspirate, organ secretions, cells, cell extracts, or cerebrospinal fluids, but are not limited thereto.

In the present invention, the candidate substance may be at least one selected from the group consisting of natural compounds, synthetic compounds, RNA, DNA, polypeptides, enzymes, proteins, ligands, antibodies, antigens, bacterial or fungal metabolites, and bioactive molecules, but is not limited thereto.

In the present invention, the agent for measuring the activity or expression level of the CAMKK2 or ACLY protein is not particularly limited, but may comprise for example, at least one selected from the group consisting of an antibody, an oligopeptide, a ligand, a peptide nucleic acid (PNA), and an aptamer that binds specifically to the CAMKK2 or ACLY.

The "antibody" as used herein refers to a substance that specifically binds to an antigen and causes an antigen-antibody reaction. For the purposes of the present invention, an antibody refers to an antibody that specifically binds to the biomarker protein. Antibodies of the present invention comprise polyclonal antibodies, monoclonal antibodies and recombinant antibodies. Such antibodies can be readily prepared using techniques well known in the art. For example, polyclonal antibodies can be produced by a method well known in the art, which includes a process of injecting an antigen of the biomarker protein into an animal, collecting blood from the animal, and obtaining serum containing the antibody. Such monoclonal antibodies can be prepared from any animal such as goat, rabbit, sheep, monkey, horse, pig, cow, dog, and the like. In addition, monoclonal antibodies can be prepared using the hybridoma method (see Kohler and Milstein (1976) European Journal of Immunology 6:511-519), or the phage antibody library technique (see Clackson et al, Nature, 352:624-628, 1991; Marks et al, J. Mol. Biol., 222:58, 1-597, 1991) well known in the art. The antibody prepared by the above method may be separated and purified using methods such as gel electrophoresis, dialysis, salt precipitation, ion exchange chromatography, and affinity chromatography. In addition, the antibodies of the present invention include functional fragments of antibody molecules as well as complete forms having two full-length light chains and two full-length heavy chains. A functional fragment of an antibody molecule means a fragment having at least an antigen-binding function, and includes Fab, F(ab'), F(ab')2, and Fv.

The "PNA (peptide nucleic acid)" as used herein refers to an artificially synthesized polymer similar to DNA or RNA, and was first introduced in 1991 by Professors Nielsen, Egholm, Berg and Buchardt of the University of Copenhagen, Denmark. Whereas DNA has a phosphate-ribose backbone, PNA has a repeated N-(2-aminoethyl)-glycine backbone linked by peptide bonds, which greatly increases the binding force and stability to DNA or RNA, and is thus used in molecular biology, diagnostic assays and antisense therapies. PNA is described in detail in reference [Nielsen PE, Egholm M, Berg RH, Buchardt O (December 1991). "Sequence-selective recognition of DNA by strand displacement with a thymine-substituted polyamide". Science 254 (5037): 1497-1500].

The "aptamer" as used herein refers to an oligonucleic acid or peptide molecule, and a general description of the aptamer is described in reference [Bock LC et al., Nature 355(6360):5646(1992); Hoppe-Seyler F, Butz K "Peptide aptamers: powerful new tools for molecular medicine". J Mol Med. 78(8):42630(2000); Cohen BA, Colas P, Brent R. "An artificial cell-cycle inhibitor isolated from a combinatorial library". Proc Natl Acad Sci USA. 95(24): 142727(1998)].

In the present invention, methods for measuring or analytically comparing the activity or expression level of the protein comprise protein chip analysis, immunoassay, ligand binding assay, MALDI-TOF (Matrix Assisted Laser Desorption/lonization Time of Flight Mass Spectrometry) analysis, SELDI-TOF (Surface Enhanced Laser Desorption/lonization Time of Flight Mass Spectrometry) analysis, radiation immunity assay, radioimmuno-diffusion method, Oukteroni immunodiffusion method, rocket immunoelectrophoresis, tissue immunostaining, complement fixation assay, two-dimensional electrophoretic assay, Liquid Chromatography-Mass Spectrometry (LC-MS), Liquid Chromatography-Mass Spectrometry/ Mass Spectrometry (LC-MS/MS), Western blotting or ELISA (enzyme linked immunosorbentassay), but are limited thereto.

In the present invention, the agent for measuring the expression level of the gene encoding CAMKK2 or ACLY may comprise at least one selected from the group consisting of primers, probes, and antisense nucleotides that specifically bind to the gene encoding CAMKK2 or ACLY.

The "primer" used herein refers to a fragment that recognizes a target gene sequence, and comprises a forward and reverse primer pair, but is preferably a primer pair that provides an analysis result having specificity and sensitivity. High specificity can be imparted when the primers amplify the target gene sequence containing complementary primary binding sites while not causing non-specific amplification because the nucleic acid sequence of the primer is inconsistent with the non-target sequence present in the sample.

In the present invention, the "probe" refers to a substance that can specifically bind to a target substance to be detected in a sample, and a substance that can specifically confirm the presence of a target substance in a sample through binding. Given probes are commonly used in the art, the type of probe is not limited, but preferably can be peptide nucleic acid (PNA), locked nucleic acid (LNA), peptide, polypeptide, protein, RNA, or DNA, with PNA being the most preferred. More specifically, the probe is a biomaterial that includes those derived from or similar to those derived from living organisms or those manufactured *in vitro,* for example, enzymes, proteins, antibodies, microorganisms, animal and plant cells and organs, nerve cells, DNA, and RNA. DNA may comprise cDNA, genomic DNA and oligonucleotides, while RNA may comprise genomic RNA, mRNA, oligonucleotides, while proteins may comprise antibodies, antigens, enzymes, peptides, and the like.

In the present invention, the "LNA (locked nucleic acids)" refers to a nucleic acid analog comprising a 2'-O, 4'-C methylene bridge. LNA nucleosides comprise the common nucleic acid bases of DNA and RNA, and can base pair according to the Watson-Crick base pairing rules. However, because of the locking of molecules due to the methylene bridge, LNA is unable to form an ideal shape in Watson-Crick base pairing. When LNAs are comprised in the oligonucleotides of DNA or RNA, LNAs can more rapidly pair with complementary nucleotide chains to increase the stability of the double helix.

In the present invention, the "antisense" refers to oligomers with a sequence of nucleotide bases and an inter-subunit backbone, in which the antisense oligomer is hybridized with a target sequence in RNA by Watson-Crick base pair formation, allowing the formation of mRNA and RNA:oligomer heteroduplexes in the target sequence. Oligomers can have a sequence that has exactly or near complementarity to the target sequence.

Since the information regarding CAMKK2 or ACLY or the gene encoding them are known, those skilled in the art can easily design primers, probes or antisense nucleotides that specifically bind to the gene encoding the protein based on this information.

In the present invention, in the process of confirming the presence and expression level of the gene, analysis methods for measuring the expression level of the gene comprise reverse transcription polymerase reaction (RT-PCR), competitive reverse transcription polymerase reaction (Competitive RT-PCR), real-time reverse transcription polymerase reaction (Real-time RT-PCR), RNase protection assay (RPA), Northern blotting or DNA chip, but are not limited thereto.

In the present invention, when the activity or expression level of at least one of CAMKK2 and ACLY measured in the biological sample is reduced after treatment of the candidate material, or the expression level of the gene encoding the protein is reduced, the method may further comprise the step of determining the candidate substance as a cancer metastasis inhibitor.

In the present invention, the cancer is a cancer in which autophagy, preferably macroautophagy, is activated, and may comprise at least one of the KRAS gene and the LKB1 gene, preferably the KRAS gene, and more preferably the KRAS gene and mutations in both the LKB1 gene.

In the present invention, the cancer may be breast cancer, ovarian cancer, colon cancer, stomach cancer, liver cancer, pancreatic cancer, cervical cancer, thyroid cancer, parathyroid cancer, lung cancer, non-small cell lung cancer, prostate cancer, gallbladder cancer, biliary tract cancer, non-Hodgkin's lymphoma, Hodgkin's lymphoma, blood cancer, bladder cancer, kidney cancer, melanoma, colon cancer, bone cancer, skin cancer, head cancer, uterine cancer, rectal cancer, brain tumor, perianal cancer, fallopian tube carcinoma, endometrial carcinoma, vaginal cancer, vulvar carcinoma, esophageal cancer, small intestine cancer, endocrine adenocarcinoma, adrenal cancer, soft tissue sarcoma, urethral cancer, penile cancer, ureteral cancer, renal cell carcinoma, renal pelvic carcinoma, central nervous system (CNS) tumor, primary CNS lymphoma, spinal cord tumor, brainstem glioma or pituitary adenoma, and may preferably be, lung cancer or pancreatic cancer, but is not limited thereto.

According to another embodiment of the present invention, it relates to a method for providing information for predicting the therapeutic response of a metastasis inhibitor of cancer comprising the step of detecting the existence of a mutation in at least one gene of KRAS and LKB1 in a biological sample isolated from a target individual.

The "target individual" as used herein refers to an individual who has developed cancer or has a high possibility of developing cancer. Here, the individual is a mammal including humans, and may be at least one selected from the group consisting of humans, rates, mice, guinea pigs, hamsters, rabbits, monkeys, dogs, cats, cows, horses, pigs, sheep, and goats, and may preferably be humans, but is not limited thereto.

The "biological sample" as used herein refers to any material, biological fluid, tissue or cell obtained from or derived from an individual, and may comprise, for example, whole blood, leukocytes, peripheral blood mononuclear cells, buffy coat, plasma, serum, sputum, tears, mucus, nasal washes, nasal aspirate, breath, urine, semen, saliva, peritoneal washings, pelvic fluids, cystic fluid, meningeal fluid, amniotic fluid, glandular fluid, pancreatic fluid, lymph fluid, pleural fluid, nipple aspirate, bronchial aspirate, synovial fluid, joint aspirate, organ secretions, cells, cell extracts, or cerebrospinal fluids, but may preferably be cancer tissue.

In the present invention, analysis methods for detecting mutations in the gene or confirming the expression level of the gene may comprise at least one selected from the group consisting of reverse transcription polymerase reaction (RT-PCR), competitive reverse transcription polymerase reaction (Competitive RT-PCR), real-time reverse transcription polymerase reaction (Real-time RT-PCR), RNase protection assay (RPA), Northern blotting, DNA chip and RNA sequencing, but are not limited thereto.

In the present invention, the agent for detecting mutations in the gene or measuring the expression level of the gene may comprise at least one selected from the group consisting of primers, probes, and antisense nucleotides that specifically bind to the gene or the transcript of the gene.

In addition, in the present invention, a method of detecting, measuring, or comparatively analyzing the expression level of a protein encoded by the gene can be performed to detect whether or not the gene is mutated, and more concretely may be performed by at least one method selected from the group consisting of protein chip analysis, immunoassay, ligand binding assay, MALDI-TOF (Matrix Assisted Laser Desorption/lonization Time of Flight Mass Spectrometry) analysis, SELDI-TOF (Surface Enhanced Laser Desorption/lonization Time of Flight Mass Spectrometry) analysis, radiation immunity assay, radioimmuno-diffusion method, Oukteroni immunodiffusion method, rocket immunoelectrophoresis, tissue immunostaining, complement fixation assay, two-dimensional electrophoretic assay, Liquid Chromatography-Mass Spectrometry (LC-MS), Liquid Chromatography-Mass Spectrometry/ Mass Spectrometry (LC-MS/MS), Western blotting or ELISA (enzyme linked immunosorbentassay), but is not limited thereto.

In the present invention, the agent for measuring the expression level of the protein comprises at least one selected from the group consisting of an antibody, an oligopeptide, a ligand, a peptide nucleic acid (PNA), and an aptamer that binds specifically to the protein, but is not limited thereto.

In addition, the method of providing information of the present invention may further comprise the step of detecting whether or not the Snail protein is acetylated in the biological sample.

In the present invention, the Snail protein may consist of amino acids represented by SEQ ID NO: 3, but is not limited thereto.

In the present invention, the method for detecting the presence or degree of acetylation of the Snail protein may comprise at least one selected from the group consisting of Western blotting, immunoprecipitation, and enzyme linked immunosorbent assay (ELISA), but is not limited thereto.

In the present invention, when a mutation is present in at least one gene of KRAS and LKB1 in the biological sample, and furthermore, when Snail protein acetylation is increased compared to the control group, it can be predicted that the cancer metastasis inhibitor has high therapeutic response. Here, the control group may be the level of acetylation of Snail protein in a corresponding sample from a normal individual, the acetylation level of Snail protein in a corresponding sample from an individual with or at high risk of developing cancer, or its median value or its average value, or the acetylation level of Snail protein in a corresponding sample from an individual who has or has a high possibility of developing cancer without mutations in KRAS and LKB1, or a median value thereof, or an average value thereof, but is not limited thereto.

In the present invention, the cancer metastasis inhibitor may be an activity or expression inhibitor of CAMKK2 or ACLY protein, or an expression inhibitor of a gene encoding the protein.

In the present invention, the activity or expression inhibitor of the CAMKK2 or ACLY protein may comprise any one or more selected from the group consisting of compounds, peptides, peptide mimetics, aptamers, antibodies, and natural products that specifically bind to the protein.

As an example of the present invention, the activity or expression inhibitor of the CAMKK2 protein may be STO-609, SGC-CAMKK2-1, KN62, KN93, AlP, ACS-I, and berbamine, but is not limited thereto.

As an example of the present invention, the activity or expression inhibitor of the ACLY protein may be BMS303141, NDI-091143, SB-204990, ETC-1002, radicicol, (-)-hydroxycitric acid and 2-chloro-1,3,8-trihydroxy-6-methylanthrone, and the like, but are not limited thereto.

In the present invention, expression inhibitors of the gene encoding CAMKK2 or ACLY may comprise any one or more selected from the group consisting of antisense nucleotides, short interfering RNA (siRNA), short hairpin RNA (shRNA) and ribozymes that complementarily bind to the gene.

In the present invention, the cancer may be a cancer in which autophagy, preferably macroautophagy, is activated.

In the present invention, the cancer may be breast cancer, ovarian cancer, colon cancer, stomach cancer, liver cancer, pancreatic cancer, cervical cancer, thyroid cancer, parathyroid cancer, lung cancer, non-small cell lung cancer, prostate cancer, gallbladder cancer, biliary tract cancer, non-Hodgkin's lymphoma, Hodgkin's lymphoma, blood cancer, bladder cancer, kidney cancer, melanoma, colon cancer, bone cancer, skin cancer, head cancer, uterine cancer, rectal cancer, brain tumor, perianal cancer, fallopian tube carcinoma, endometrial carcinoma, vaginal cancer, vulvar carcinoma, esophageal cancer, small intestine cancer, endocrine adenocarcinoma, adrenal cancer, soft tissue sarcoma, urethral cancer, penile cancer, ureteral cancer, renal cell carcinoma, renal pelvic carcinoma, central nervous system (CNS) tumor, primary CNS lymphoma, spinal cord tumor, brainstem glioma or pituitary adenoma, and may preferably be, lung cancer or pancreatic cancer, but is not limited thereto.

According to another aspect of the present invention, there is provided a method for inhibiting metastasis of cancer comprising the step of administering the composition comprising an inhibitor of the activity of at least one protein of calcium/calmodulin-dependent protein kinase kinase 2 (CAMKK2) and ATP citrate lyase (ACLY), or an inhibitor of the expression of the gene encoding the protein as an active ingredient to a subject.

Descriptions of Calcium/calmodulin-dependent protein kinase kinase 2 (CAMKK2), ATP citrate lyase (ACLY), an activity inhibitor of at least one protein of the present invention, and inhibitors of expression of genes encoding the proteins and carcinoma have already been described in detail, and thus descriptions thereof are omitted to avoid excessive redundancy.

### ADVANTAGEOUS EFFECTS OF THE INVENTION

When the composition of the present invention is used, cancer metastasis in cancers in which autophagy is activated can be effectively inhibited.

### BRIEF DESCRIPTION OF DRAWINGS

FIG. 1A illustrates the results of performing the Matrigel invasion assay on HBEC30KT-derived cancer-progressing cell lines (HBEC30KT cell, HBEC30KT-shTP53; HBEC-TP53 cell, HBEC30KT-shTP53/KRAS G12V; HBEC-K cell, HBEC30KT-shTP53/KRAS G12V/shLKB1; HBEC-KL cell). The left panel is a photograph (x20) of cells infiltrating the Matrigel-coated transwell membrane observed under a bright field microscope, and the right panel is the quantified result thereof.
FIG. 1B illustrates the results of performing a scratch wound healing assay in HBEC30KT-derived cancer progression cell lines, and the left panel is a photograph (x20) of the migrated cells under a light microscope while the right panel is the quantified result thereof.
FIG. 1C illustrates the accumulation of each protein in a steady state by Western blotting for total cell lysates obtained from a KRAS mutant (K) lung cancer cell line and a KRAS and LKB1 co-mutated (KL) lung cancer cell line.
FIG. 1D illustrates the results of the Matrigel invasion assay of the K cell line and the KL cell line.
FIG. 1E illustrates the results of the Matrigel invasion assay after each siRNA treatment in KL cell lines (A549, NCI-H157, NCI-H647, HCC44).
FIG. 1F illustrates the effect of SNAI1 knockdown on tumor metastasis in the A549-luc tail vein injection model, and A549-luc cells with or without SNAI1 knockdown were intravenously injected into nude mice (n=8 mice per group). Bioluminescence images (left) in each group at 4 weeks after injection and results of analyzing the total degree of metastasis through photonic flux (right) are shown.
FIG. 1G illustrates H&E stain photographs (top) of lung sections from the SNAI1 knockdown group and the control group, and the number of metastasized nodules in the lungs of each group (bottom).
FIG. 1H illustrates a graph of changes in cell viability depending on the dose when 72 hours elapsed after treatment with AMG-510 (0.01-10 uM) in 4 K cell lines (black) and 3 KL cell lines (red) containing the KRAS G12C mutation. FIG. 1L illustrates the results of Matrigel invasion assay after treatment with AMG-510 (1 uM) for three KL cell lines. The upper panel shows bright field micrographs, and the lower panel shows the quantification results.
FIG. 1J illustrates the Western blotting results of the expression level of each protein after treating the cell line with AMG-510 (1 uM) for 48 hours.
FIGS. 2A and 2B illustrates the steady-state accumulation of each protein and phospho-protein through Western blot with respect to the total cell lysate obtained from the HBEC30KT-derived cell line.
FIG. 2C illustrates the prognostic effect of target gene expression of EMT transcription factors in three independent LUAD cohorts (TCGA, GSE41271, GSE72094). In the TCGA-LUAD sample, genes showing a significantly positive correlation (P<0.05, Spearman rank correlation test) with each transcription factor were selected as target genes. The single-sample gene set enrichment analysis score of each patient's target gene was used as a threshold for differentiating old patient groups with low and high EMT transcription factor activity.
FIG. 2D illustrates the effects of KRAS-deficient or translocated LKB1 expression on Snail protein levels at 72 hours after transfection with KRAS siRNA or at 24 hours after transfection with LKB1 cDNA (1 ug) by Western blotting.
FIG. 3A illustrates the ROS expression level confirmed using the H2DCFDA probe in the HBEC-K cell line and the HBEC-KL cell line.
FIG. 3B illustrates the mitochondrial ROS expression levels in four K cell lines and KL cell lines.
FIG. 3C illustrates the analyzed results of the expression level of each gene through real-time PCR after treating the HBEC-K cell line and the HBEC-KL cell line with 150 uM HzOz for 6 hours.
FIG. 3D illustrates the steady-state accumulation of phosphorylated-AMPK protein by Western blot on total cell lysates from HBEC-K and HBEC-KL cell lines.
FIG. 3E illustrates the degree of Snail accumulation through Western blot as a result of knocking down AMPK with siRNA in HBEC-KL cell line (left), and the result of analyzing the 2D invasion ability through Matrigel invasion assay (right).
FIGS. 3F and 3G illustrate the Western blot results of p-AMPK, total AMPK, and Snail expression after the HBEC-KL cell line was treated with HzOz or N-acetylcysteine (NAC) for 6 hours.
FIG. 3H illustrates the Western blot results of changes in the expression level of each protein after treating the HBEC-KL cell line with the CDDO-ME NRF2 activator for 24 hours.
FIG. 3I illustrates the steady-state accumulation of phosphorylated-CAMKII protein by Western blot of total cell lysates of HBEC-K and HBEC-KL cell lines.
FIG. 3J illustrates changes in the expression level of each protein after treating the HBEC-KL cell line with BAPTA, a calcium chelator, or calmidazolium, a calmodulin inhibitor, for 1 hour.
FIG. 3K illustrates the Western blot results showing the changes in Snail expression after genetically knocking down CAMKK2 with shRNA in HBEC-KL cell line.
FIGS. 3L and 3M illustrate the changes in Snail accumulation and 2D invasion ability confirmed by Western blot and the result of Matrigel invasion assay after treatment of HBEC-KL cell lines with the CAMM2 inhibitor STO-609 (0, 25, or 50 uM) for 24 hours.
FIG. 3N illustrates the role of CAMM2 inhibitor STO-609 (0, 25 uM) on the invasive ability of KC cell line spheroids in 3D culture conditions in KL cell line. Changes in infiltration length after treatment with 25 uM STO-609 were compared.
FIG. 4A illustrates the analysis results of the expression level of each gene through real-time PCR analysis in the HBEC-K cell line and the HBEC-KL cell line.
FIG. 4B illustrates the analysis results of the expression level of each gene through real-time PCR analysis in four K cell lines and KL cell lines.
FIG. 4C illustrates the accumulation of p62 and NRF2 proteins in a normal state by performing Western blot on the total cell lysate from the HBEC-K cell line and the HBEC-KL cell line. FIG. 4D illustrates the effect of translocation p62 expression on Snail protein expression in the HBEC-KL cell line, and the protein expression level was confirmed by Western blotting 24 hours after transformation.
FIG. 4E illustrates the expression levels of phospho-AMPK and Snail by Western blotting after treatment of 4 KL cell lines with STO-609 for 24 hours.
FIG. 4F illustrates the accumulation of Snail protein through Western blot after treating KL cell lines with 1 uM AMG-510 and/or 50 uM STO-609.
FIG. 4G illustrates a picture (x 20) of HBEC-KL cell line after treating with STO-609 for 24 hours and performing a scratch wound healing assay to observe the migrating cells under a light microscope (x 20) (top) and the quantified result (bottom).
FIG. 4H illustrates the analysis results of the 3D invasion ability of tumor spheres before and after treatment with 25 uM STO-609 for 6 days. The arrow indicates the front of the infiltration, and the scale bar is 200 um.
FIG. 5A illustrates the detection of lysosomes and autophagy resolvers in the normal state through immunofluorescence in the HBEC-KL cell line (scale bar, 100um). LC3B (green dots) represents autophagosomes, LysoTracker (red dots) represents lysosomes, and DAPI was used to visualize the nucleus.
FIG. 5B illustrates the Western blot results of LC3 protein in HBEC-K and HBEC-KL cell lines treated with 50 uM chloroquine (CQ) for 6 hours.
FIG. 5C illustrates the relative luciferase activity at steady state in the HBEC-K and HBEC-KL cell lines transiently transfected with the CLEAR motif-driven luciferase reporter construct. Relative luciferase intensities were expressed as fold change relative to HBEC control cells.
FIGS. 5D and 5E illustrate the relative luciferase activity after stably transforming the TFEB-luciferase reporter construct into the HBEC-KL cell line and then treating it with N-acetylcysteine (NAC) or 50 uM STO-609 for 24 hours.
FIGS. 5F and 5G illustrate the Western blotting results showing changes in the expression level of Snail protein in total cell lysates of the HBEC-KL cell line after inhibition of autophagy by treatment with siRNA or chloroquine (CQ) for 12 to 48 hours.
FIGS. 5H and 5I illustrate the changes in the expression level of Snail protein after treatment with chloroquine (CQ) or STO-609 in the presence of 20 nM bortezomib in the HBEC-KL cell line.
FIG. 5J illustrates the expression level of each protein in the normal state with respect to the total cell lysate of the HBEC-K cell line and the HBEC-KL cell line by Western blotting.
FIG. 5K illustrates the expression level of Snail protein after treatment with the GSK3β inhibitor CHIR99021 (3 uM, treatment for 5 hours) in the presence of 50 uM chloroquine (CQ) in the HBEC-KL cell line.

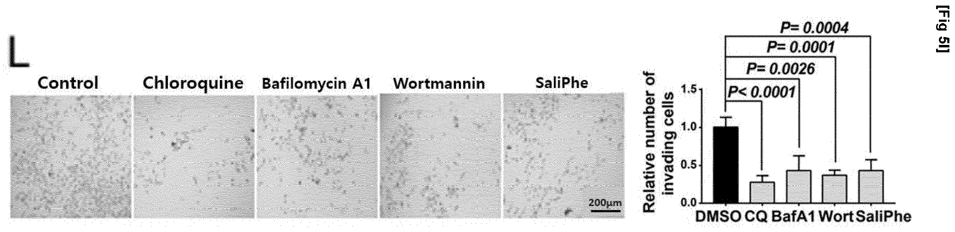

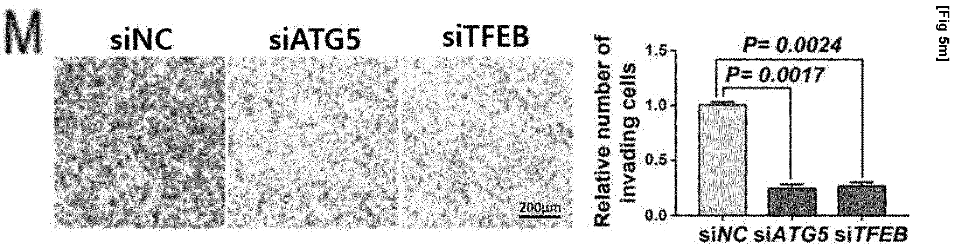

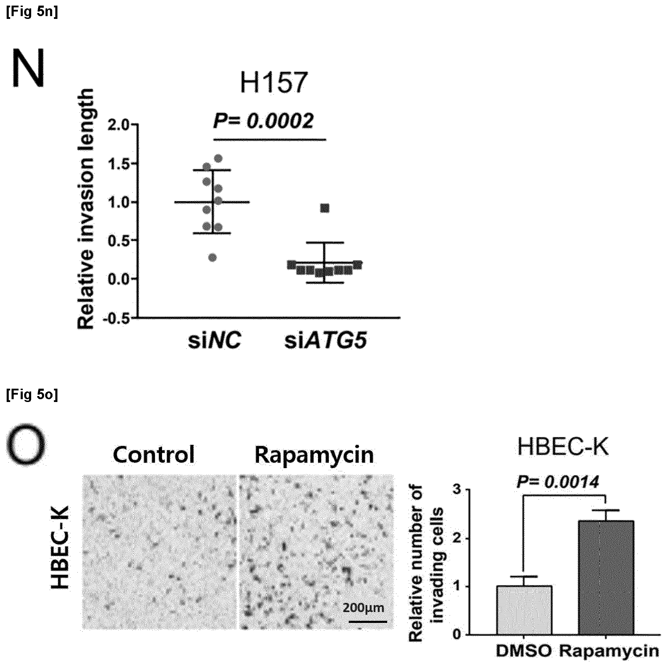

FIGS. 5L, 5M and 5O illustrate the effect on matrigel invasiveness after genetic knockdown (M) by treating the HBEC-KL cell line with 25 uM of CQ (lysomotropic weak base), 10 nM of bafilomycin A1 (BafA1: V-ATPase inhibitor), 1 uM of wortmannin (Wort: PI3K inhibitor), and 10 nM of saliphenylhalamide (Sali: V-ATPase inhibitor) for 24 hours (L,O) or treating with siRNA. FIGS. 5L and 5M illustrate the results of the Matrigel invasion assay in the HBEC-KL cell line, and the HBEC-K cell line in FIG. 5O, and the left panel shows bright field micrographs of cells infiltrating through the Matrigel-coated transwell membrane, and the right panel shows the quantitative analysis results.
FIG. 5N illustrates the effect of ATG5 siRNA genetic knockdown on the invasiveness of KL cell line spheroids in 3D culture conditions. The change in invasion length after genetic knockdown of ATG5 siRNA was confirmed.
FIG. 6A illustrates the Western blotting results of changes in the expression level of the LC3 protein after treatment of HBEC-KL cell lines with N-acetylcysteine (NAC) or STO-609 in the presence of 50 uM chloroquine (CQ).
FIG. 6B illustrates the results of mRFP-GFP-LC3 lysosomal delivery assay analysis after treatment of HBEC-KL cell lines with 10 mM N-acetylcysteine (NAC) or 50 uM STO-609 for 24 hours. Representative GFP-LC3, RFP-LC3 and overlapping images are shown. In addition, red puncta (mRFP+/GFP-) and yellow puncta (mRFP+/GFP+) per cell were quantified and graphed (n = 15 cells/treatment).
FIG. 6C illustrates the Western blotting results on the effect of AMPK siRNA-mediated genetic knockdown on TFEB expression levels in the HBEC-KL cell line.
FIGS. 6D and 6E illustrate the effect of KRAS deficiency (D) or translocation LKB1 expression (E) on TFEB protein in HBEC-KL cell lines, which were confirmed by Western blotting 72 hours after KRAS siRNA transformation and 24 hours after LKB1 cDNA (1 ug) transformation.
FIG. 6F illustrates the change in the expression level of the SNAI1 gene through real-time PCR after treatment of the HBEC-KL cell line with 50 uM chloroquine (CQ) for 24 hours (left) or siRNA genetic knockdown for 48 hours (right).
FIG. 6G illustrates the analysis results of polyubiquitinated Snail after transformation with Flag-Snail after treatment of HBEC-KL cell line with 50 uM chloroquine (CQ) for 24 hours. Here, Snail is the aanalysis of polyubiquitination through Western blot using anti-ubiquitin antibody after immunoprecipitation from 1 ug sample on anti-Flag M2 affinity gel (40 ul).
FIG. 6H illustrates the Western blotting results for changes in the expression level of each protein after treatment of HBEC-KL cell lines with 50 uM chloroquine (CQ).
FIG. 6I illustrates the Western blotting results of change in Snail expression and Matrigel invasion assay results confirming the change in invasion ability after genetic knockdown of ATG5 and TFEB siRNA in the KL cell line.
FIG. 6J illustrates the results of the scratch wound healing assay after treating the HBEC-KL cell line with 50 uM chloroquine (CQ) for 24 hours, and the left panel analyzes the migrated cells by light microscopy (x20) and the right panel shows the quantified results.
FIG. 6K illustrates a 3D invasion image of tumor cell spheres, and the red circle represents the spheroid core while the arrow represents the invasion front (scale bar: 200 um).
FIG. 7A illustrates the volcano plots of 225 metabolites expressed in 14 KRAS/LKB1co-mutant lung cancer cell lines (KL) and 31 KRAS mutant lung cancer cell lines (K) under normal conditions in the Cancer Cell Line Encyclopedia (CCLE) metabolome data, and the red dots represent metabolites that are statistically highly expressed in KL cell lines and blue dots represent metabolites that are low in expression. In addition, the right side shows a heat map showing cell line-specific expression levels of 11 highly up-regulated metabolites in KL cell lines.
FIG. 7B illustrates the expression level of acetyl-CoA in a steady state in the HBEC-K cell line and the HBEC-KL cell line.
FIGS. 7C and 7D illustrate changes in the expression level of acetyl-CoA after each genetic knockdown in the HBEC-KL cell line.
FIG. 7E illustrates changes in citrate expression levels after HBEC-KL cell lines were treated with 8 mM methyl pyruvate (MP), 3 mM a-keto-glutarate (MOG), or 200 µM rosiglitazone in the presence of 50 uM chloroquine (CQ).
FIG. 7F illustrates the results of measuring the accumulation of Snail protein through Western blotting after the HBEC-KL cell line was treated with MP/MOG/rosiglitazone and chloroquine (CQ) in combination.
FIG.7G illustrates the expression level of each protein through Western blotting after treatment of HBEC-K cell lines with 2 uM rapamycin.
FIG. 7H illustrates a diagram showing acetyl-CoA metabolism in mammalian cells.
FIGS. 7I and 7J illustrate the effect of acetyl-CoA expression level and matrigel invasiveness upon ACLY and ACSS2 siRNA-mediated knockdown in the HBEC-KL cell line.
FIG. 7K illustrates the effect on matrigel invasiveness upon 10 mM acetate treatment and/or ACSS2 siRNA mediated knockdown in HBEC-KL cell lines.
FIG. 7L illustrates the effect on citrate expression levels upon CS and IDH1 siRNA-mediated knockdown in HBEC-KL cell lines.
FIG. 7M illustrates the effect on Snail accumulation upon CS and ACLY siRNA-mediated knockdown in the presence of 8 mM MP in HBEC-KL cell line (left), and the effect on Snail accumulation upon CS, IDH1, and ACLY siRNA-mediated knockdown in the presence of 3 mM MOG (right).
FIG. 7N illustrates the effect on Snail accumulation upon IDH1 siRNA-mediated knockdown with 50 uM chloroquine (CQ) treatment in the presence of 3 mM MOG in the HBEC-KL cell line.
FIG. 7O illustrates the effect on matrigel invasiveness and cell migration ability upon ACACA siRNA-mediated knockdown in HBEC-KL cell lines.
FIG. 7P illustrates the effect on matrigel invasiveness upon treatment with the CBP inhibitor C646 (25 uM) and/or 10 mM acetate.
FIG. 8A illustrates a diagram comparing metabolites in HBEC-KL cells transfected with ATG5-specific siRNA and negative control siRNA (n=5 per group), and while the color of the circle indicates the relative level of the metabolite, the red border indicates the metabolite upregulated in KL cells as shown in FIG. 7A.
FIG. 8B illustrates the steady state citrate levels in the HBEC-K cell line and the HBEC-KL cell line.
FIG. 8C illustrates changes in citrate levels upon ATG5 siRNA-mediated knockdown in HBEC-KL cell lines.
FIGS. 8D and 8E illustrate the fold change in acetyl-CoA levels compared to the control group after treatment with 50 uM chloroquine (CQ) in the HBEC-KL cell line and the KL cell line.
FIG. 8F illustrates the change in the citrate expression after treatment with 50 uM STO-609 in the HBEC-KL cell line.
FIG. 8G illustrates changes in matrigel invasiveness after ACLY and ACSS2 genetic knockdown in KL cell lines. The left panel shows bright field micrographs of cells infiltrating through the matrigel-coated transwell membrane, and the right panel shows the quantitative analysis results.
FIG. 8H illustrates changes in matrigel invasiveness after treatment with 10 mM acetate for 24 hours in the HBEC-KL cell line.
FIG. 8I illustrates changes in matrigel invasiveness afterACACAsiRNA-mediated knockdown in the KL cell line.
FIGS. 8J and 8K illustrate changes in matrigel invasiveness after CREBBP siRNA siRNA-mediated knockdown in HBEC-KL cell line (FIG. 8J) and KL cell line (FIG. 8K).
FIG. 8L illustrates changes in matrigel invasiveness after treatment with 50 uM C646 for 24 hours in the HBEC-KL cell line.
FIG. 9A illustrates the steady-state total protein acetylation levels (left) and the immunoprecipitation-immunoblot results of the level of acetylated Snail in the HBEC-K and HBEC-KL cell lines under normal conditions.
FIGS. 9B and 9C illustrate the immunoprecipitation-immunoblot results of the level of acetylated Snail after treatment with 50 uM chloroquine (CQ) or genetic knockdown in HBEC-KL cell lines.
FIG. 9D illustrates the Western blotting result of the expression level of Snail protein (left) and the quantitative concentration analysis results of Snail for beta-actin (right) after 2 uM rapamycin (5-hour treatment) and/or ACLY siRNA-mediated knockdown.
FIG. 9E illustrates the Western blotting results for changes in the expression level of each protein after 2 hours of treatment with CBP inhibitor C646 (25 uM) in HBEC-KL cell lines (left) and KL cell lines (right). In addition, changes in the expression level of the SNAI1 gene through real-time PCR after treating the HBEC-KL cell line with 25 uM C646 for 2 hours is shown.
FIG. 9F illustrates the Western blotting results of the change in the expression level of Snail protein after treatment with CREBBP siRNA for 72 hours in HBEC-KL cell line (left) and KL cell line (right).
FIG. 9G illustrates the change in the expression level of each protein after treating the HBEC-KL cell line with 3 uM HDAC inhibitor SAHA (vorinostat) for 15 hours (top), and the immunoprecipitation-immunoblot results of the level of acetylated Snail after treatment with 3 uM SAHA for 15 hours (bottom).
FIG. 9H illustrates results of the Matigel invasiveness assay after SNAI1 siRNA-mediated knockdown and/or SAHA treatment of HBEC-KL cell lines.
FIG. 9I illustrates the Western blotting results of the accumulation of Snail protein after treating the HBEC-KL cell line with 3 uM SAHA and 50 uM chloroquine (CQ) for 24 hours.
FIG. 9J illustrates the Western blotting results of the level of Snail protein (left), results of matrigel invasiveness analysis under the same conditions (middle), and bar plots from the two experiments (right) when 24 hours elapsed after transformation of potential wild-type or acetyl-defective Snail (K146R, K187R) to the K cell line.
FIG. 9K illustrates the immunoprecipitation-immunoblot result of phosphorylated/acetylated or K48 polyubiquitinated Snail assay after transfection of wild-type Snail (pFLAG-Snail WT) or acetylation-defective Snail mutant (pFLAG-SnaiIK146R) into A549 cells and/or treatment with 25 uM C646.
FIG. 9L illustrates the immunoprecipitation-immunoblot results of the polyubiquitinated Snail assay after transfection of HA-ubiquitin and wild-type Snail (pFLAG-Snail WT) or acetylation-defective Snail mutant (pFLAG-SnaiIK146R) into A549 cells and/or treatment with 25 uM C646 for 2 hours (left) and/or treatment of 3 uM SAHA for 5 hours (right).
FIG. 10A illustrates the Western blotting results of the expression level of Snail protein after treating with acetate at each concentration in the HBEC-KL cell line for 24 hours (left), and changes in the expression level of the SNAI1 gene by real-time PCR (right) after treatment with 10 mM acetate for 24 hours.
FIG. 10B illustrates the Western blotting results of the expression of Snail protein after treating with ACACA siRNA in the HBEC-KL cell line for 48 hours (left), and changes in the expression level of the SNAI1 gene by real-time PCR (right) after the same treatment.
FIG. 10C illustrates the Western blotting results of the degree of total protein acetylation after treatment with 10 mM acetate for 24 hours in HBEC-KL cell line (left), and immunoprecipitation-immunoblot results of acetylated Snail under the same conditions.
FIG. 10D illustrates the Western blotting results of the degree of total protein acetylation after treating ACLY siRNA-mediated knockdown in the HBEC-KL cell line for 48 hours (left), and immunoprecipitation-immunoblot results of acetylated Snail under the same conditions.
FIG. 10E illustrates the accumulation of Snail protein in HBEC-KL cell lines treated with ACLY inhibitors BMS-301341, NDI-091143, and SB-204990 for 24 hours, 24 hours, and 2 hours, respectively, and 20 nM bortezomib for 3 hours, and changes in matrigel invasiveness after 50 uM BMS3013141 treatment for 24 hours, 1 uM NDI-091143 treatment for 24 hours, and 20 uM SB-204990 treatment for 2 hours. The bottom left panel shows bright field micrograph pictures of cells infiltrating through the Matrigel-coated transwell membrane, and the bottom right panel shows the quantitative analysis results.
FIG. 10F illustrates acetylation of total protein after CAMKK2 shRNA-mediated knockdown or treatment with 50 uM STO-609 for 48 hours in HBEC-KL cell lines (top), and immunoprecipitation-immunoblot results of acetylated Snail after 48 hours of 50 uM STO-609 treatment (below).
FIGS. 10G and 10H illustrate Western blotting results of the degree of total protein acetylation after treatment with 50 uM chloroquine (CQ) for 24 hours or TFEB/ATG5 siRNA for 48 hours in HBEC-KL cell lines.
FIG. 10I illustrates the degree of total protein acetylation after treatment with rapamycin for 5 hours in the HBEC-K cell line.
FIG. 10J illustrates Western blotting results of changes in the expression level of each protein after treatment with 2 uM rapamycin for 5 hours (left) or translocation TFEB expression for 24 hours (right) in HBEC-K cell lines. pS6 and S6 are mTOR activity markers, and LC3 corresponds to an autophagy flux marker.
FIG. 10K illustrates the immunoprecipitation-immunoblot results of acetylated Snail after treatment with 2 uM rapamycin in the HBEC-KL cell lines.
FIG. 10L illustrates Western blotting results of changes in Snail expression level after treatment with SAHA (3 uM) and/or 100 uM cyclohexamide in the HBEC-KL cell lines.
FIG. 11A illustrates measurements of changes in luciferase activity after transfection for 24 hours to express translocation TFEB or acetyl-defective TFEB4KR in HBEC-K cell lines transiently transfected with the CLEAR motif-driven luciferase reporter construct (left), and measurements of changes in luciferase activity after treatment with 3 uM SAHA for 5 hours under the same conditions (right).
FIG. 11B illustrates Western blotting results of changes in LC3-II expression levels (left), and quantitative concentration analysis results of HSP90 (right) after transfection for 24 hours to express potential TFEB or acetyl-deficient TFEB [4KR] in the presence of 50 uM chloroquine (CQ) in the HBEC-K cell line.
FIG. 11C illustrates Western blotting results of TFEB and phospho-TFEB (Ser211) expression levels in the normal state (left) and immunoprecipitation-immunoblot results of the level of acetylated TFEB in the HBEC-K cell line and HBEC-KL cell line.
FIG. 11D illustrates immunofluorescence analysis (scale bar, 25 µm) of TFEB in normal state using an anti-TFEB antibody (green) (left), and the results of quantifying the number of cells with TFEB in the nucleus in the above cell lines in four independent experiments (right) in the HBEC-K cell line and HBEC-KL cell line.
FIG. 11E illustrates the immunofluorescence analysis of TFEB expression level after treatment with 10 mM acetate for 24 hours or 3 uM SAHA for 5 hours (scale bar, 25 µm) (left), and the results of quantifying the number of cells with TFEB in the nucleus in the above cell lines in four independent experiments (right) in the HBEC-KL cell line.
FIG. 11F illustrates changes in luciferase activity after treatment with 10 mM acetate for 24 hours or treatment with 3 uM SAHA for 5 hours in the HBEC-KL cell line stably transfected with the CLEAR motif-driven luciferase reporter construct.
FIG. 11G illustrates Western blotting results of LC3-II accumulation (left) and quantitative concentration analysis results of beta-actin (right) after 24 hour treatment of 10 mM acetate or 5 hour treatment of 3 uM SAHA in the presence of 50 uM chloroquine (CQ) in the HBEC-KL cell line.
FIG. 11H illustrates the results of performing the mRFP-GFP-LC3 lysosomal delivery assay after 24 hour treatment of 10 mM acetate or 15 hour treatment of 3 uM SAHA in the HBEC-KL cell line, and representative GFP-LC3, RFP-LC3 and overlapping images are shown. In addition, red puncta (mRFP+/GFP-) and yellow puncta (mRFP+/GFP+) per cell were quantified and graphed (n = 15 to 30 cells/treatment).
FIG. 11I illustrates a diagram of immunoblot results of the effect on the accumulation of each marked protein as a result of exposure of HBEC-KL cells to 10 mM acetate for 24 hours using beta-actin as a control (left), and the immunoprecipitation-immunoblot result of TFEB acetylated in HBEC-KL cells under the same conditions (right) is also shown.
FIG. 11J illustrates a diagram of immunoblot results of the effect on the accumulation of each marked protein as a result of exposure of HBEC-KL cells to 3 µM SAHA for 18 hours hours using beta-actin as a control (left), and the immunoprecipitation-immunoblot result of TFEB acetylated in HBEC-KL cells under the same conditions (right) is also shown.
FIG. 11K illustrates the real-time PCR analysis results of changes in the expression level of the TFEB gene after treatment of HBEC-KL cell lines with 10 mM acetate for 24 hours (n = 2) or 3 uM SAHA for 15 hours (n = 3).
FIG. 11L illustrates the immunoprecipitation-immunoblot result of the level of acetylated TFEB after siRNA-mediated knockdown of ACLY expression in HBEC-KL cell line (left), and results of measuring luciferase activity after knocking down ACLY expression as above and treatment with 2 uM rapamycin for 5 hours in the HBEC-KL cell line stably transfected with the CLEAR motif-driven luciferase reporter construct (right).
FIG. 11M illustrates Western blotting results of the accumulation of each protein after treating HBEC-KL cell lines with 1 uM FK506 for 1 hour and 10 mM acetate for 24 hours or 3 uM SAHA for 15 hours (left), and results of measuring changes in relative luciferase activity under the same conditions in the HBEC-KL cell line stably transfected with the CLEAR motif-driven luciferase reporter construct (right).
FIG. 11N illustrates the immunoprecipitation-immunoblot results of the level of acetylated TFEB after treatment of HBEC-KL cell lines with 50 uM STO-609 for 24 hours.
FIG. 11O illustrates Western blotting results of the degree of accumulation of TFEB protein after HBEC-KL cell lines were treated with 50 uM chloroquine (CQ) for 24 hours with 10 mM acetate for 24 hours or 3 uM SAHA for 15 hours.
FIG. 12A illustrates the results of comparing citrate expression levels between 41 pancreatic cancer cell lines and 710 non-pancreatic cancer cell lines and non-lung cancer cell lines using CCLE metabolome data.
FIG. 12B illustrates Western blotting results of the accumulation of Snail and acetyl histone H3 after treatment of pancreatic cancer cell lines with 100 uM chloroquine (CQ) for 24 hours.
FIG. 12C illustrates changes in matrigel invasiveness after treating the pancreatic cancer cell line with 100 uM chloroquine (CQ) for 24 hours, and the bright-field micrographs of cells infiltrating through the matrigel coated transwell membrane (top) and quantification results (bottom) are shown.
FIG. 12D illustrates changes in the expression levels of acetyl-CoA after treating pancreatic cancer cell lines with 100 uM chloroquine (CQ) for 24 hours.
FIG. 12E illustrates the immunoprecipitation-immunoblot results of acetylated Snail after treatment of pancreatic cancer cell lines with 100 uM chloroquine (CQ) for 24 hours.
FIG. 12F illustrates Western blotting results of changes in Snail accumulation after ACLY siRNA-mediated knockdown in pancreatic cancer cell lines.
FIG. 12G illustrates analysis results of matrigel invasiveness after ACLY siRNA-mediated knockdown in pancreatic cancer cell lines.
FIG. 12H illustrates Western blotting results of change in the expression level of Snail protein after translocation expression of acetyl-deficient Snail (K146R or K187R) in pancreatic cancer cell lines (left), and the result of matrigel invasiveness analysis (right).

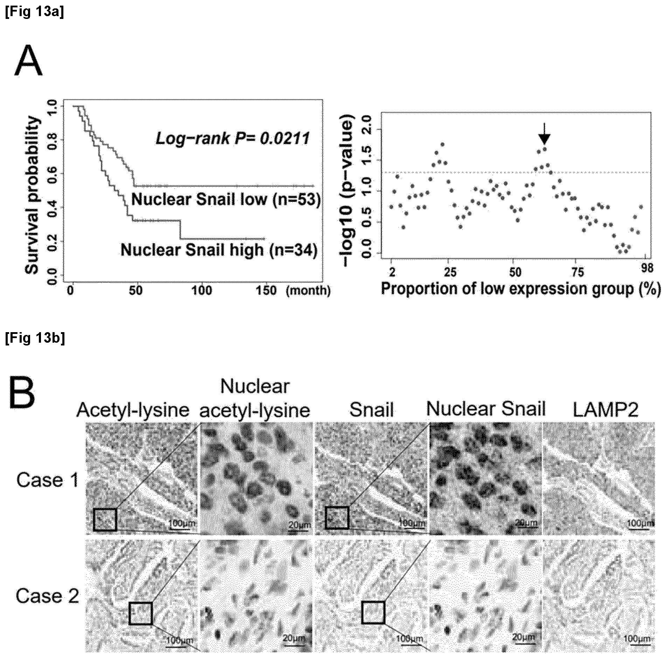

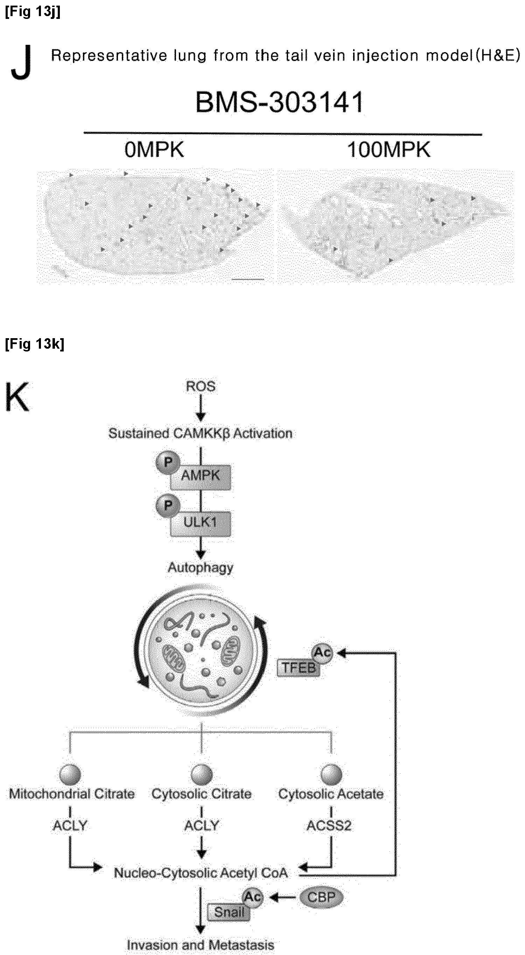

FIG. 13A illustrates the prognostic effect of Snail protein in the nucleus in a lung adenocarcinoma tissue microarray. Patients were divided into two groups according to the expression level of nuclear proteins (product of the median intensity and percentage of positively stained cells). The X axis represents the percent threshold in the nuclear low Snail protein expression score group. The red and blue origins on the right mean hazard ratios greater than 1 (high intranuclear Snail protein expression score group with poor prognosis) and less than 1 (high intranuclear Snail protein expression score group with good prognosis), respectively (right). Kaplan-Meier analysis shows the evaluation of overall survival following surgical operation in 87 lung adenocarcinoma low- and high-expression groups indicated by arrows in the right panel (left).
FIG. 13B illustrates IHC analysis results of total and nuclear acetyl-lysine, total and nuclear Snail, and LAMP2 protein expression levels in 87 LUAD tissues (scale bars: 20 um, 100 um).
FIG. 13C illustrates the results of comparing acetyl-lysine and LAMP2 expression levels in the nucleus in the low- and high-expression groups of Snail in the nucleus of FIG. 13A.
FIG. 13D illustrates immunoprecipitation-immunoblot analysis results of the level of acetylated Snail after treatment with 50 uM STO-609 for 24 hours in the A549-luc cell line.
FIG. 13E illustrates role of STO-609 on cancer metastasis in an A549-luc tail vein injection model. After intravenous injection of A549-luc cells into nude mice, vehicle or STO-609 (25 mg/kg and 100 mg/kg qd) was injected for 28 days starting one day after the injection. A picture taken of a bioluminescent image when 4 weeks have elapsed after injection is shown (above). Analysis results of the total metastasis burden by photonic flux are shown (below).
FIG. 13F illustrates H&E-stained images of lung sections injected with vehicle or STO-609 (100 mg/kg qd). Red arrows denote transition nodules (scale bar 4 mm).
FIG. 13G illustrates the number of metastasized nodules in the lungs of the vehicle and STO-609 (25 mg/kg or 100 mg/kg) administration groups.
FIG. 13H illustrates the role of BMS303141 on cancer metastasis in an A549-luc tail vein injection model. A549-luc cells were intravenously injected into nude mice, and then vehicle or BMS303141 (100 mg/kg q.d.) was injected for 20 days starting one day after the injection. A bioluminescence image when 3 weeks have elapsed after injection is shown.
FIG. 13I illustrates the analysis results of the total metastasis burden in the vehicle and BMS303141 administration groups by photonic flux.
FIG. 13J illustrates a photograph of H&E staining of lung sections, and red arrows indicate metastasis nodules (scale bar 2 mm).\
FIG. 13K illustrates a predictive model of autophagy-induced metastasis in lung cancer.
FIG. 14A illustrates the prognostic effect of total Snail expression in lung adenocarcinoma tissue microarrays. The red and blue origins on the right mean hazard ratios greater than 1 and less than 1, respectively. Kaplan-Meier analysis shows the evaluation of overall survival following surgical operation in 87 lung adenocarcinoma low- and high-expression groups indicated by arrows in the right panel.
FIG. 14B illustrates the result of comparison of acetyl-lysine and LAMP2 expression levels in the nucleus in the low- and high-expression groups of Snail in FIG. 14A.

### DETAILED DESCRIPTION OF INVENTION

Hereinafter, the present invention will be described in more detail by way of examples. However, the examples are only for illustrating the present invention, and the contents of the present invention are not limited by these examples.

### Examples

### Cell line

HBEC30 analogue cell progression series cell lines (HBEC30KT, HBEC-TP53, HBEC-K, and HBEC-KL cell lines) and 11 human NSCLC cell lines (A549, HCC44, NCI) were obtained from Michael A. White (UT Southwestern Medical Center, Texas, USA) -H157, NCI-H647, NCI-H460, NCI-H1155, NCI-H358, NCI-H441, HCC461, NCI-H2030, NCI-H2122). Three human NSCLC cell lines (Calu-1, NCI-H1373, HCC1171) and a pancreatic cancer cell line were purchased from Korea Cell Line Bank (KCLB). Three pancreatic cancer cell lines (KP4, KP4-1, and PK-59) were purchased from Riken, and the A549-luciferase cell line was obtained from the JCRB cell bank.

### Media and Reagents

AACL4 RPMI 1640 [GIBCO, 2.05 mM L-glutamine] medium supplemented with 0.02 mg/ml insulin, 0.01 mg/ml transferrin, 25 nM sodium selenate, 50 nM hydrocortisone, 10 mM HEPES, 1 ng/ml EGF, 0.01 mM ethanolamine, 0.01 mM O-phosphoethanolamine, 0.1 nM Triiodothyronine, 2 mg/ml BSA, and 0.5 mM sodium pyruvate medium and supplemented with 2% fetal bovine serum (GIBCO 16000-044) and 1% penicillin-streptomycin (Gibco, 15140122) was used to culture the HBEC30KT progression series. NSCLC cell line and pancreatic cancer cell line were maintained in RPMI-1640 medium (Gibco, 11875-093) supplemented with 5% (v/v) and 10% (v/v) fetal bovine serum (GIBCO 16000-044), respectively, and 1% penicillin-streptomycin (Gibco, 15140122) at 37 °C under 5% CO₂ conditions. The A549-luciferase cell line was maintained at 37 °C under 5% CO₂ condition in MEM alpha (Gibco, 12561-056) supplemented with 10% (v/v) fetal bovine serum (GIBCO 16000-044) and 1% penicillin-streptomycin (Gibco, 15140122).

### Western blot

Cells were harvested, washed with PBS, and lysed on ice with RIPA buffer containing protease and phophatase inhibitor cocktail (GenDEPOT). After 15 minutes of incubation, the cell lysates were centrifuged at 15,000 rpm at 4 °C for 10 minutes. Protein concentration was measured by Bradford assay (Bio-rad, 500-0006). Equal amounts of total protein were placed on an SDS gel electrophoresis and transferred to a PVDF membrane (Bio-rad). The membrane was blocked with 5% skim milk for 1 hour at room temperature and incubated overnight at 4 °C with primary antibody in a buffer containing 0.1% Tween-20. Subsequently, the membrane was washed three times with Tween-PBS buffer and incubated for 1 hour at room temperature using a secondary antibody diluted in blocking buffer containing 0.1% Tween-20. Subsequently, the membrane was washed three times for 10 minutes each using Tween-TBS. Densitometry of Western blots was measured using imaged software.

### Cell treatment

For inhibition of autophagy, cells were treated with 50 µM chloroquine (Sigma, C6628) for 12 to 24 hours. For autophagy activation, cells were treated with 2 µM rapamycin (Selleckchem, S1039) for 5 hours. Modulation of intracellular ROS was performed with N-acetyl-cysteine (10 mM) (Sigma, A7250), hydrogen peroxide solution (100 µM to 200 µM) (Sigma, 216763), or CDDO-ME NRF2 activator (50 nM, 100 nM) (Sellckchem, S8078) for 4 to 24 hours. Intercellular calcium signal control was performed for 1 hour using BAPTA-AM (20 µM) (Sigma, A1076) or Calmidazolium Chloride (2.5 µM) (Sigma, C3930). C646 (25 µM) (Sigma, SML0002), curcumin (10 µM) (Selleckchem, S1848) CBP/p300 inhibitor or vorinostat (3 µM) (Selleckchem, S1047) HDAC inhibitor was treated for 24 hours to modulate snail protein acetylation, and CHIR99021 GSK3b inhibitor (3 µM) (Sigma, SML1046) was treated for 24 hours to control Snail protein phosphorylation. For TFEB protein phosphorylation, tacrolimus (1 µM) (Selleckchem, S5003) was treated for 1 hour. For nutrient replenishment, cells were treated with 10 mM sodium acetate (Sigma, S2889), 8 mM methyl pyruvate (Sigma, 371173), or 3 mM dimethyl 2-oxoglutarate (Sigma, 349631) for 24 hours. For ACLY inhibitor treatment, cells were treated with 50 µM BMS303141 (Selleckchem, S0277) for 24 hours, and PPARr agonist rosiglitazone (Selleckchem, S2556) was treated with 200 µM concentration for 24 hours. STO-609 (Sigma, S1318), a CAMKK2 inhibitor, was treated to cells at a concentration of 25 µM or 50 µM for 24 hours, and AMG-510 (Selleckchehm 8830), a KRASG12C inhibitor, was treated to cells at a concentration of 100 nM or 1 µM for 48 hours. During siRNA treatment, each siRNA was treated to a concentration of 40 to 50 nM and treated together with Lipofectamine RNAiMax (Thermo Fisher Scientific, 13778150) for transformation, and were used for experiments 48 to 72 hours later. For cDNA treatment, each cDNA was treated to a concentration of 1 to 2 µg in a 6-well plate and treated with Lipofectamine 2000 (Thermo Fisher Scientific, 11668019) for transformation, and were used for experiments 24 hours later.

### Immunoprecipitation

For immunoprecipitation, equal amounts of lysate and Co-IP buffer (40 mM HEPES, pH 7.4, 120 mM NaCl, 2 mM EDTA, 0.3% CHAPS (Sigma, 10810118001), 10 mM pyrophosphate (Sigma, 221368), 10 mM glycerophosphate (Sigma, G9422), 50 mM NaF, phosphatase and protease inhibitor mixture) were mixed. Cell lysates (1 µg protein) were incubated overnight at 4° C on anti-FLAG^{®}M2 Affinity Gel (Sigma, A2220) or anti-acetyl-lysine antibody coated agarose (ImmuneChem Pharmaceuticals, ICP0388). The immunoprecipitate was then washed three times in IP buffer, and the immunoprecipitated complex was dissolved and separated by boiling in IP buffer for 5 minutes.
For SDS-PAGE electrophoresis, the immunoprecipitated complex was transferred to a nitrocellulose membrane and the membrane was blocked and then incubated for 1 hour at room temperature with Primary antibody and secondary antibody (VeriBlot for IP Detection Reagent (HRP) (Abcam, #ab131366), and anti-mouse IgG for IP (HRP) (Abcam, #ab131368)).
After washing 3 times, the membrane was incubated with the secondary antibody. Band signals were enhanced with the ECL Western Blot Substrate Kit (Pierce, 32106).

### Acetvl-CoA measurement

Intracellular acetyl-CoA levels were calculated in the pmol range using the PicoProbe Acetyl-CoAAssay Kit (Biovision, K317). Fluorescence levels were measured using Ex/Em=535/589 nm with an Envision Multimode Plate Reader (PerkinElmer 2105-0010, Waltham, MA, USA). After correcting the background in all readings, the value of each sample was determined and normalized to the protein concentration of each sample.

### Citrate assay

Intracellular citrate levels were calculated in the pmol range using a citrate assay kit (Biovision, K655). After sonication, proteins were removed from the supernatant using a 10 kDa molecular weight cutoff spin column, followed by incubation at room temperature for 30 minutes with 50 µl citrate reaction mixture comprising enzyme mixture, developer and citrate probe. Citrate content was measured by reading OD 570 nm with an Envision Multimode Plate Reader (PerkinElmer 2105-0010, Waltham, MA, USA). After correcting the background in all readings, the value of each sample was determined and normalized to the protein concentration of each sample.

### In vitro invasion assay

For the 2D invasion assay, the inserts were pre-coated with 300 µg/ml Matrigel (Corning, 354234). After staining with tryptophan blue dye (Bio-Rad, 1450013), 1×10 5 live cells per 8-um-pore cell-culture insert (Costar, 3422) were seeded in 0.5% ACL medium. 10% ACL medium was added to the outside of the insert. In the case of lung cancer and pancreatic cancer cell lines, after inoculation in 1% RPMI medium, 20% RPMI medium was added to the outside of the insert. Plates were incubated for 24 hours at 37 °C. After incubation, the insert was fixed with 3.7% PFA, washed, stained with 0.4% crystal violet, and the upper membrane was washed and dried.

The 3D spheroid cell invasion assay was performed using the Cultrex 3D spheroid cell invasion assay (R&D Systems, 3500-096-K). Briefly, cells resuspended in 1X spheroid-forming ECM solution were seeded at 4,000 viable cells per well of a 96-well plate and incubated at 37°C for 48 hours to form spheroids. When the spheroids were formed, they were inserted into the infiltration matrix, supplemented with a culture medium containing STO-609 (Sigma, S1318) or DMSO, and reprocessed by replacing the medium for 3 days. After culturing the 3D spheroid invasion assay plate for 7 days, the degree of spheroid invasion was quantified using imaged software. Three independent protrusions at the furthest distance from the spheroid boundary per well were measured and used for analysis to compare spheroid invasiveness.

### Immunofluorescence

Cells were seeded onto coated glass coverslips and maintained in ACL medium for 48 hours. After washing the cells three times with PBS, they were incubated in 3.7% paraformaldehyde for 10 minutes and fixed. Thereafter, the cells were washed three times with PBS and subjected to cell permeabilization in PBS containing 0.1% Triton X-100 for 10 minutes. After washing with PBS three times, the sample was blocked for 30 minutes at room temperature with PBS containing 0.1% Triton X-100 and 5% goat serum. Samples were incubated with primary antibody (1:150), followed by incubation with anti-rabbit/mouse Alexa Fluor 488/568 secondary antibody (Thermo Fisher Scientific, A-11001, 11004, 11008, 11011) and ProLong Gold Antifade Mountant with DAPI (Thermo Fisher Scientific, P36931). Images were taken with an Axio Imager M2 microscope (Carl Zeiss, Stockholm, Sweden) equipped with a 63x oil objective and analyzed with ZEN version 3.0 software.

### qRT-PCR

At 72 hours after siRNA transfection (post-transfection) and 12 to 24 hours after chemical treatment, total RNA was isolated with the RNeasy miniprep kit (QIAGEN, 74134). After synthesizing complementary DNA (cDNA) using the TOPscript^{™} cDNA synthesis kit (Enzynomics, EZ0054), qRT-PCR was performed with TOPreal^{™} qPCR 2X PreMIX (SYBR Green with low ROX) (Enzynomics, RT501M). GAPDH and beta-actin were used for RNA normalization. qRT-PCR was performed with a StepOne Plus (Applied Biosystems) instrument.

### Plasmid design

A mutant construct of Snail lysine (K) to arginine (R) was constructed by introducing a sense mutation into the SNAI1 coding sequence through site-specific mutation. A wild-type Snail expression vector was purchased from Origene and used as a mutation template. The primer sequences used were as follows: Snail K146R F: 5'- CTC TGA GGC CAG GGA TCT CCA GG -3' 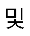 R: 5'- AGC TGG GCC AGC TGC TTG -3'. Snail K187R F: 5'- AAC CTG CGG GAG GGC CTT CTC TAG-3' 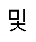 R: CCG CAG ACG CAG GGC AGC -3'. Snail's Lys146 and Lys187 were mutated, respectively.

### UPLC Q-TOF MS

ATG5 and negative control siRNA transfection was performed in five 100 cm² dishes per group. The sample was used after culturing for 48 hours, and medium was aspirated as much as possible, and then the cells were washed with 5 ml of cold PBS (no Mg2+/Ca2+). After vacuum aspiration of the PBS, metabolites were extracted by adding 1 ml 80% methanol (-80 °C) per dish, and the lysate was collected with a cell scraper and transferred to an Eppendorf tube on dry ice. After vortexing for 10 minutes in a cold room, insoluble debris was removed by centrifugation at 13,300g at 4 °C for 10 minutes, the supernatant was transferred to an Eppendorf tube on dry ice, and the pooled extract was stored at -80 °C before LC-MS analysis.

UPLC analysis was performed using a Waters Acquity^{™} Ultra Performance LC system (Waters MS Technologies, Manchester, UK). Chromatographic separation was performed on an ACQUITY UPLC BEH C18 column (100 mm × 2.1 mm, 1.7 µm) at a column temperature of 40 °C. The mobile phase consists of solvent A (0.1% aqueous formic acid, v/v) and solvent B (0.1% formic acid in methanol, v/v). Optimized UPLC dissolution conditions are as follows: 0.0-1.5 min, 1% B; 1.5-6.5 min, 10-20% B; 6.5-9.0 min, 20-70% B; 9.0-12.0 min, 70-99%; 12.0-16.0 min, -99%; 17.0-20.0 min, 1%. The flow rate was set to 0.3 mL/min and the injection volume was set to 5 ul, and the eluate was permeated into a SYNAPTTM G2 Quadrupole Time-of-flight Mass Spectrometer (Waters, Manchester, UK). For the positive electrospray mode, the capillary voltage and cone voltage were set to 3.1 kV and 40 V, respectively. For the negative electrospray mode, the capillary voltage and cone voltage were set to -2.5 kV and 40 V, respectively. The desolvation gas was set to 800 L/h at a temperature of 350 °C, the cone gas was set to 50 L/h, and the source temperature was set to 120 °C.

### Animal testing

7- to 9-week-old BALB/c-nu Slc mice were obtained from SLC, Inc. (Shizuoka, Japan) and used. A549-luc cells (#JCRB1414) or SNAI1 shRNA-mediated knockdown A549-luc cells were injected at 3-4.5 × 10⁶ cells through the tail vein of mice. After 3 to 4 weeks, metastasis was monitored by bioluminescence imaging analysis. More specifically, after injecting D-luciferin (75 mg/kg) (PerkinElmer, 122799) into the abdominal cavity of the mouse, images were obtained using an IVIS imaging system (IVIS 200, PerkinElmer, Waltham, MA, USA) in the supine position.. Lung tissue was obtained for histopathological analysis. Images were analyzed using commercially available software (ImageScope; Aperio Technologies). In the STO-609 efficacy test, STO-609 (25 mg, 100 mg/kg/day) was orally administered to mice on the day of injection. In the BMS303141 efficacy test, BMS303141 (100 mg/kg/day) was orally administered to mice on the day of injection.

### Luciferase reporter assay

The HBEC-KL cell line stably expressing the TFEB reporter stably expressing the CLEAR motif-driven luciferase reporter construct were obtained through puromycin selection after Lenti-5x CLEAR Rluc-CMV-mCherry-T2A-Puro-expressing lentiviral particles were transduced, After each treatment, the luciferase activity was confirmed using the Renilla Luciferase Assay System (Promega, E2810). To compare the TFEB reporter activity with other cell lines (HBEC30KT, HBEC-K, and HBEC-KL), the pGL4.70 5X CLEAR Rluc and pGL4.14 Fluc vectors were transiently co-transfected and normalized luciferase activity was measured in Dual -Measured by Luciferase^{®} Reporter Assay System (Promega, E1910).

### ROS assay

Intracellular ROS was detected using CM-H2DCFDA (Thermo Fisher Scientific, C6827). Specifically, after inoculating 5,000 cells for each cell line, 1 day later, the cells were stained with CM-H2DCFDA at 37 ° C. for 30 minutes, and the medium was replaced with EBSS. Fluorescence was detected at Ex/Em = 485/535 nm using a Varioskan Flash Spectral Scanning Multimode Reader (Thermo Fisher Scientific 4.00.53). Background was corrected for all readings, and the values of each sample were determined and normalized to the CTG assay values. Mitochondrial ROS was detected using the MitoSox Mitochondrial Superoxide indicator (Thermo Fisher Scientific, M36008). 3,000 to 7,000 cells per each cell line were initially inoculated, and after 1 day, the cells were cultured in MitoSOX at 37° C. for 20 minutes, and then the medium was replaced with EBSS. Fluorescence was detected at Ex/Em = 510/580 nm using a Varioskan Flash Spectral Scanning Multimode Reader (Thermo Fisher Scientific 4.00.53). Background was corrected for all readings, and the values of each sample were determined and normalized to the CTG assay values.

### Multiplex fluorescent immunohistochemistry

Human TMA paraffin sections of human lung adenoma tissue microarrays from 92 lung cancer patients were purchased from US Biomax. For immunohistochemical analysis, paraffin was removed from the paraffin sections, rehydrated, and antigen retrieval was performed with boiled 10 mM sodium citrate pH6. Epitope retrieval was performed using the BOND Epitope Retrieval Solution 2 kit (Leica Biosystems, AR9640). Immunofluorescence signals were visualized using OPAL 7-Color automation IHC kit (Akoya, NEL82100KT), TSA dyes 520 (LAMP2, Rabbit polyclonal anti-LAMP2 Atlas Antibodies Cat# HPA029100: RRID: AB_10795022, 1:1200), 690 (Acetyl lysine, Rabbit polyclonal anti-acetyl lysine Immunofluorescence signal visualization using Cell Signaling Technology Cat#9441: RRID: AB_331805, 1:400), 620 (Snail, Rabbit polyclonal anti-Snail Atlas Antibodies Cat#HPA069985: RRID: AB_2732146, 1:300) and spectral DAPI. Stained slides were covered with HIGHDEF^{®} IHC fluoromount (Enzo, ADI-950-260-0025) and scanned using the Vectra^{®} 3.0 Automated Quantitative Pathology Imaging System (PerkinElmer). To obtain image data, color separation, cell segmentation, and cell phenotyping were performed using inForm Advanced image analysis software (version 2.2, PerkinElmer). For analysis, a representative value was calculated by multiplying the median intensity of cells classified as positive for each marker by the percentage.

### Normalization and preprocessing of public gene expression data

After directly downloading the gene expression of the TCGA (The Cancer Genome Atlas) LUAD (Lung Adenocarcinoma) sample using the TCGAbiolinks R package, the FPKM value was transformed with log2 (FPKM + 1), and the sample was converted to z-score. Gene expression microarray datasets (GEO accession GSE41271, GSE72094) were downloaded and log2-transformed.

### Public cancer cell line data

Mutation and metabolome data of lung adenocarcinoma cell lines from the Cancer Cell Line Encyclopedia (CCLE) were downloaded from the CCLE data portal (https://portals.broadinstitute.org/ccle). A cell line comprising a missense mutation in KRAS and a non-silent mutation in LKB1 was termed "KL cell line". Cells comprising a KRAS mutation but no mutation in LKB1 were termed "K cell lines".

### Statistical analysis

All statistical experiments were performed using R statistical software (ver. 4.0.0). A two-sided Student's t-test was performed. A Spearman rank correlation test was performed to evaluate the relationship between the two variables.

### Prediction of transcriptional tensile activity

Only genes showing a significant correlation (P<0.05, spearman rank correlation test) with the expression of each transcription factor in TCGA-LUAD were considered targets. Genes with positive correlations were considered up-regulated, and genes with negative correlations were considered down-regulated. For each sample, a modified version of gene-set enrichment analysis (single-sample GSEA, ssGSEA) was performed with target genes of transcription factors. The activity of each transcription factor was calculated by subtracting the ssGSEA score of the downregulated gene from the ssGSEA score of the upregulated gene.

### Survival analysis

Clinical data were collected from the LUAD cohort (TCGA, GSE41271, GSE72094). Based on the gene expression level or ssGSEA score for each sample, the samples were divided into two groups, prognosis was predicted using the Kaplan-Meier survival rater, and significant differences were evaluated using the log-rank test. Hazard ratio (HR) was calculated to define the direction of prognosis, and a poor prognosis was predicted when the HR was greater than 0 and the gene expression or ssGSEA score was high.

For TMA survival analysis, total or nuclear Snail protein expression by immunohistochemistry was ranked and used as a threshold value to determine low and high groups. The difference in overall survival between the low and high expression level groups was evaluated by the log-rank test.

### Snail-induced cell invasiveness and increased anticancer treatment resistance in KRAS and LKB1 co-mutated gastric cancer cell lines

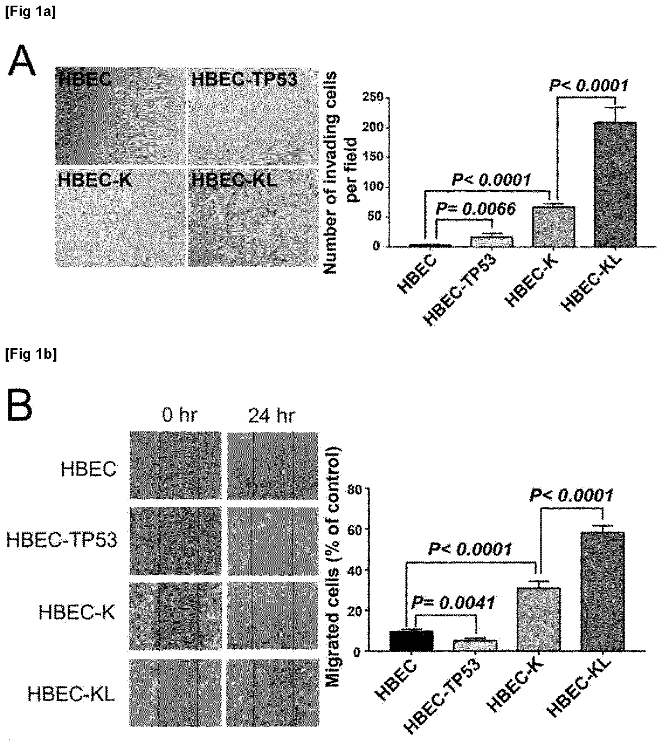

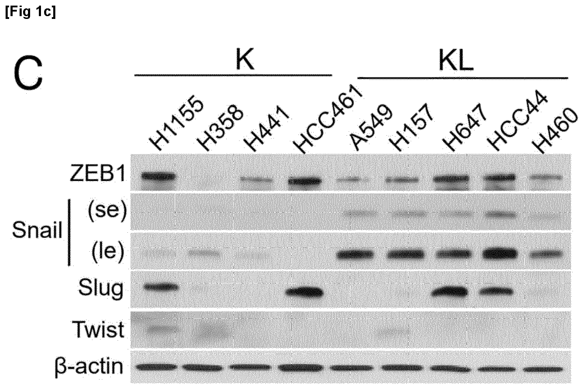

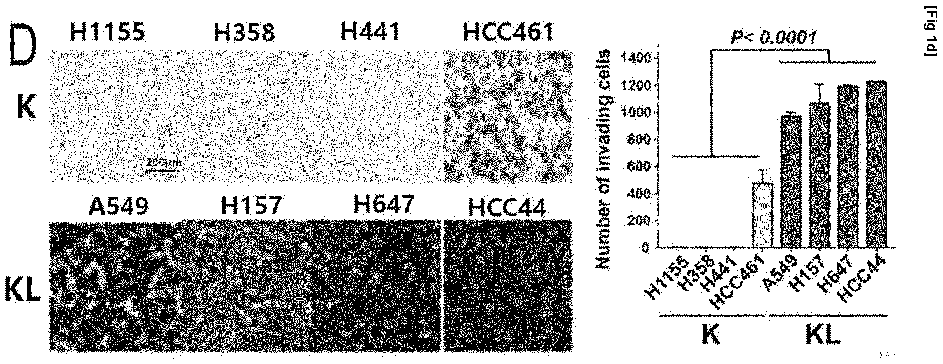

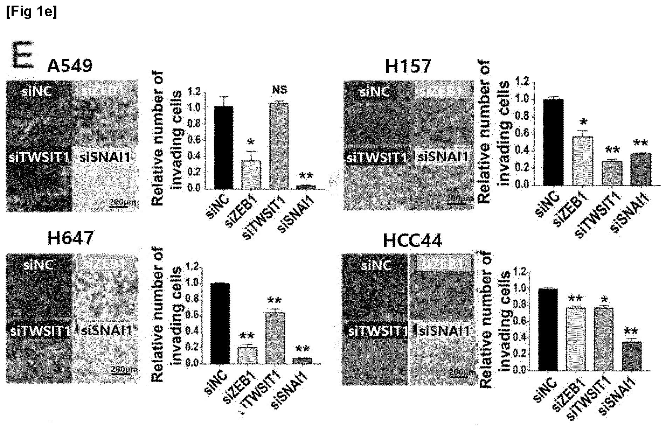

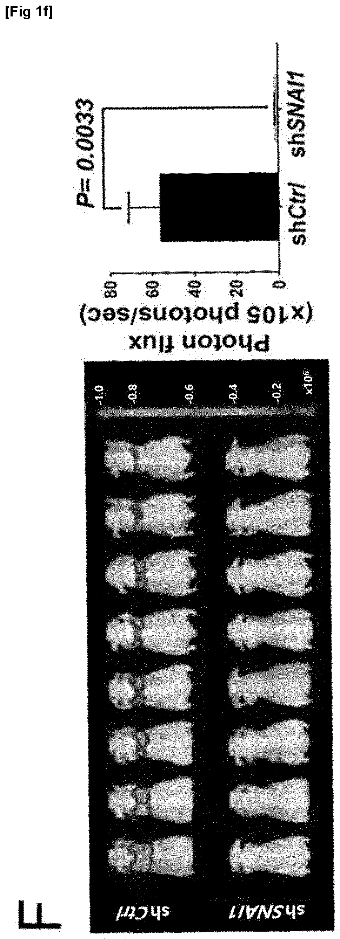

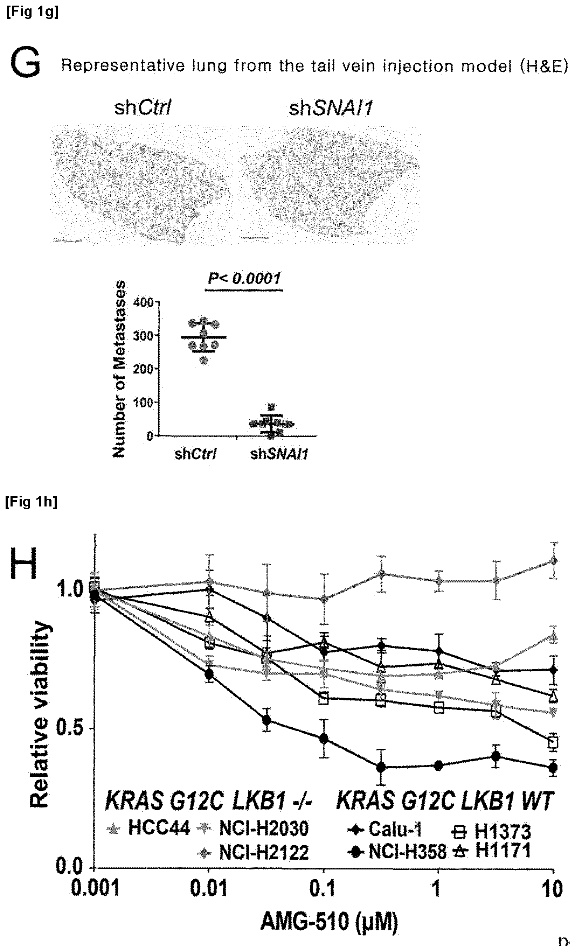

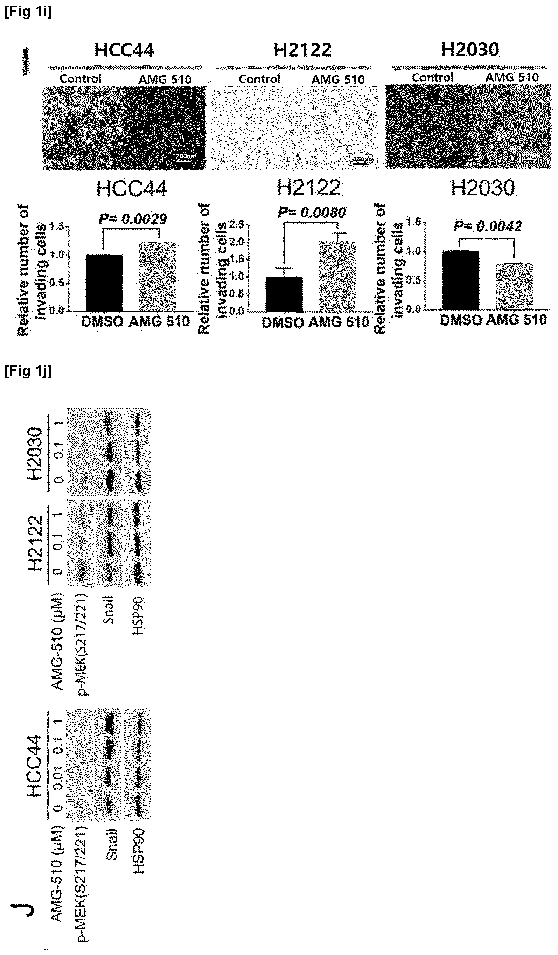

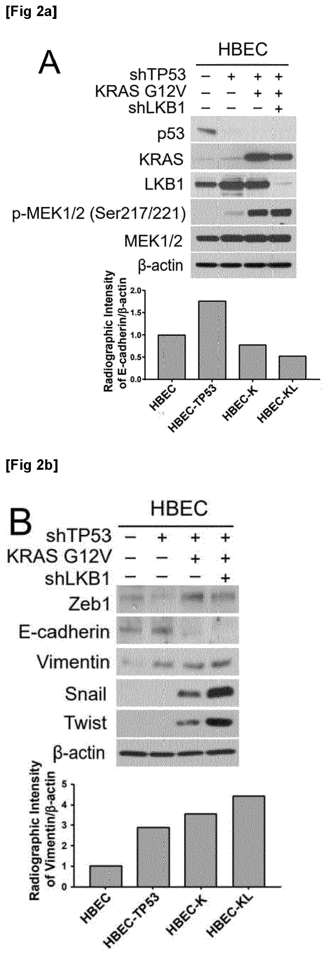

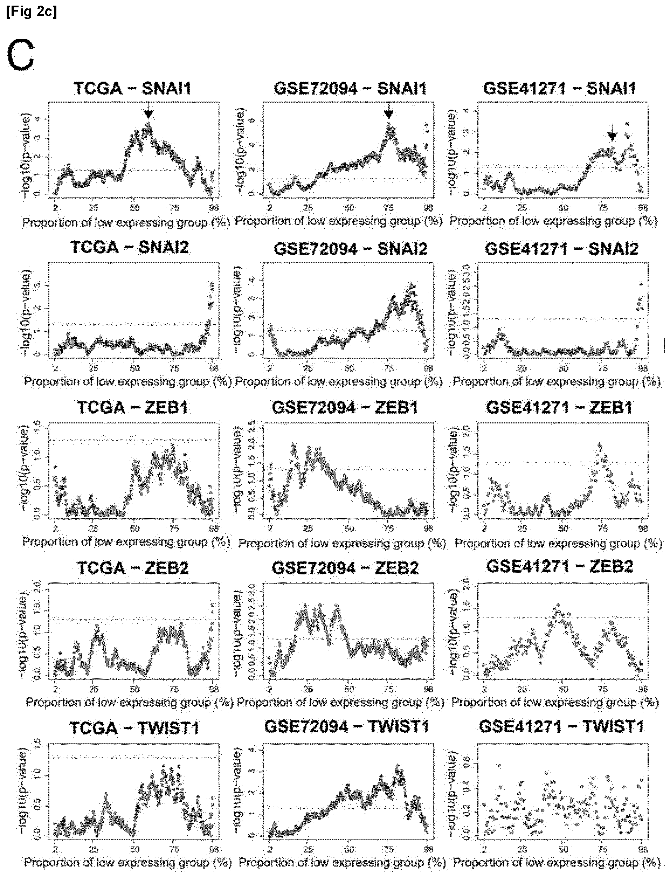

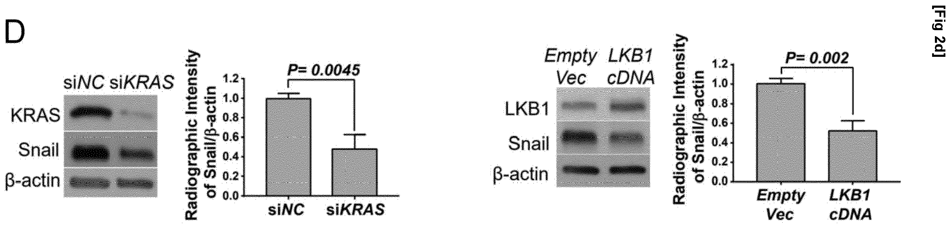

After inducing cancer progression from normal human bronchial epithelial cells (HBECs) through inhibition of p53 (HBEC30KT-shTP53; HBEC-TP53 cells), KRAS G12V expression (HBEC30KT-shTP53/KRAS G12V; HBEC-K cells) and inhibition of LKB1 (HBEC30KT-shTP53/KRAS G12V/shLKB1; HBEC-KL cells) (FIG. 2A), as a result of confirming the expression level of EMT (epithelial to mesenchymal transition) subtype-related proteins in various lung cancer cell lines, in the gastric cancer cell line (HBEC-KL cells) with mutations in both KRAS and LKB1, the epithelial marker E-cadherin decreased while the mesenchymal marker vimentin level increased (Fig. 2B), corresponding to the EMT subtype, and cell invasiveness and mobility increased (Figs. 1A and 1B). As a result of checking the expression levels of Snail, Slug, Zeb1 and Twist, known as EMT transcription factors in lung cancer cell lines KRASmut (K; NCI-H1155, NCI-H358, NCI-H441, HCC461) and KRASmut/LKB1mut (KL; A549, NCI-H157, NCI-H647, HCC44, NCI-H460), it was confirmed that the expression level of Snail increased the most in the KL cell line and the HBEC-KL cell line compared to the K cell line and the HBEC-K cell line (FIG. 2B, 1C). There was also a significant difference in the degree of cell invasiveness between the K cell line and the KL cell line, which can be seen to be correlated with the difference in Snail expression level (FIG. 1D). Relatedly, expression levels of SNAI1 target genes showed poor prognosis with respect to survival in independent cohorts, while other transcription factors showed inconsistent or opposite results (Fig. 2C). Further experiments revealed that both oncogenic KRAS and LKB1 deficiency are essential for maintaining Snail expression levels (Fig. 2D). In addition, it was confirmed that the invasiveness of various KL cell lines was significantly inhibited upon knockdown of SNAI1 (FIG. 1E). In a cancer metastasis mouse model in which SNAI1 knockdown A549 cells were injected through the tail vein, cancer metastasis was reduced compared to the control group injected with an empty vector, suggesting that Snail promotes cancer metastasis *in vivo* (Fig. 1F, 1G).

In order to confirm whether KRAS and LKB1 mutations induce resistance to anticancer treatment, the results of confirming the resistance of three KL NSCLC cell lines having KRAS-G12C mutation to anticancer treatment (KRAS(G12C) inhibitor AMG-510) showed that in the case of the H2122 cell line, complete resistance was exhibited even at high concentration (10 umol/L), and in the case of the other HCC44 and H2030 cell lines, the cell growth inhibitory effect was only insignificant (FIG. 1H). When treated with AMG-510, not only was the therapeutic effect insignificant, but in two of the three KL cell lines, Snail expression level and cell invasiveness also increased (FIGS. 1L and 1J).

Through this, it was found that KRAS (G12C) inhibitors showed limited therapeutic effects in KRAS and LKB1 mutant cancers and promoted cancer metastasis. In addition, it was found that increased Snail expression levels in KRAS/LKB1 mutant lung cancer cells increased cell invasiveness and induced resistance to anticancer treatment.

### Increased Snail stability and invasiveness by increased ROS in KRAS/LKB1 co-mutant cancer cells

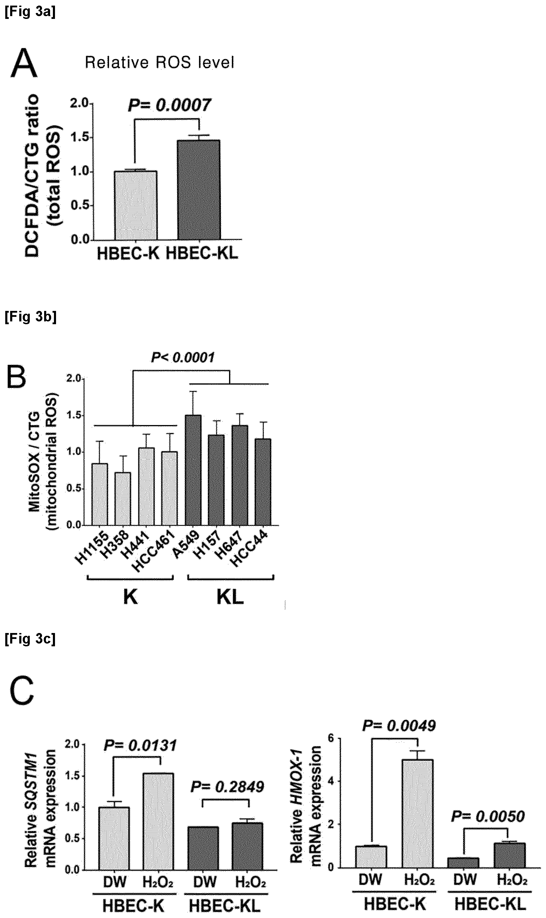

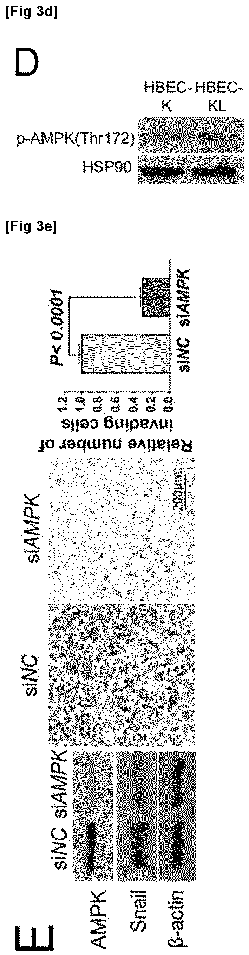

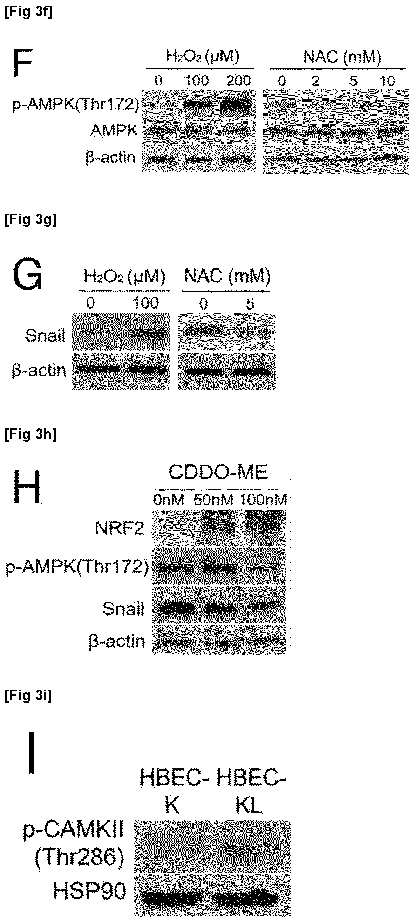

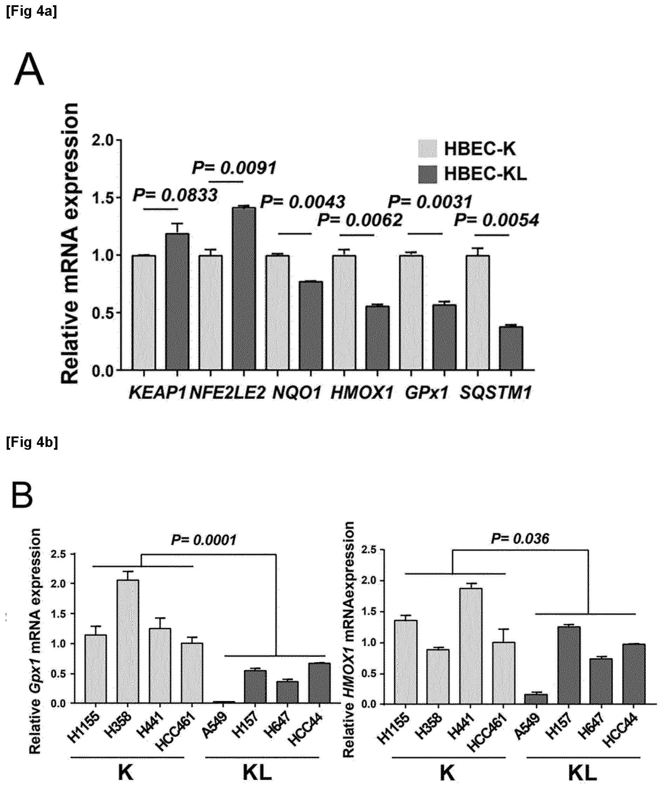

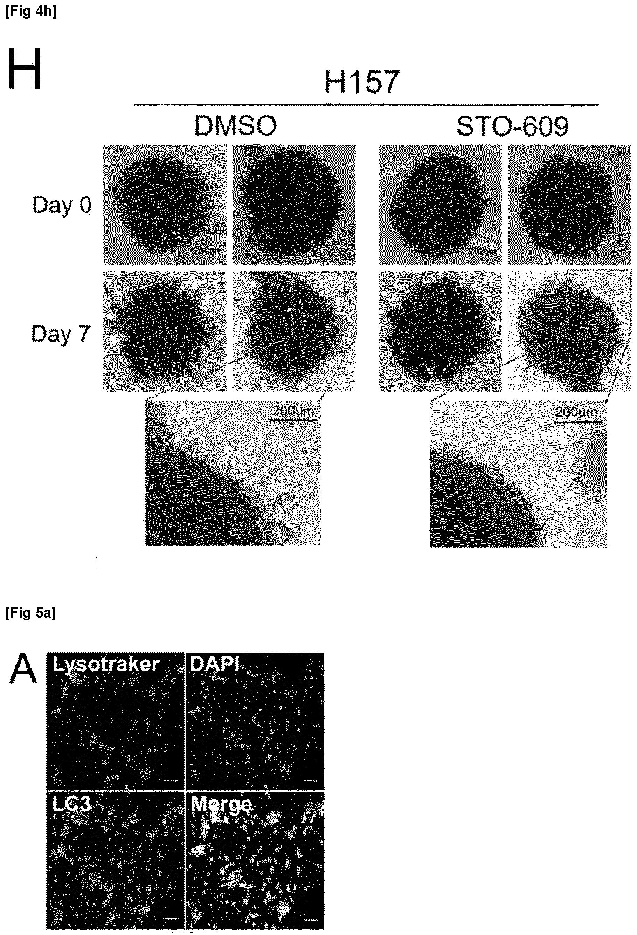

As a result of comparing ROS expression levels in K (KRASmut, wild-type LKB1), HBEC-K (KRASmut, P53mut, wild-type LKB1), KL (KRASmut, wild-type LKB1) and HBEC-KL (KRASmut, P53mut, LKB1mut) cell lines, KL and HBEC-KL cell lines showed higher expression levels of ROS compared to HBEC-K and K cell lines, (FIGS. 3A and 3B), but it was confirmed that the expression level of antioxidant genes was significantly reduced (FIG. 4A, 4B). Since an increase in oxidative stress activates AMPK and limits mitochondrial ROS production, as a result of confirming whether an increase in ROS level activates AMPK in KL lung cancer cell lines, it was confirmed that the expression level of phospho-AMPK T172 was increased in HBEC-KL cells (FIG. 3C). In addition, it was confirmed that invasiveness was reduced upon AMPK knockdown through siRNA (FIG. 3D), and that ROS-induced AMPK activation in HBEC-KL cells increased invasiveness. As a result of confirming whether the regulation of steady-state ROS levels in KL cells affects AMPK activation, it was found that AMPK phosphorylation and Snail expression were increased by H2O2 treatment, at T172, but AMPK phosphorylation and Snail expression were decreased by N-acetyl cysteine (NAC) treatment (FIGS. 3E and 3F).

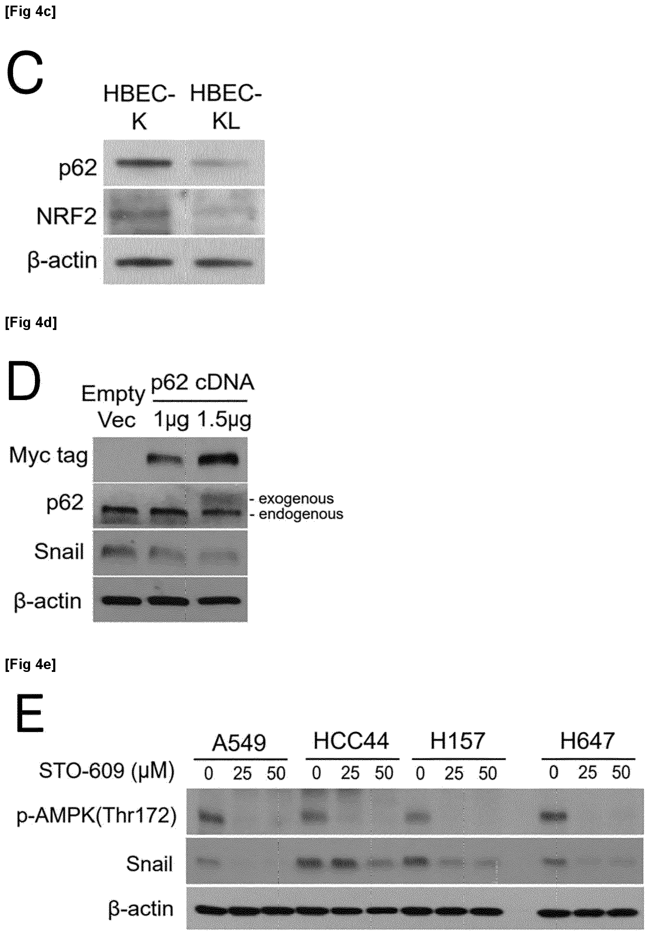

Under non-stress conditions, Kelch-like ECH-associated protein 1 (Keap1) acts like an adapter for the E3 ubiquitin ligase, and NF-E2-related factor 2 (NF-E2-related factor 2; Nrf2) promotes proteosomal degradation. On the other hand, under oxidative stress conditions, increased p62 competes with Nrf2 for Keap1 binding, stabilizing the transcriptional activity of Nrf2 and its target genes. However, in the KL cell line, p62 was not induced under oxidative stress conditions (Fig. 3G left), and thus the induction of antioxidant enzymes was not reduced (Fig. 3G right). In this regard, treatment with CDDO-ME, a KEAP1 antagonist, reduced the level of phospho-AMPK T172 due to NRF2 activation (Fig. 3H). In addition, Snail expression was reduced by p62 expression in KL cell lines (Fig. 4C). From this, it was found that inhibition of p62 in the KL cell line maintains a high level of ROS in a steady state and causes damage to the NRF2-dependent antioxidant response.

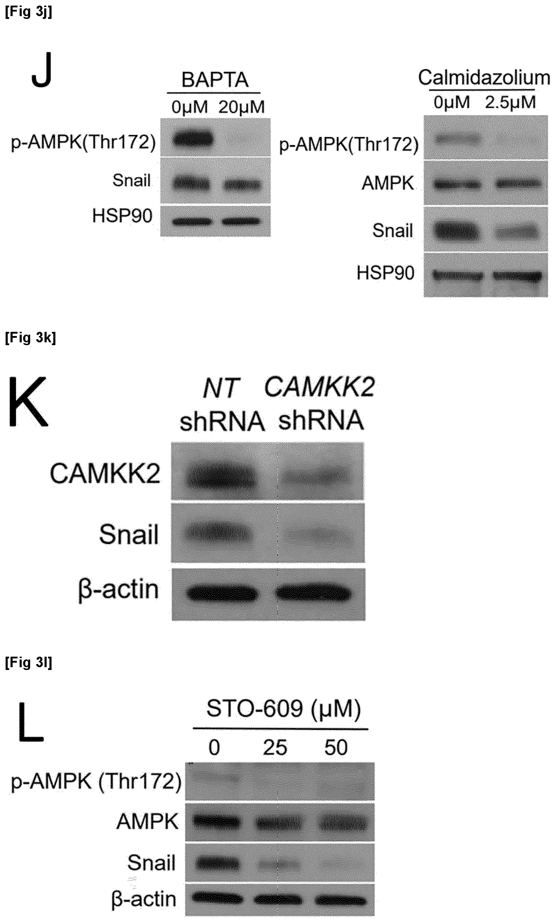

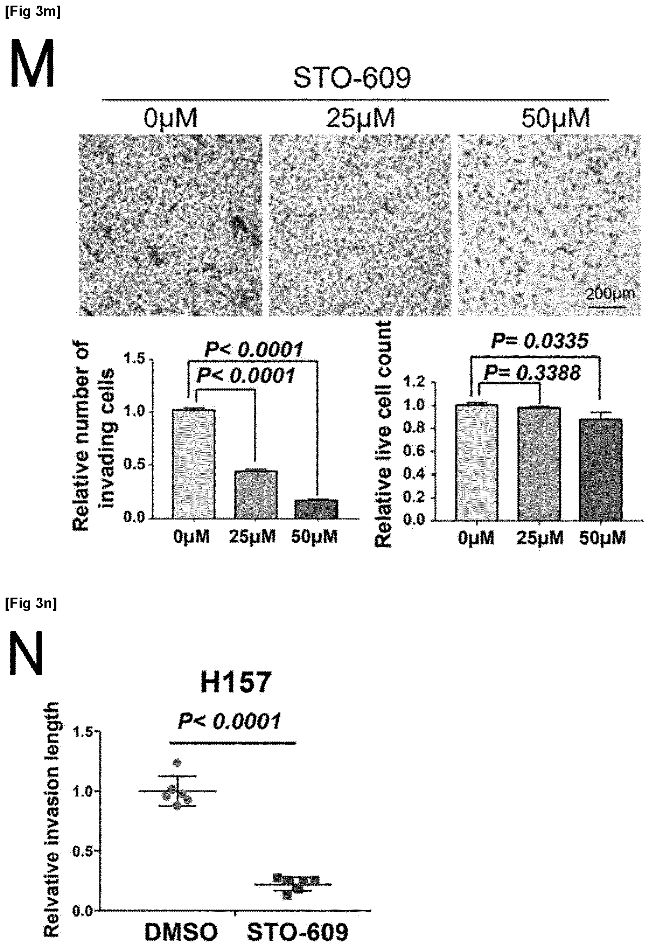

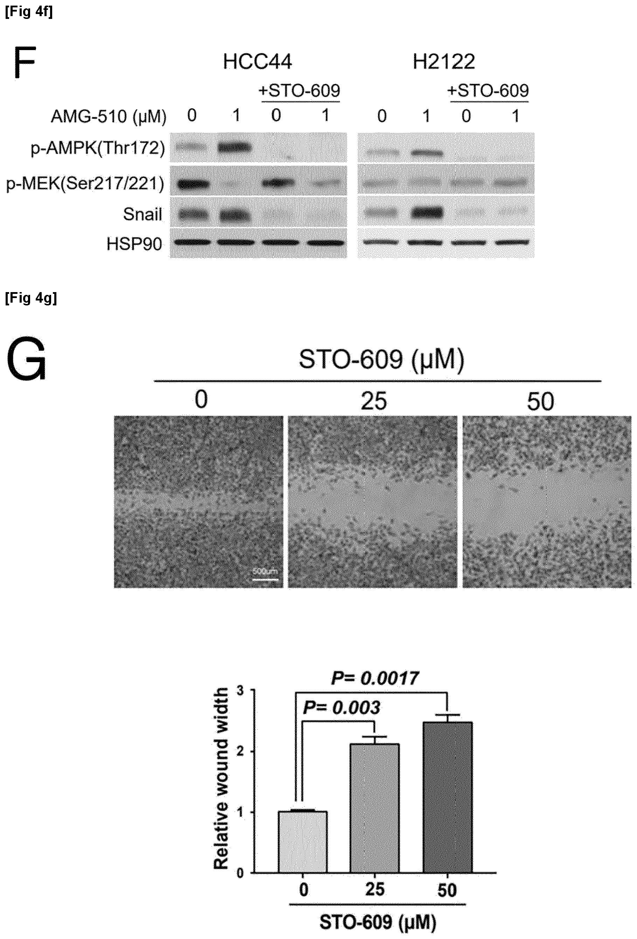

As it is known that AMPK activation in cellular energy-stress is mediated by LKB1, whereas ROS-induced AMPK activation is achieved by an increase in intracellular calcium through CAMKK activation, as a result of confirming whether CAMKK activation induces AMPK activation in KL cells, it was confirmed that the level of phospho-CAMKII T286, which can be seen as a biomarker for intercellular calcium levels, was increased in HBEC-KL cells (FIG. 3I). In addition, it was confirmed that inhibition of calcium signal by BAPTA, a calcium chelator, and calmidazolium, a calmodulin inhibitor, reduced AMPK phosphorylation and Snail expression levels (FIG. 3J). Moreover, it can be seen that genetic and chemical CAMKK2 inhibition in HBEC-KL and KL cells reduced phospho-AMPK T172 and Snail expression levels as did ROS or calcium signal inhibition (Fig. 3K, 3L, 4D). In addition, it was confirmed that the abnormal increase in Snail expression level by AMG-510 was reduced by STO-609 (FIG. 4E). Eventually, it was confirmed that 2D and 3D invasiveness and cell mobility were reduced upon inhibition of CAMKK (FIG. 3M, 4F, 4G, 3N).

From this, it was found that the increase in ROS level due to the impaired antioxidant defense ability in KL lung cancer cells prolongs the activation of the ROS/Ca2+-CAMKK-AMPK pathway, thereby increasing the expression level of Snail and also increasing cell invasiveness.

### Increased invasiveness by activation of CAMKK-AMPK-dependent autophaqolysosome through stabilization of Snail protein independent of GSK3β

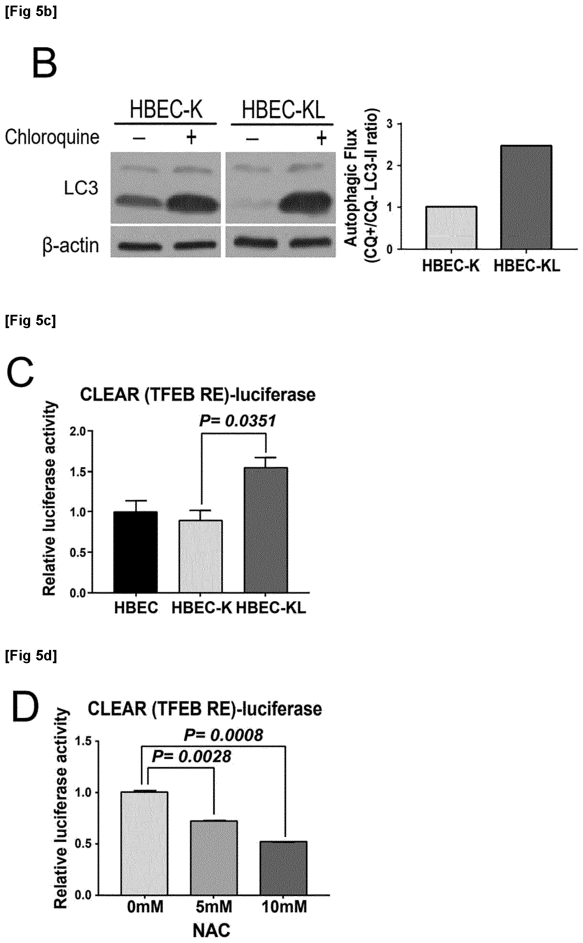

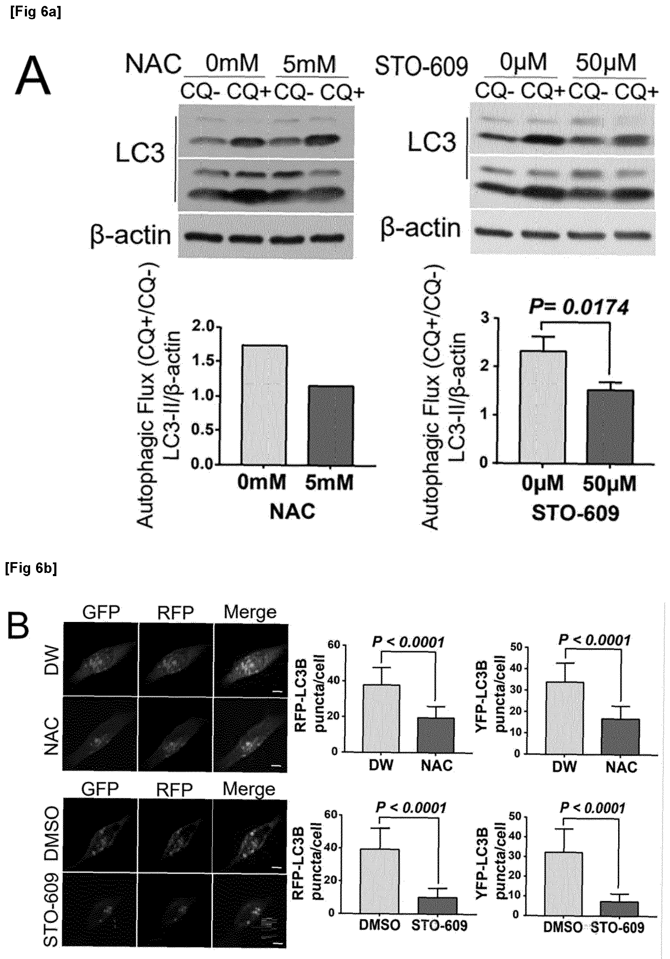

81% of LysoTracker-positive cells in HBEC-KL cells co-localized with LC3 (FIG. 5A), indicating that the majority of the cells were autophagolysosomes. In fact, as a result of inhibiting lysosomal function with chloroquine (CQ), high levels of LC3-II were accumulated (FIG. 5B), suggesting that HBEC-KL cells have a high standard of basal autophagic flux. It was confirmed that the autophagic flux in KL cells was increased by ROS/CAMKK signaling, and it was confirmed that it decreased upon treatment with NAC (ROS scavenger) and STO-609 (CAMKK inhibitor) (FIG. 6A, 6B).

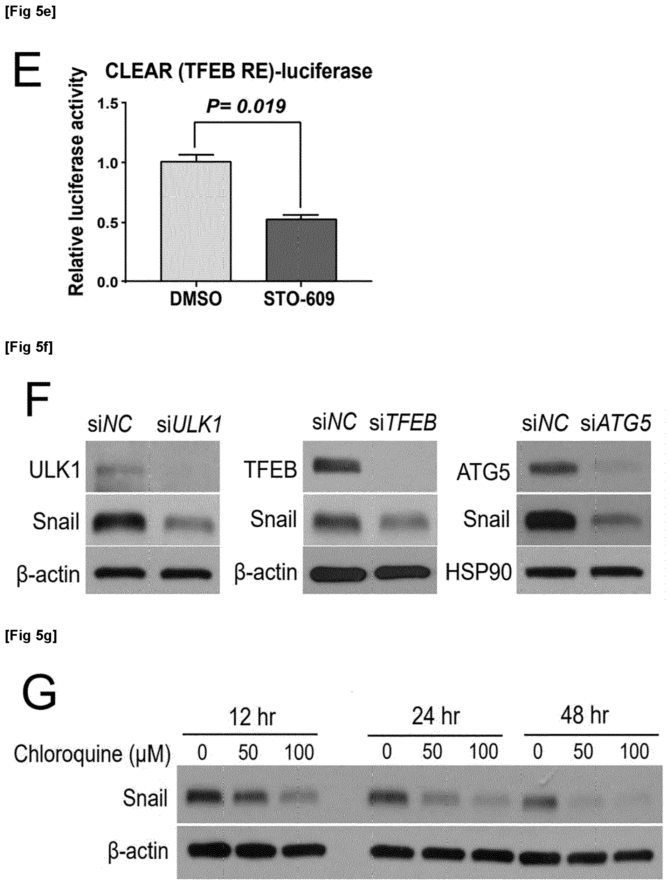

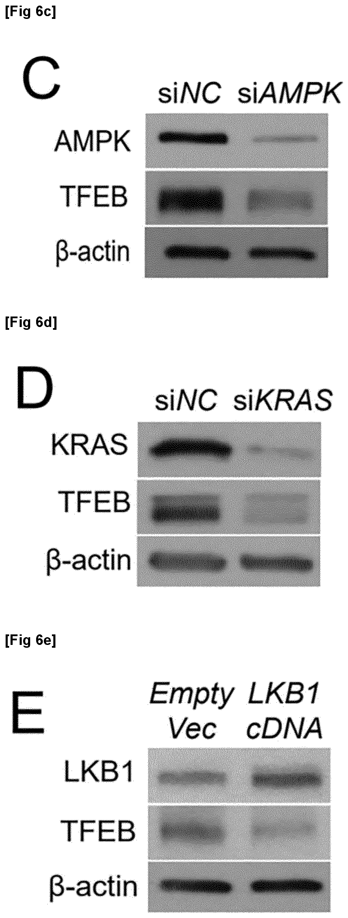

It was confirmed that at least part of the autophagic flux in the HBEC-KL cell line was due to increased CLEAR activity (FIG. 5C). Transcription factor EB (TFEB) corresponds to the main regulator of the CLEAR network, and it was confirmed that TFEB was reduced upon genetic knockdown of PRKAA1 (AMPKa1) (Fig. 6C), and that the proto-oncogenic mutations of KRAS and LKB1 are required to maintain TFEB levels (Fig. 6D, 6E). In addition, it was confirmed that CLEAR activity was reduced upon inhibition of ROS and CAMKK (FIGS. 5D and 5E). Through this, it was found that the activated ROS/Ca2+-CAMKK-AMPK signaling pathway activates autophagy through TFEB-mediated activation of the CLEAR network.

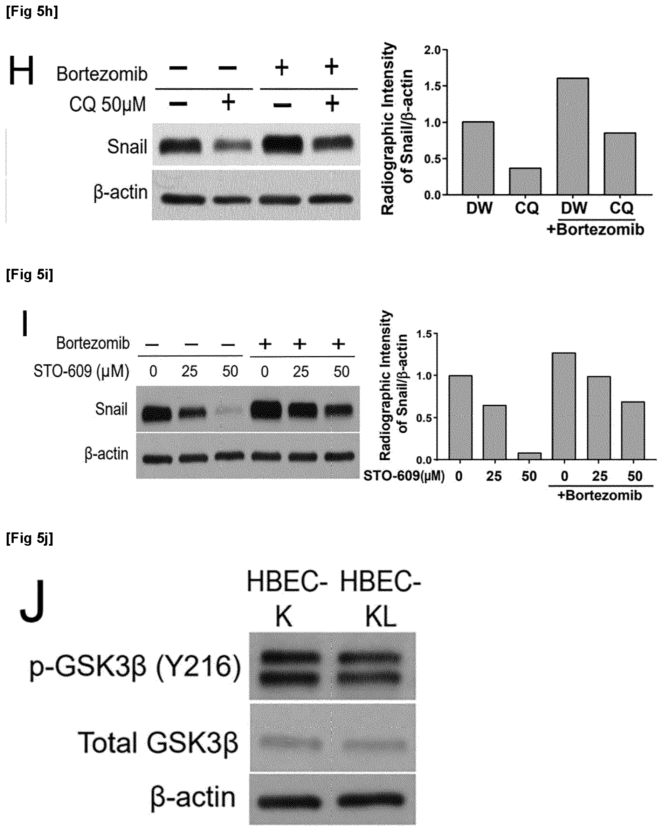

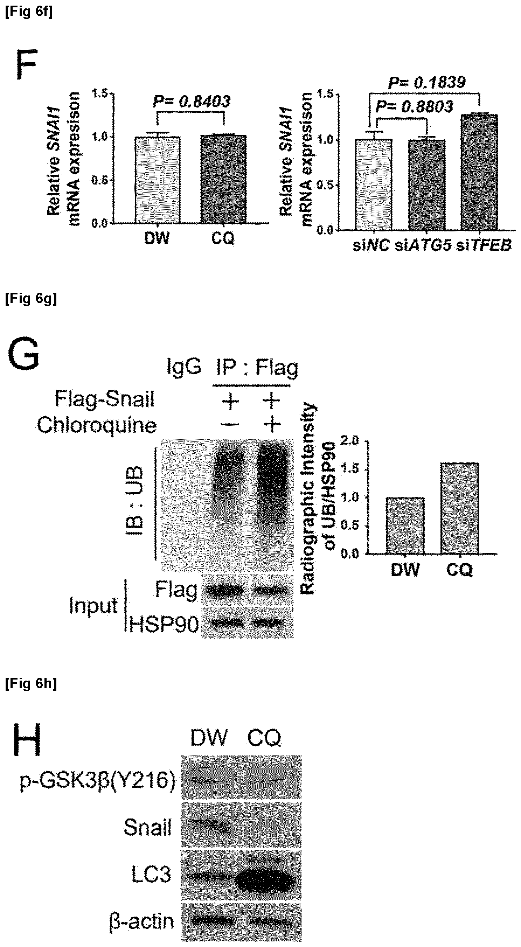

Next, upon genetic knockdown of ULK1, ATG5, and TFEB, which are critical regulators of autophagy, or inhibition of autophagy by chloroquine (CQ), it was confirmed that although the expression level of Snail protein was reduced (Fig. 5F, 5G), there was no change in mRNA expression level (Fig. 6F). Through this, it was found that autophagy stabilizes Snail. On the other hand, the reduction of Snail by chloroquine (CQ) or STO-609 was restored when bortezomib, a proteasome inhibitor, was also treated (FIG. 5H, 5I). Phosphorylation of ser11, ser100 and ser96 of Snail enables proteasome degradation by ubiquitination, and the degree of autophagy inhibition was increased upon Snail ubiquitination, and it was found that the stability of Snail protein changed the degree of autophagy inhibition (FIG. 6G).

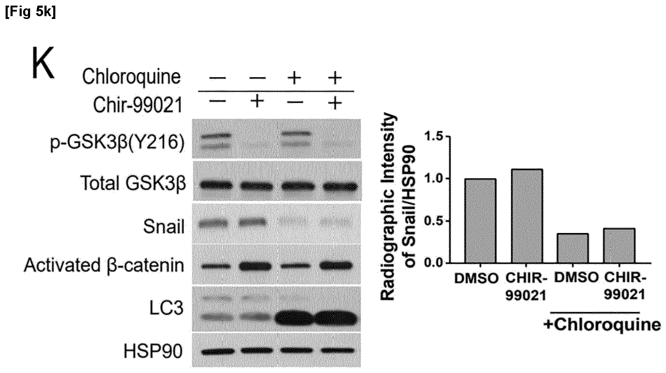

Snail phosphorylation is regulated by GSK3β, and whether the stability of Snail protein regulated by autophagolysosomes depends on GSK3β activity was confirmed. Expression levels of total GSK3β and activated GSK3β (phospho-GSK3β Y216) were not increased in KL cells (Fig. 5J), indicating that KRAS/LKB1 co-mutation did not affect GSK3β activity. In addition, CHIR99021, a GSK3β inhibitor, restored the expression levels of β-catenin and other phosphorylation substrates under conditions of autophagy inhibition, but did not restore the levels of Snail protein (FIG. 5K). In addition, phospho-GSK3β Y216 expression did not increase the degree of autophagy inhibition (Fig. 6H). Through this, it was found that Snail protein stability by the autophagosome was not dependent on GSK3β activation.

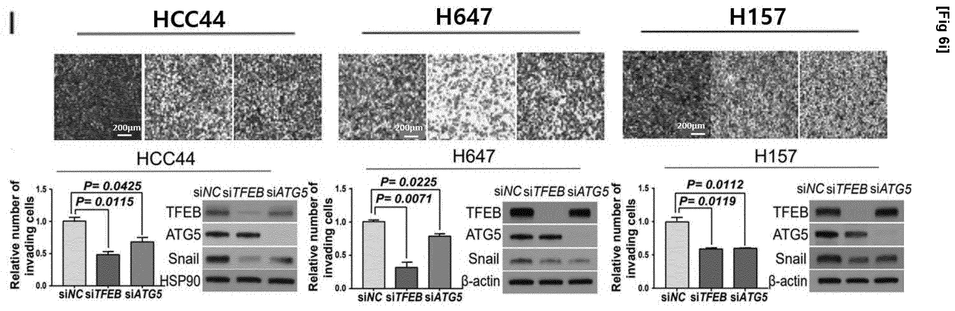

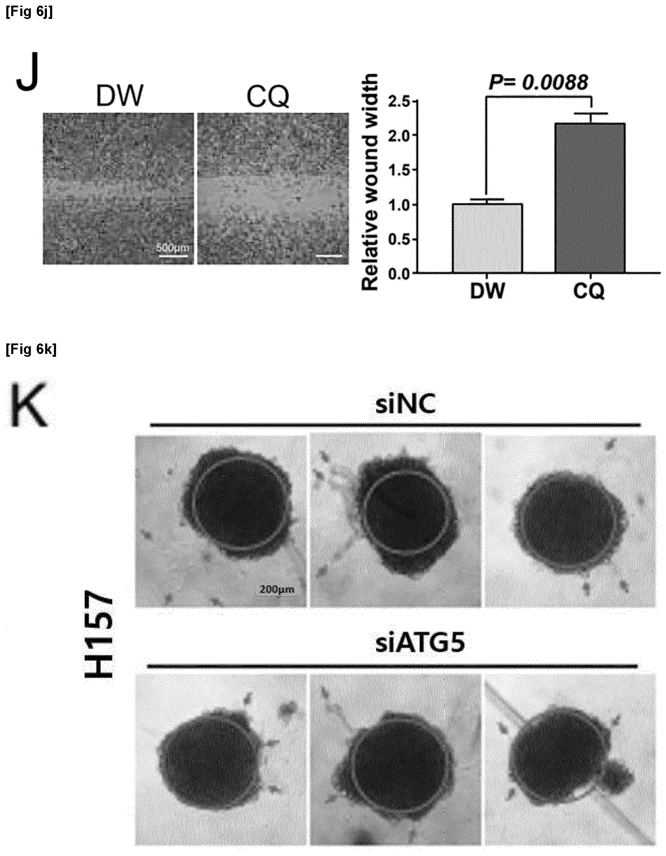

As a result of inhibiting autophagy through various drugs or genetic knockdown in HBEC-KL cell line and KL cell line in order to confirm whether the role of promoting cancer growth in the late stage of autophagy is due to invasiveness through EMT promotion and Snail stabilization, cell invasiveness and migration were reduced (Fig. 5L, 5M, 6I, 6J, 6K, 5N), and as a result of activating autophagy in the HBEC-K cell line through the mTOR inhibitor rapamycin, permeability increased (FIG. 5O). Through this, it was found that autophagy is essential for the invasiveness of cancer cells. In addition, it was found that the CAMKK-AMPK-dependent activation of autophagy resolvers increases the invasiveness of KL cancer cell lines through Snail stabilization.

### Increased invasiveness according to acetyl-CoA increased by autophagy in KL cells

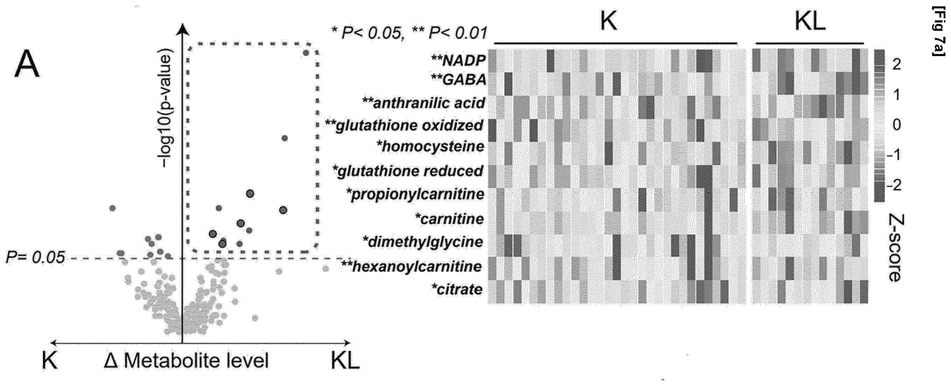

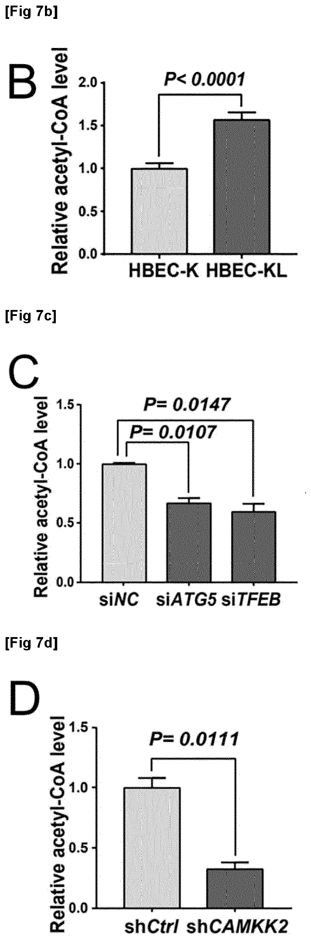

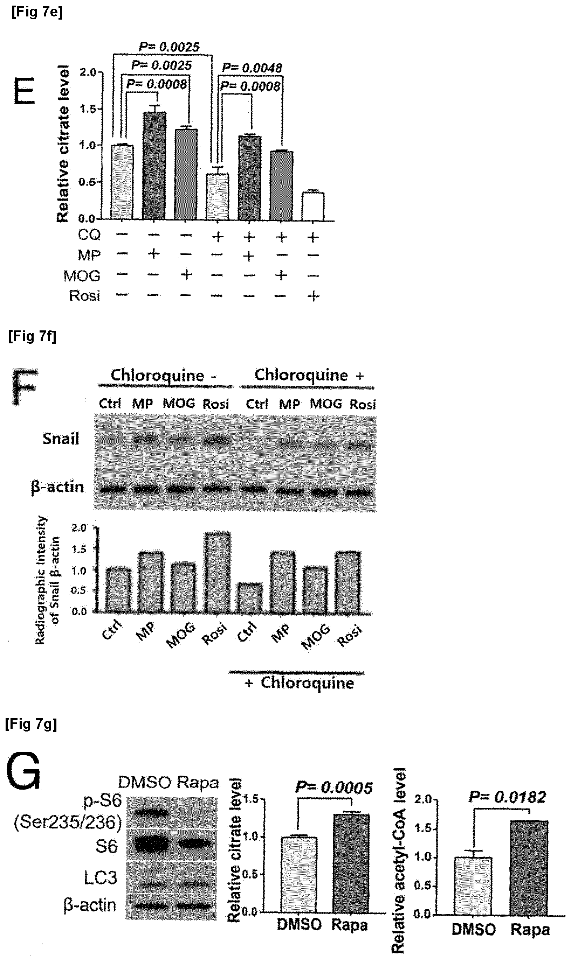

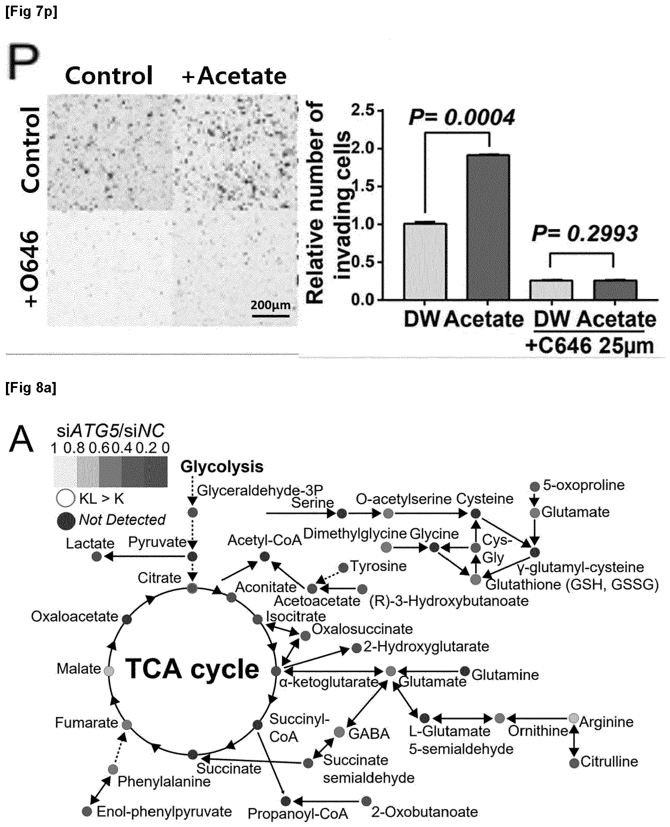

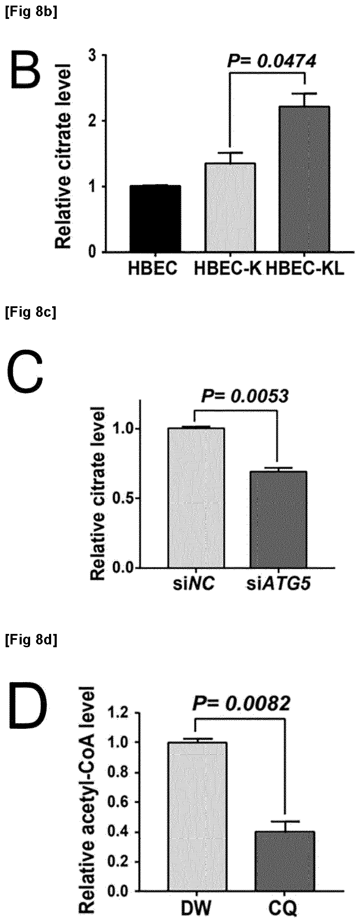

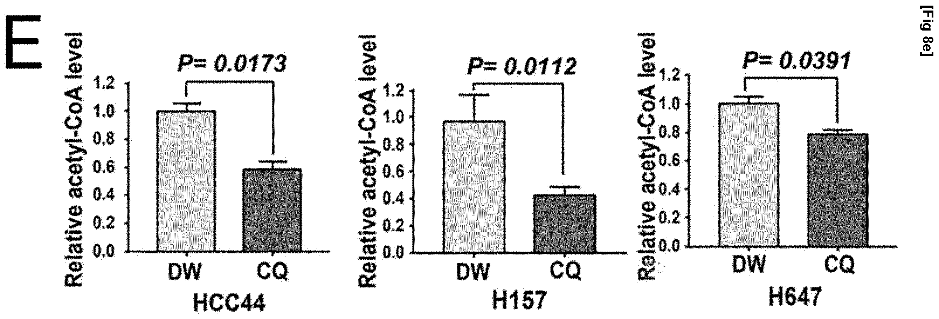

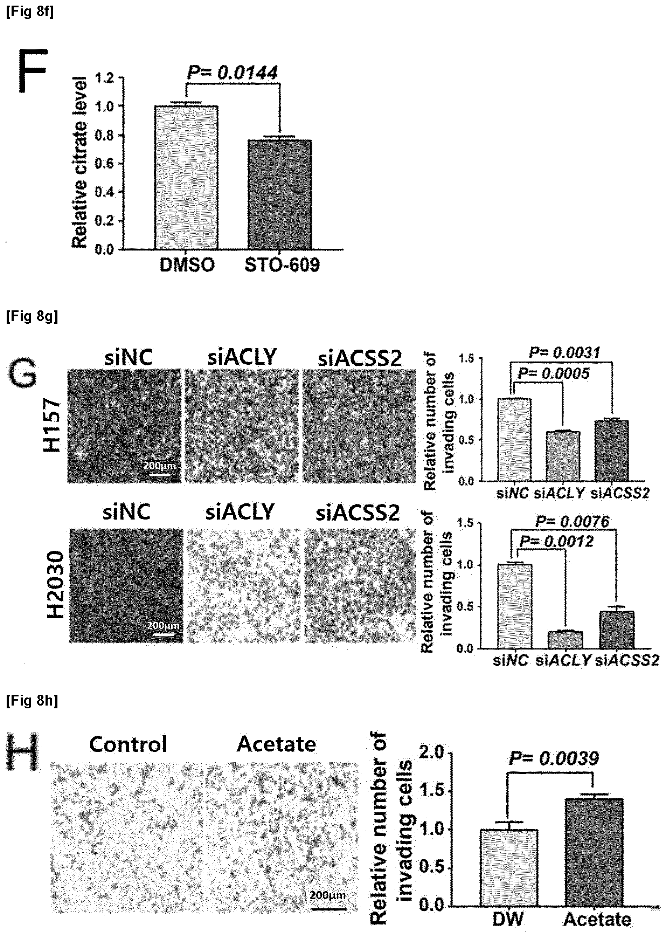

As a result of analyzing the CCLE metabolome dataset, it was confirmed that compared to K cells (N = 30), NADP, anthranillic acid, glutathione, hexanolycarnitine, GABA (gamma-Aminobutyric acid), homocysteine, citrate, carnitine, propionlycarnitine and dimethylglycine, among 225 metabolites, were highly expressed in KL cancer cell lines (N = 14). As a result of LC-MS (liquid chromatography and mass spectrometry) analysis, the expression levels of glutathione, GABA, citrate, and dimethylglycine among the 10 metabolites significantly decreased when autophagy was inhibited (Fig. 7A, 8A), and it was found that these metabolites were supplied by increased autophagy in KL cell lines. Glutathione is the main reducing power that maintains cellular redox homeostasis and is expected to play an important role in KL cancer cells with elevated ROS. In parallel, increased GABA and dimethylglycine will function through glutamate and glycine, which are precursors of glutathione, respectively. On the other hand, as citrate, a precursor of acetyl-CoA, is known to regulate invasion and metastasis in prostate cancer, breast cancer, and hepatocellular carcinoma, and as a result of confirming that the supply of autophagy-inducing citrate increases the level of acetyl-CoA in cells and thereby increases the invasiveness of KL cells the levels of citrate and acetyl-CoA were increased in HBEC-KL cells compared to HBEC-K cells (FIGS. 7B and 8B). In addition, it was confirmed that the expression levels of citrate and acetyl-CoA decreased upon inhibition of autophagy as well as inhibition of CAMKK in HBEC-KL cell lines and KL cell lines (FIGS. 8C, 7C, 8D, 8E, and 7D). Intracellular citrate is mainly synthesized from pyruvate and a-ketoglutarate, and delivery of their cell-permeable forms, methyl pyruvate (MP) and dimethyl-2-oxoglutarate (MOG), completely reverses the effect of chloroquine (CQ) on citrate and Snail levels (FIG. 7E, 7F). In the same vein, activation of autophagy by rapamycin in HBEC-K cells increased citrate and acetyl-CoA levels (Fig. 7G). Through this, it was found that KL cells maintain high levels of acetyl-CoA through the supply of citrate by activated autophagy.

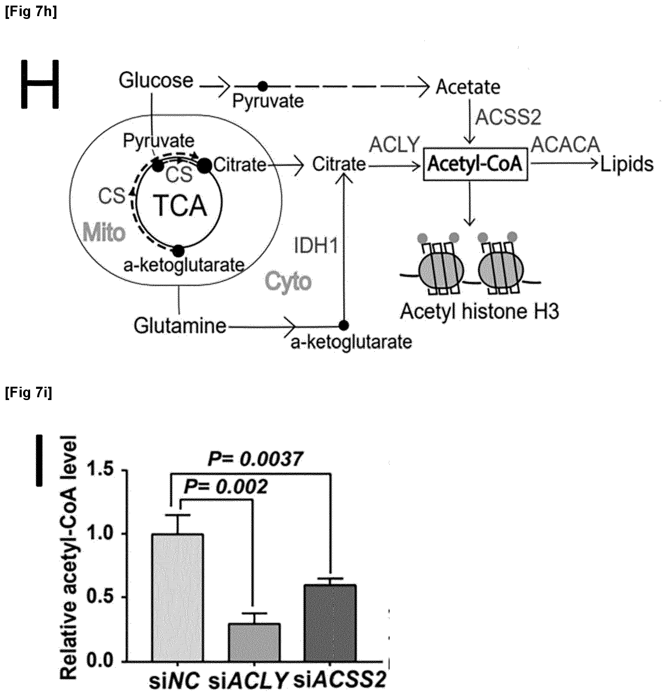

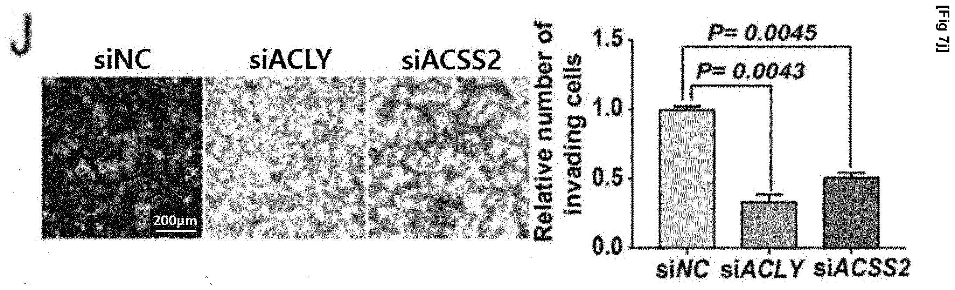

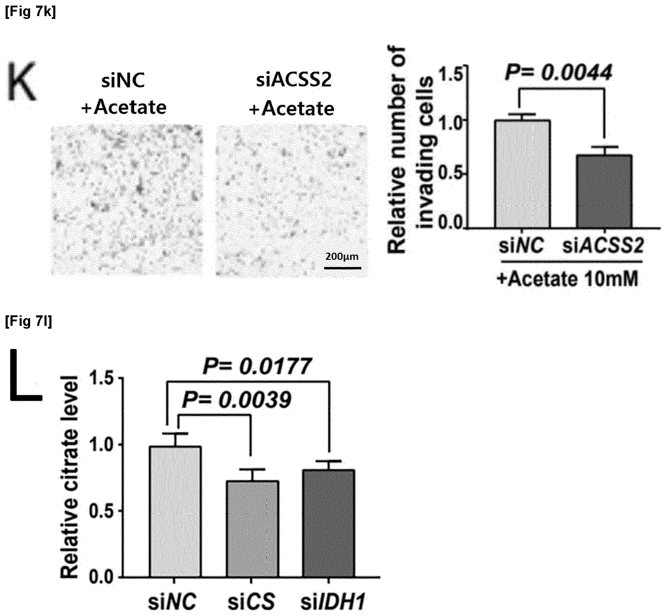

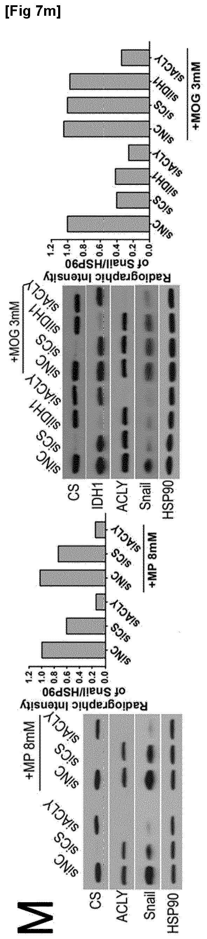

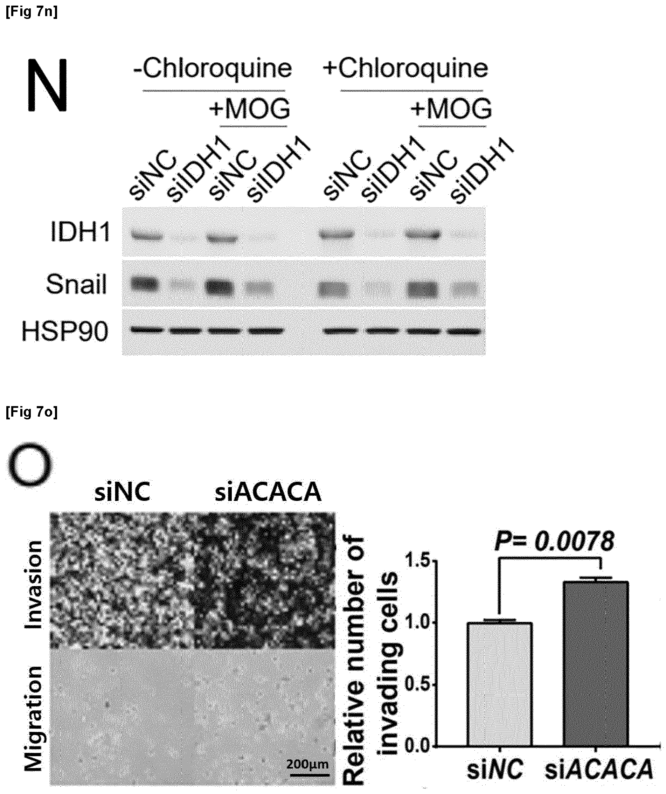

Citrate can be synthesized by two pathways: mitochondrial citrate synthase (CS) and cytosolic isocitrate dehydrogenase 1 (IDH1). Acetyl-CoA is synthesized from citrate by ATP citrate lyase (ACLY) or from acetate by Acetyl-CoA synthetase 2 (ACSS2). Acetyl-CoA is then used as a precursor to synthesize lipid substances or functions as an acetyl group donor for protein acetylation. In order to prove the correlation between citrate-acetyl-CoA and invasiveness of KL cells, the enzymes were knocked down with siRNA to confirm changes in invasiveness of KL cells. Acetyl-CoA levels were reduced upon ACLY and ACSS2 knockdown (FIG. 7I), and invasiveness was reduced in HBEC-KL and KL cells (FIGS. 7J and 8G). In KL cells, invasiveness increased when treated with acetate (FIG. 8H), whereas invasiveness decreased when ACSS2 knocked down (FIG. 7K), suggesting that acetate is converted to acetyl-CoA through ACSS2 to induce an invasive type. Moreover, siCS and silDH1 also reduced citrate levels to a similar extent (Fig. 7L), indicating that both the mitochondria-dependent pathway (CS) and the independent pathway (IDH1) act on the citrate pool in KL cells. The expression level of Snail protein was also decreased by siCS and silDH1 (Fig. 7M), whether MP, which acts as a direct substrate of CS, and MOG, which acts as a direct or indirect substrate of IDH1 and CS, could restore the reduced expression level of Snail was tested. As a result, while the Snail expression level decreased by siCS was not increased by MP, the expression level of Snail decreased by silDH1 was restored by MOG. As a result, it was found that MOG bypassed IDH1 through the mitochondrial TCA cycle, but MP was restricted to bypass CS (Fig. 7M). The same recovery pattern was observed in the chloroquine (CQ) treatment condition, and it was found that the CQ treatment condition did not significantly affect the mitochondria-dependent supply (FIG. 7N). However, Snail levels did not recover when ACLY was reduced in the MOG supplemented condition (Fig. 7M). As a result, it was found that both acetate and citrate act as sources of acetyl-CoA regardless of the pathway in KL cells, and acetyl-CoA acts as a key metabolite to increase invasiveness of KL cells.

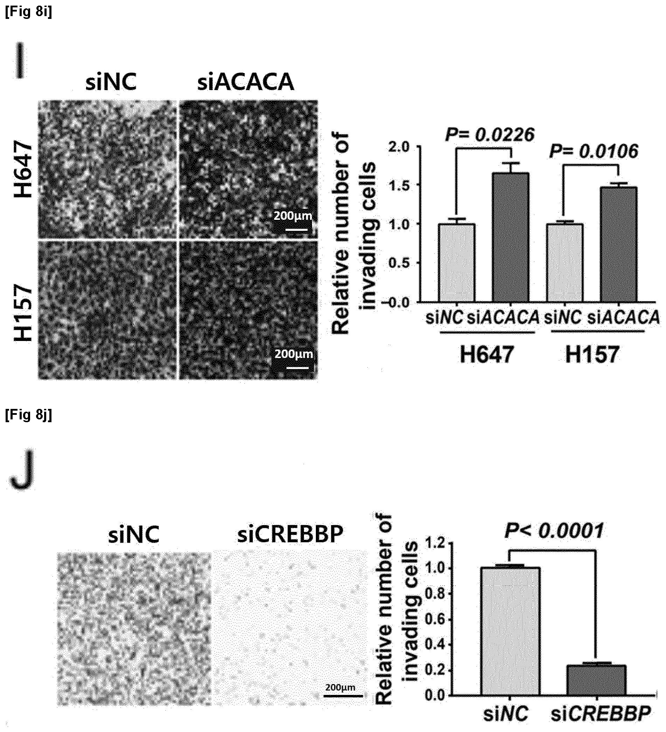

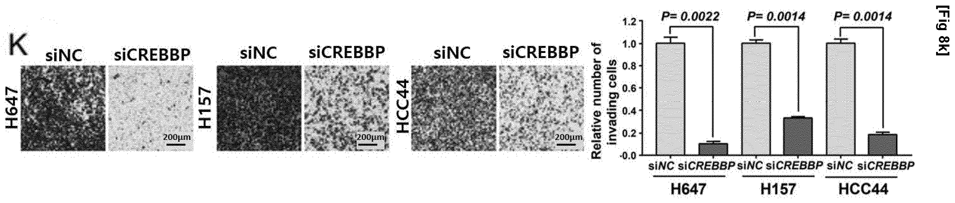

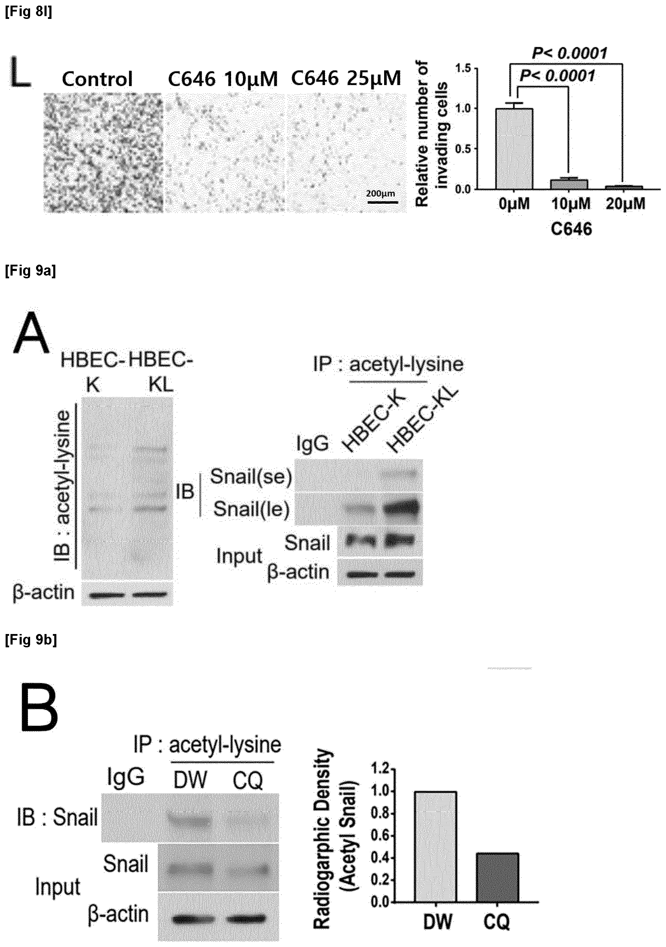

Acetyl-CoA is mainly used for fat synthesis and protein acetylation (FIG. 7H), so to confirm whether acetyl-CoA-mediated fatty acid synthesis regulates cell invasiveness, acetyl-CoA carboxylase 1 (ACC1) was suppressed. However, invasiveness was increased even when ACC1, which increases the expression level of acetyl-CoA in the HBEC-KL cell line and the KL cell line, was inhibited, suggesting that fatty acid synthesis was not the main factor in inducing the invasiveness (Fig. 7O, 8I). On the other hand, upon knockdown of CBP (CREB-binding protein), invasiveness decreased (Fig. 8J, 8K), and chemical inhibition of CBP acetyltransferase also reduced the effect of acetate on invasiveness (Fig. 8L, 7P). From this, it was found that activation of autophagy generates acetyl-CoA precursors, and invasiveness is increased in KRAS/LKB1 co-mutant lung cancer cells through acetylation of target proteins.

### Increased invasiveness through CBP-mediated Snail acetylation of autophagy-induced acetvl-CoA in KL cells

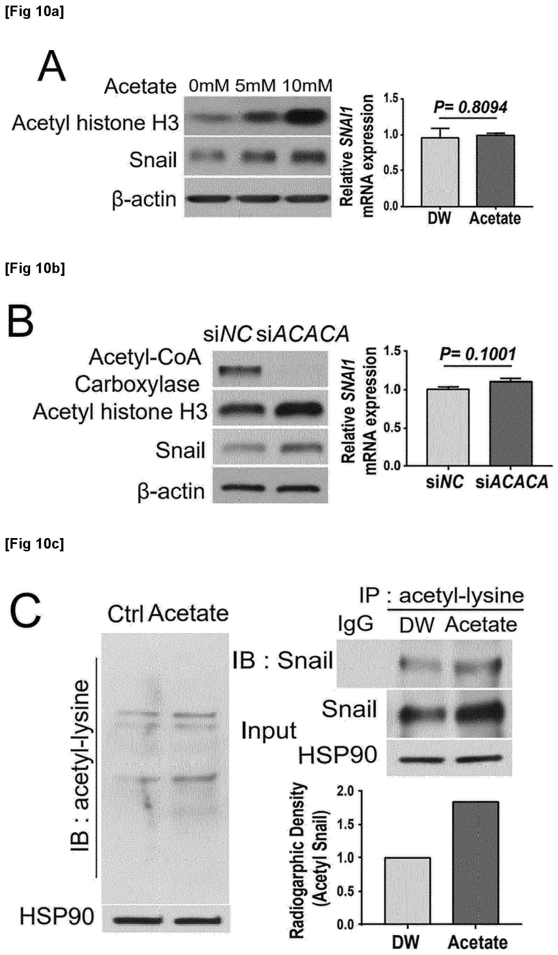

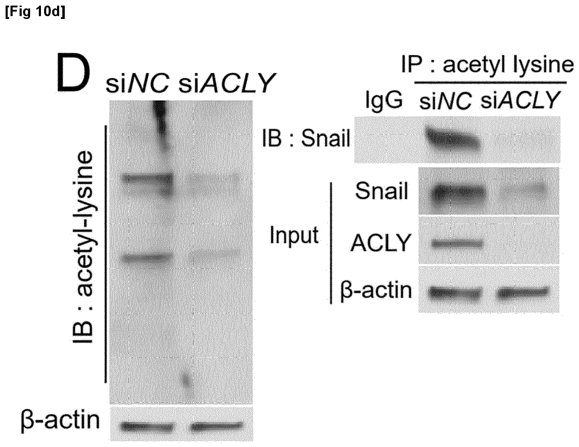

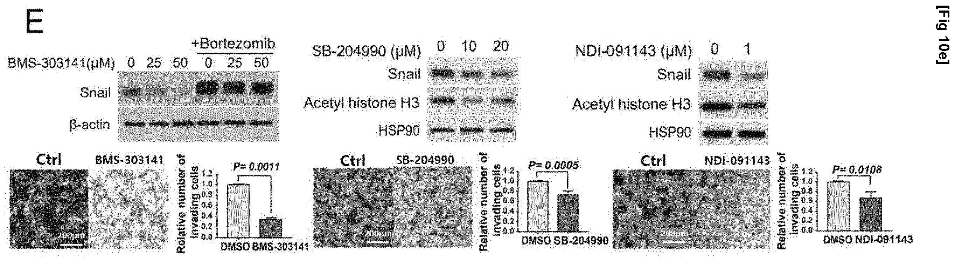

The expression level of Snail in KL cancer cells showed a high correlation with cell invasiveness (FIGS. 1D and 1E), and Snail expression level also changed due to changes in the citrate pathway (7F, 7M). To confirm whether acetyl-CoA-induced invasiveness is regulated by Snail, changes in Snail expression level according to changes in acetyl-CoA abundance were confirmed. Acetate supplementation and ACACA genetic knockdown had no effect on Snail mRNA expression levels, but increased protein expression levels (FIG. 10A, 10B). In addition, the level of acetylated Snail was also increased upon supplementation with acetate (FIG. 10C), whereas the level of protein acetylation and acetylated Snail were decreased upon genetic knockdown of ACLY, an enzyme that converts citrate to acetyl-CoA (Fig. 10D). In addition, Snail and Matrigel invasion were reduced when treated with the ACLY inhibitors BMS303141, NDI-091143 and SB-204990, but when treated with the proteasome inhibitor Bortezomib, the Snail reduction caused by the ACLY inhibitor was restored. (FIG. 10E). Through this, it was found that acetyl-CoA induces Snail acetylation and thereby increases stability by inhibiting ubiquitination.

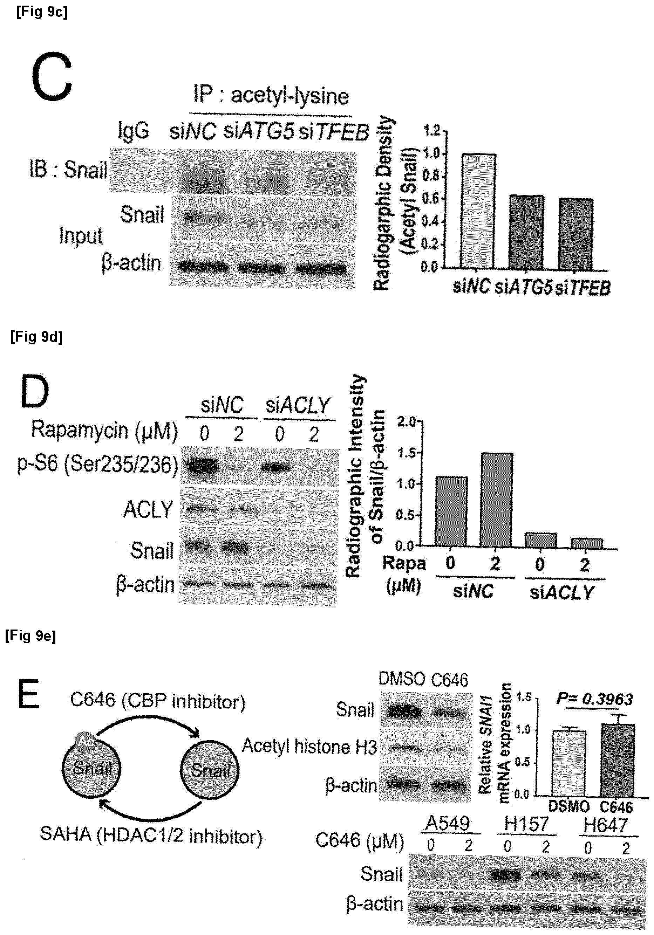

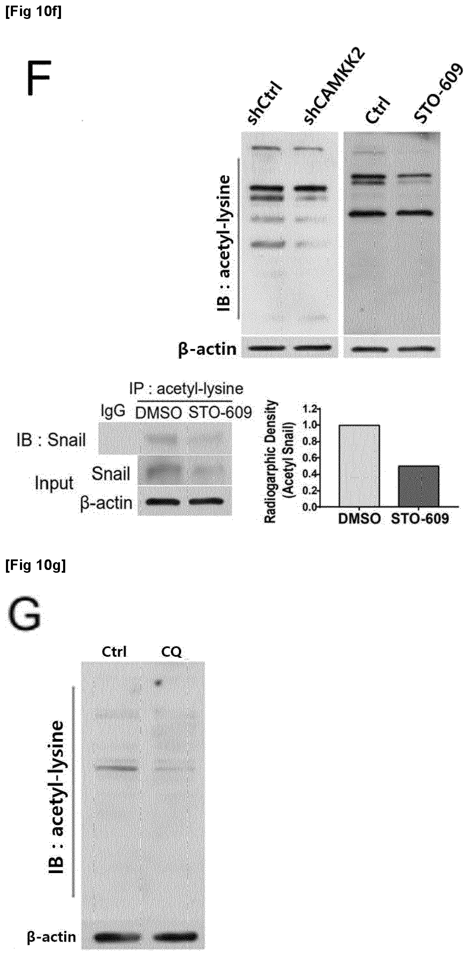

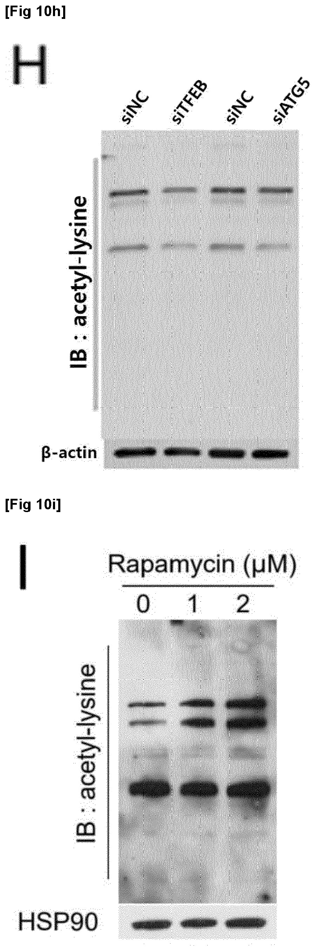

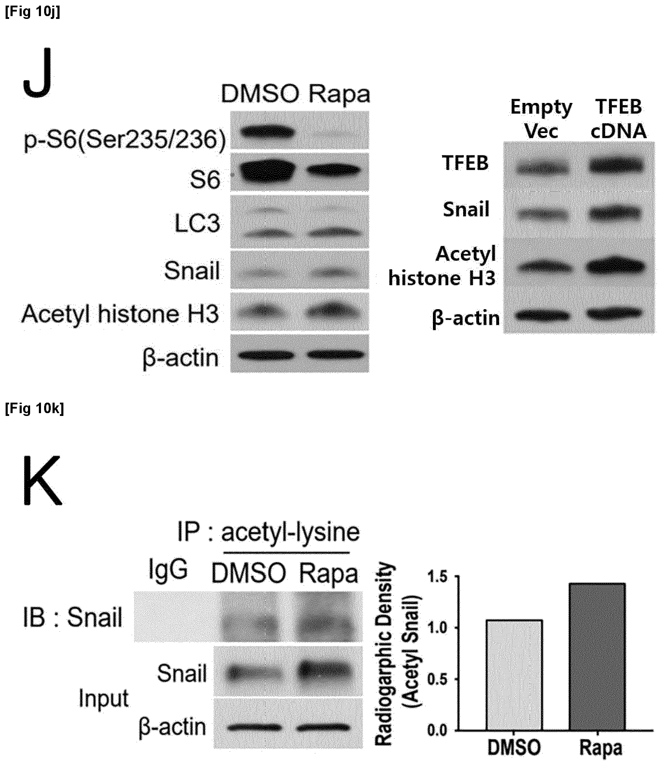

Consistent with these results, protein acetylation was increased in the HBEC-KL cell line compared to the HBEC-K cell line, and the level of acetylated Snail was also increased (FIG. 9A). Since autophagy activated by acetyl-CoA supply functions in KL cell lines, genetic and chemical inhibition of CAMKK2, an upstream regulator, reduced overall protein acetylation as well as acetylated Snail levels (FIG.10F). Likewise, inhibition of autophagy by chloroquine (CQ) or siA TG5/TFEB showed the same effect (FIG. 10G, 10H, 9B, 9C). On the other hand, upon activation of autophagy by mTOR inhibition and/or locomotor TFEB expression in HBEC-K cells, representative proteins in response to protein acetylation, acetylated histone H3, nuclear-cytosolic acetyl-CoA levels, and levels of acetyl Snail all increased (FIG. 10I, 10J, 10K). In addition, no increase in Snail due to autophagy activation was observed in ACLY-depleted KL cells (Fig. 9D). From this, it was found that activated autophagy-induced acetyl-CoA increases Snail acetylation.

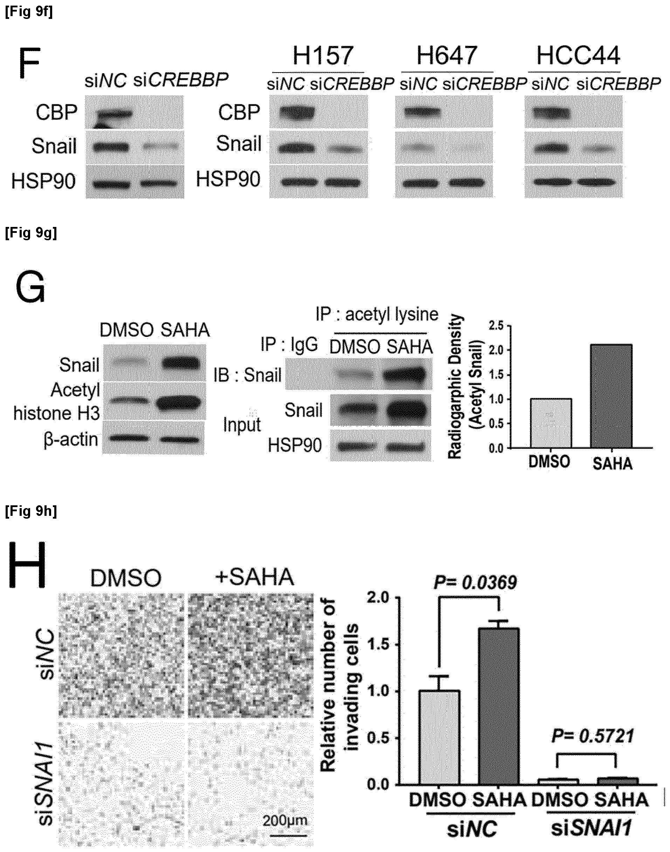

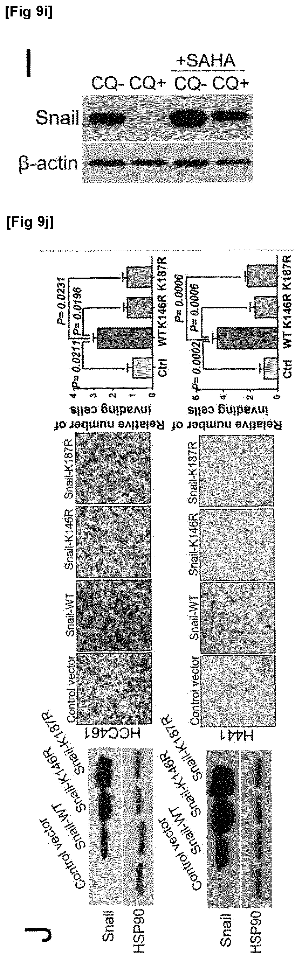

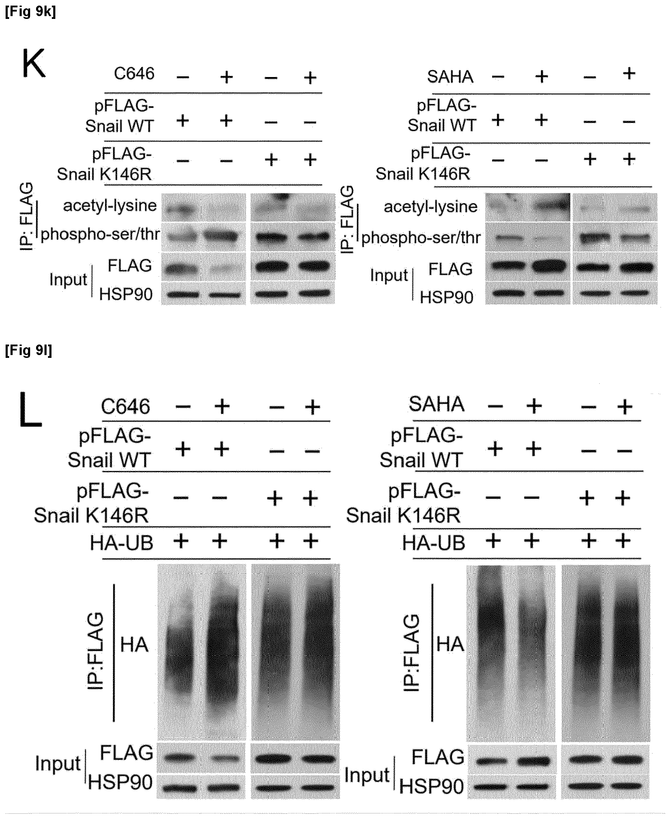

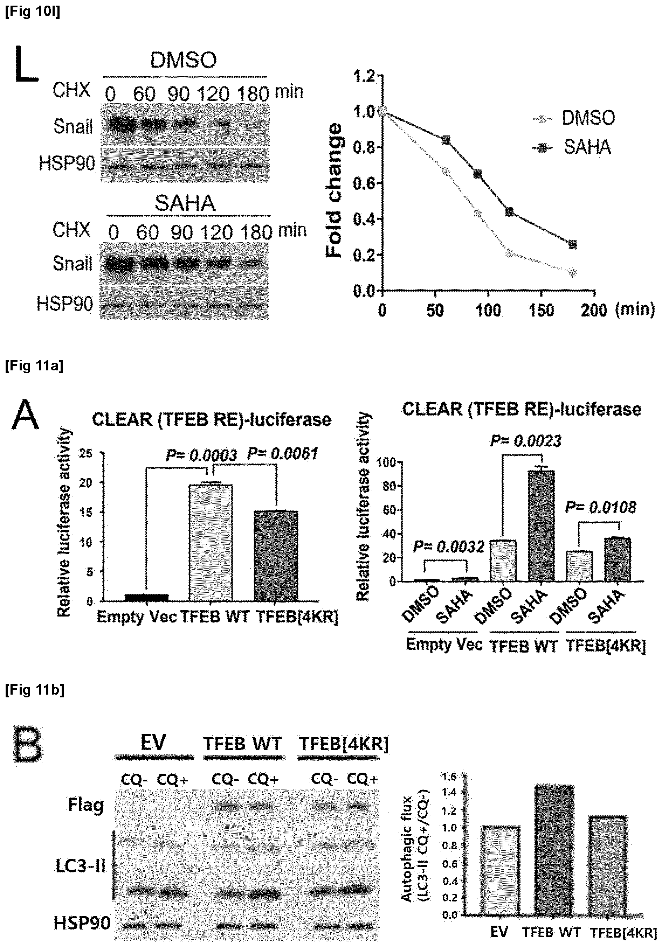

Since acetyltransferase CBP acetylates Snail using acetyl-CoA, and histone deacetylases (HDAC) class 1,2 deacetylate Snail, Snail expression levels were reduced upon siRNA-mediated CBP (siCREBBP) knockdown in HBEC-KL cell lines and KL cell lines (Fig. 9E, 9F), which is due to reduced stability of some proteins, as no change was observed at the mRNA level (FIG. 9E). On the other hand, when treated with SAHA (vorinostat), an HDAC inhibitor, the level of acetylated Snail increased and the degree of matrigel-infiltration increased (Fig. 9G), but the opposite pattern was shown upon knockdown of SNAI1 (Fig. 9H). From this, it was found that the increase in cell invasion by HDAC inhibitors was dependent on Snail. It can be seen that at least part of the SAHA-induced Snail stabilization was regulated by Snail-acetylation rather than protein synthesis (Fig. 10L). In addition, Snail reduction by chloroquine (CQ) was restored upon SAHA treatment, confirming that autophagy induces Snail acetylation (FIG. 9I). There are two evolutionarily conserved lysine residues (K146, K187) that are acetylated in Snail, and it was confirmed that invasiveness was significantly reduced in these acetylation-deficient Snails (Snail-K146R and Snail-K187R) and that Snail acetylation was required for the increase in invasiveness of KL cell lines (Fig. 9J). Upon deacetylation of wild-type Snail through the CBP inhibitor C646, it was confirmed that phosphorylation increased consistently with K48 polyubiquitination (Fig. 9K left). On the other hand, acetylation of wild-type Snail by SAHA was confirmed to reduce phosphorylation consistently with reduced K48 polyubiquitination (Fig. 9L left). However, both C646 and SAHA did not affect the phosphorylation and acetylation of K146R Snail (Fig. 9K right, Fig. 9L right).

From this, it was found that autophagy-induced acetyl CoA increases invasiveness of KL cells through CBP-mediated Snail acetylation.

### Promotion of a positive feedback loop for autophagy activation due to increased TFEB acetylation

In general, since the mammalian target of rapamycin complex 1 (mTORC1) activated by LKB1 loss is known to inhibit CLEAR activity through TFEB phosphorylation, inhibition of CLEAR activity in KL cell lines could not be predicted. Therefore, it was hypothesized that TFEB acetylation would be involved in CLEAR activation in KL cell lines. To confirm the effect of acetylation of TFEB on transcriptional regulatory activity, WT TFEB and acetylation-deficient TFEB [4KR] were overexpressed and CLEAR activity was measured. In fact, in the case of the acetylation-deficient TFEB [4KR] mutant in the HBEC-KL cell line, CLEAR activity was significantly reduced (Fig. 11A left). In addition, it was found that the TFEB[4KR] mutant was less reactive than the SAHA treatment (Fig. 11A right). Consistently, the TFEB[4KR] mutant showed reduced autophagy activity compared to the wild type (FIG. 11B). From this, it was found that acetylation activates TFEB function.

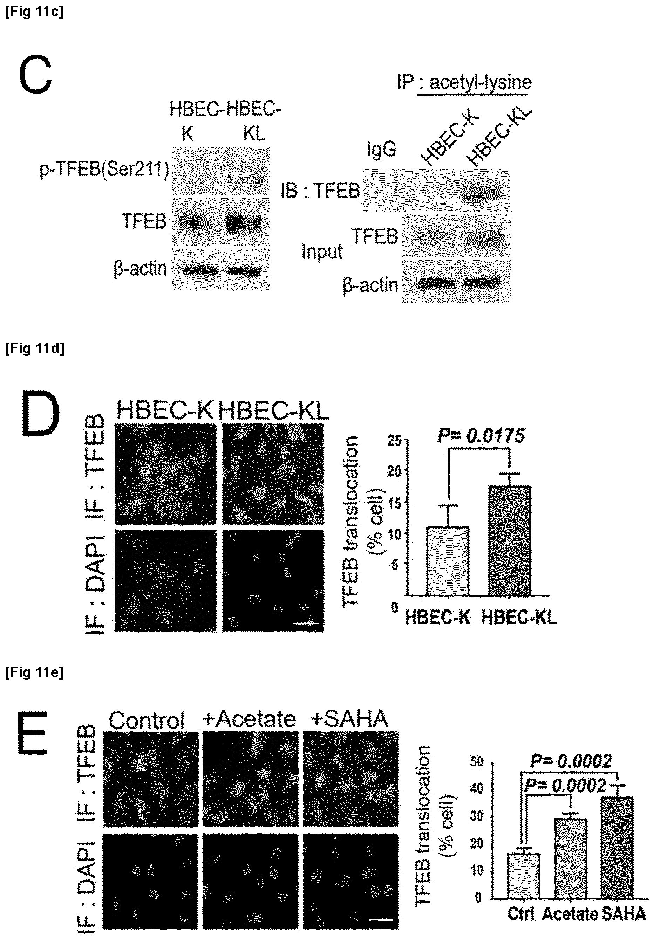

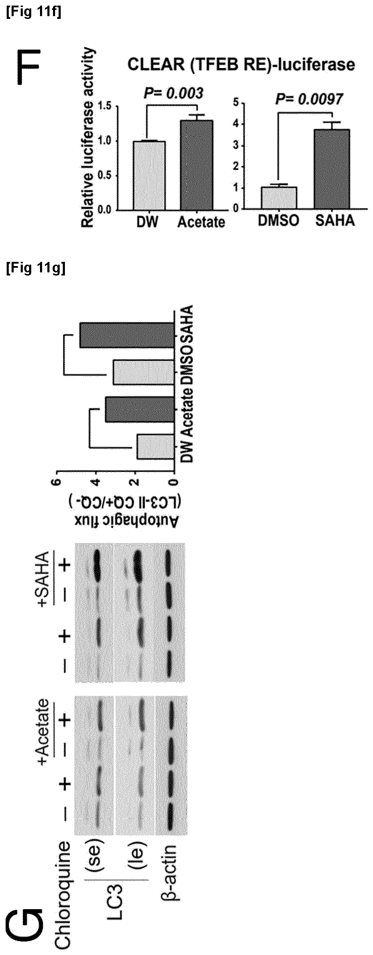

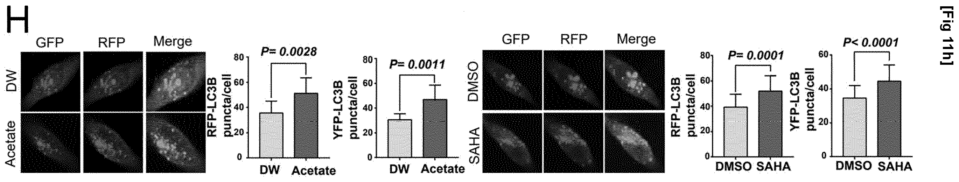

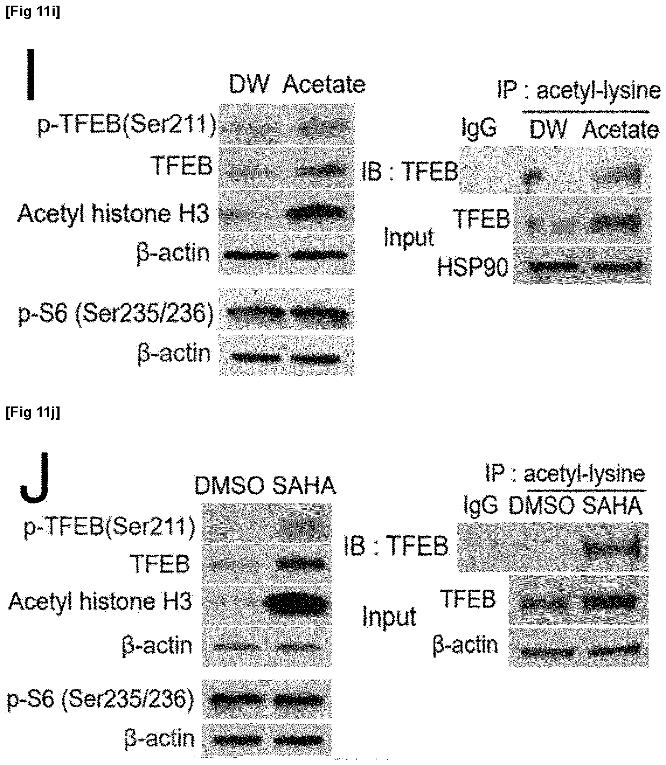

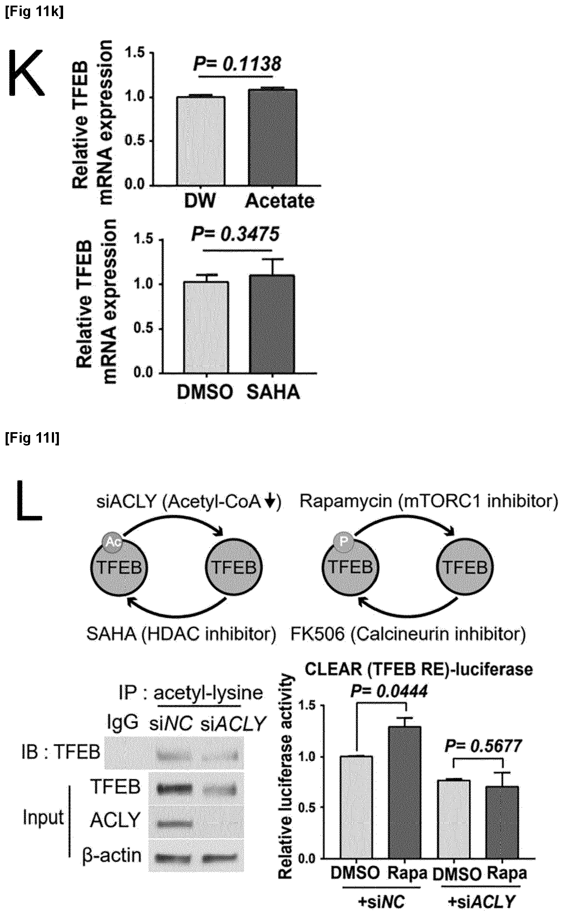

Based on the fact that overall protein acetylation was increased in the HBEC-KL cell line (FIG. 9A), it was hypothesized that acetyl-CoA enrichment acetylates TFEB to increase the autophagy quencher system. In the HBEC-KL cell line, high TFEB phosphorylation was observed at serine position 211, but the level of acetylated TFEB was higher in the HBEC-KL cell line than in the HBEC-K cell line (FIG. 11C). To confirm the seemingly conflicting results of TFEB modification, TFEB localization was compared in the HBEC-K and HBEC-KL cell lines. As a result, consistent with the increased CLEAR activity in the HBEC-KL cell line (FIG. 5C), a high level of TFEB localized in the nucleus was confirmed (FIG. 11D). Consistent with these results, acetate and SAHA increased the level of nuclear-localized TFEB, CLEAR activity and autophagy flux (FIGS. 11E, 11F, 11G, 11H). On the other hand, TFEB acetylated by acetate and SAHA, phosphorylated TFEB, and total TFEB protein levels increased, but there was no significant change in mRNA level (Fig. 11I, 11J, 11K), suggesting that acetyl-TFEB was found to be protected from proteasomal degradation. Since the level of phospho-RPS6 (p-S6) was kept constant even by acetate and SAHA treatment, it was found that the increase in phosphate TFEB was independent of mTORC1 activity (FIGS. 11I and 11J).

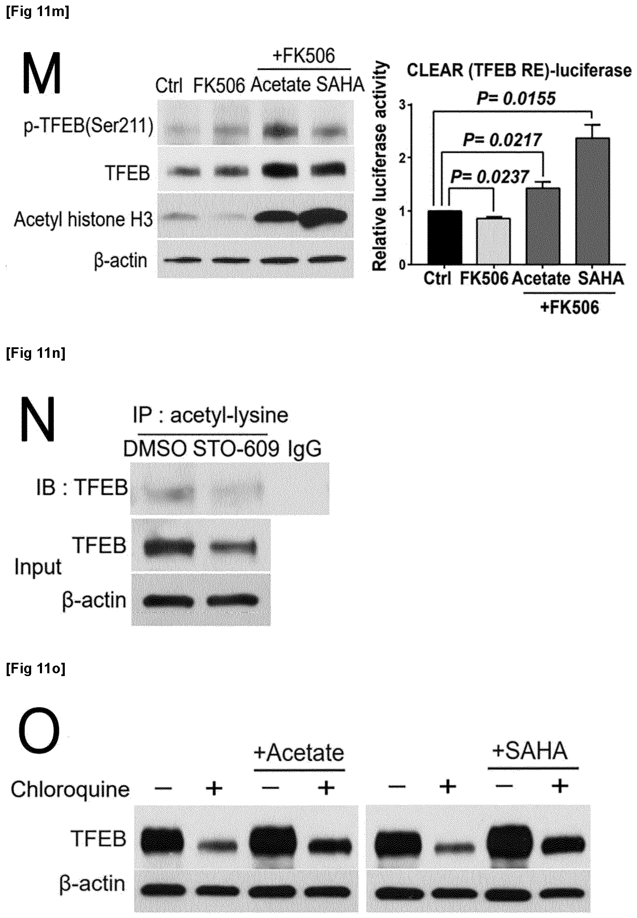

Since TFEB phosphorylation and acetylation occur simultaneously (Fig. 11C, 11L, 11J), each modification was regulated in various ways, and the resulting change in TFEB activity was evaluated. It was confirmed that TFEB acetylated by ACLY genetic knockdown was reduced in the HBEC-KL cell line (Fig. 11L, left). Under these conditions, rapamycin, an mTOR inhibitor that inhibits TFEB phosphorylation, could not restore CLEAR activity (Fig. 11L, right). On the other hand, FK506, a calcineurin inhibitor, decreased CLEAR activity by increasing phospho-TFEB (Fig. 11M, left). When acetate and SAHA were treated under these conditions, it was confirmed that the effect of FK506 was overcome and CLEAR activity was increased (Fig. 11M, right). From this it can be seen that TFEB phosphorylation functions primarily by regulating its activity via cytosolic maintenance vs nuclear localization, whereas TFEB acetylation functions by stabilizing TFEB from proteasomal degradation by simultaneously increasing TFEB in an activated form located in the nucleus and an inactivated form located in the cytosol. Although the former is more dominant for TFEB activity, increased TFEB activation can activate autophagy/lysosomes under conditions of increased TFEB phosphorylation.

Next, it was confirmed whether inhibition of autophagy damages the positive feedback loop. As shown in FIG. 11N, STO-609 effectively reduces acetylated TFEB. On the other hand, acetate and SAHA partially restored the reduced TFEB protein level in the chloroquine (CQ) treatment condition (FIG. 11O). These results were consistent with the involvement of TFEB acetylation in maintaining total TFEB protein activity. From these results, it was found that autophagy-derived acetyl-CoA is due to autophagy activation through TFEB activation.

### Increased invasiveness related to autophagy/acetyl-CoA/acetyl-Snail axis in KRAS mutant pancreatic cancer cells

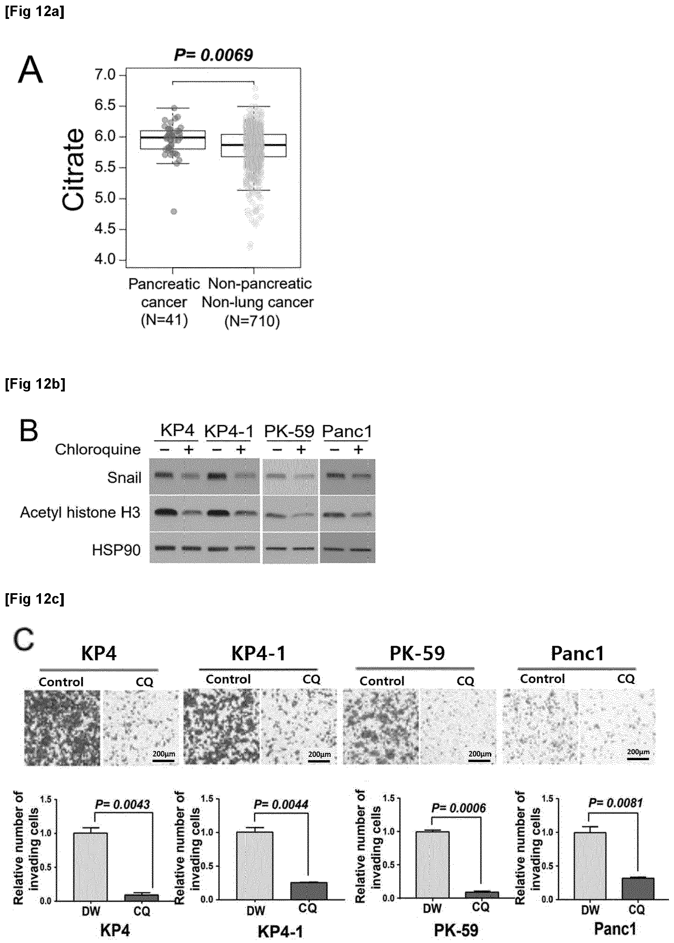

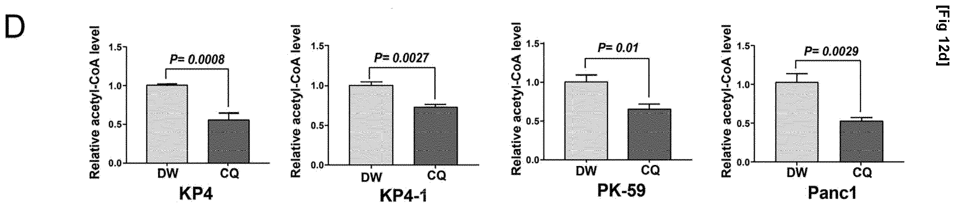

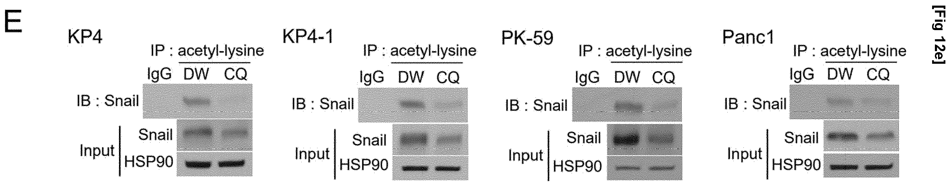

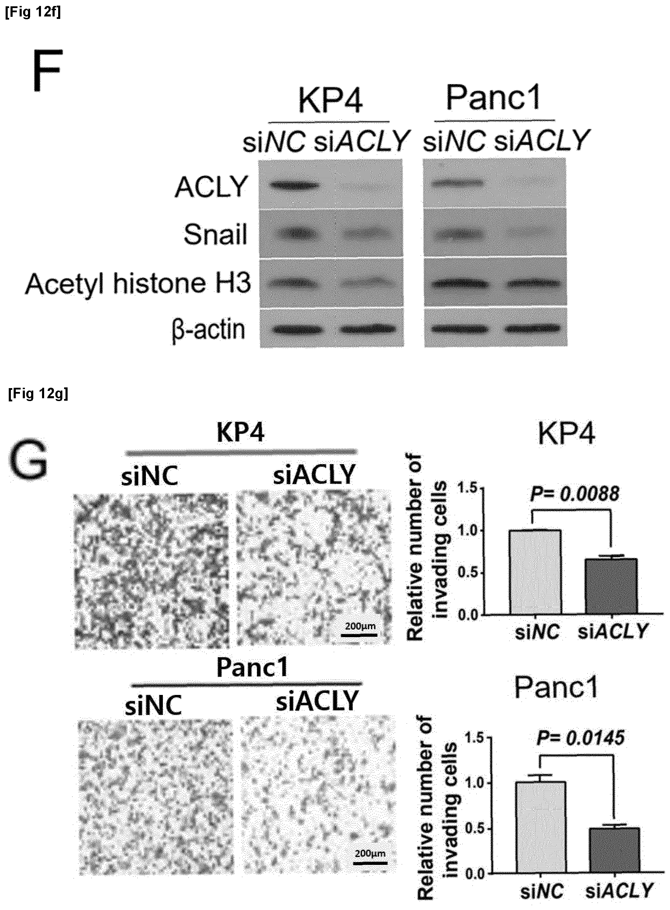

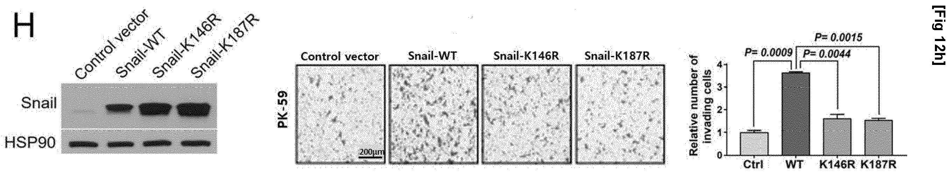

KRAS-mutant pancreatic ductal carcinoma is characterized by increased dependence on autophagy and micropinocytosis, so the following experiment was performed to confirm that the same logic in the previous experiment can be applied to KRAS-mutant pancreatic cancer cell lines physiologically similar to KL cells. As a result of analyzing CCLE metabolomic data, it was confirmed that citrate was expressed at a higher level in pancreatic cancer cell lines compared to other cancer cells (FIG. 12A). As a result of confirming the autophagy/acetyl-CoA/acetyl-Snail axis in KRAS-mutant pancreatic cancer cells, acetyl-CoA, acetylated Snail, and 2D Matrigel transwell invasion were reduced when autophagy was inhibited by chloroquine (CQ) (FIG. 12B, 12C, 12D, 12E). In addition, it was confirmed that the Snail expression level and invasiveness decreased even during ACLY genetic knockdown (FIGS. 12F and 12G). In addition, as in the KL cell line, it was confirmed that acetylation-deficient Snails (Snail-K146R and Snail-K187R) in pancreatic cancer cells had reduced invasiveness compared to the control group (FIGS. 12H and 12I). That is, in both KL cells and KRAS-mutant pancreatic cancer cells, it was found that acetyl-CoA corresponds to a direct downstream major metabolite for enhancing invasiveness.

### Validation of augophagy/acetyl-CoA/acetyl-Snail axis via tissue microarray and metastatic inhibition of KL cells with pharmaceutical inhibitors in vivo

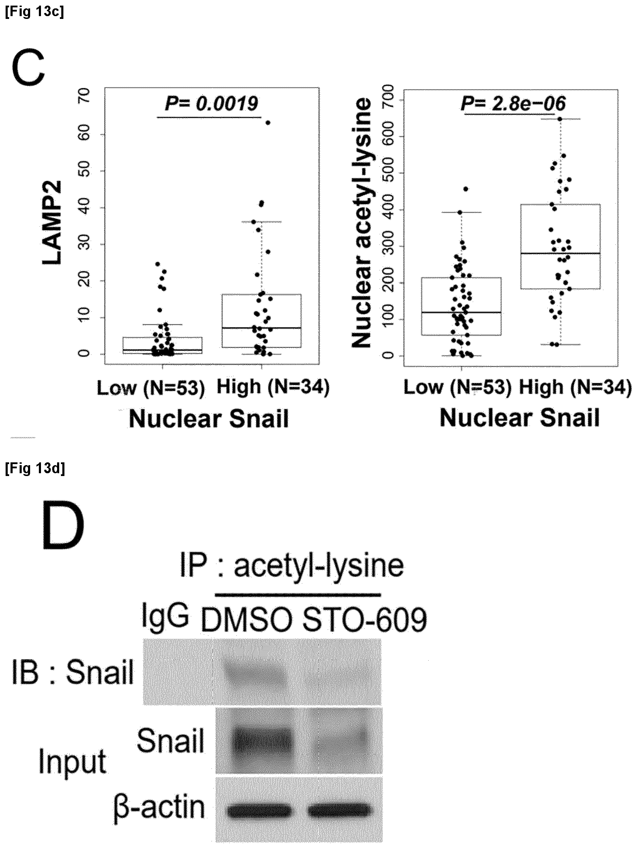

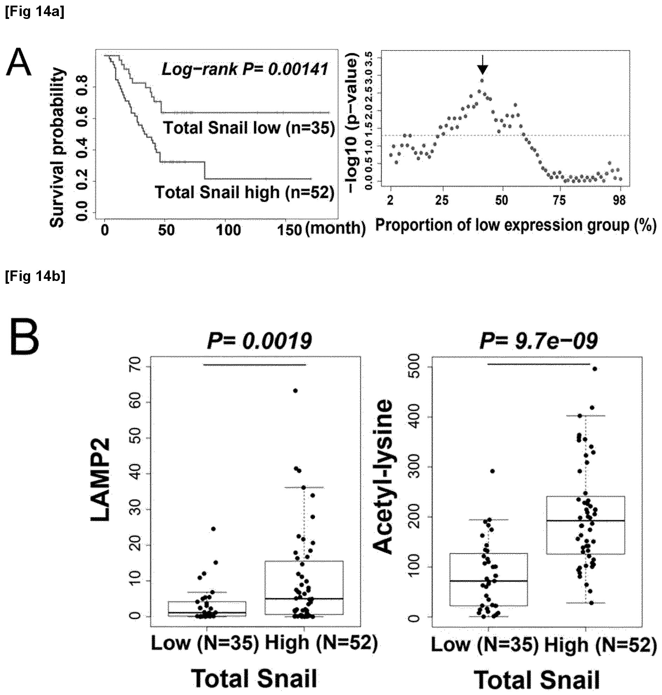

The autophagy/acetyl-CoA/acetyl-Snail axis was clinically verified through survival data of lung cancer patients and tumor tissue microarray (TMA). Total snail and nuclear snail intensity were highly associated with poor prognosis (short overall survival) in the TMA cohort (FIGS. 13A, 13B, 14A). Moreover, nuclear acetyl-lysine (a marker of nuclear acetyl-CoA), total acetyl-lysine (a marker of intracellular acetyl-CoA), and LAMP2 (a marker of autophagolysosomes) were all very high in tumor tissues with high total snail and nuclear snail intensity (FIG. 13C, 14B), suggesting that autophagy/acetyl-CoA/acetyl-Snail axis was intact in human lung cancer tissue.

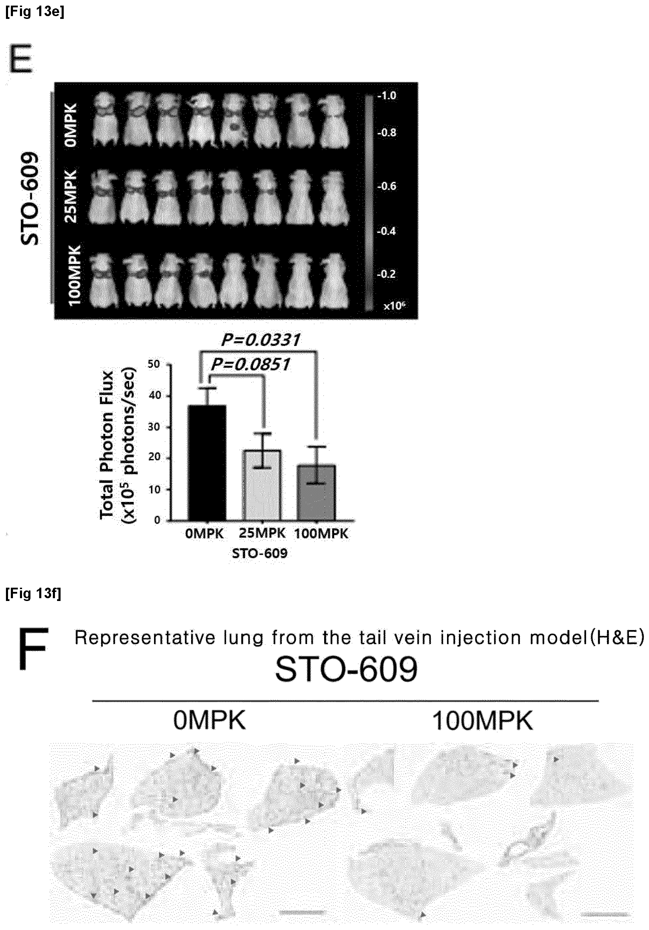

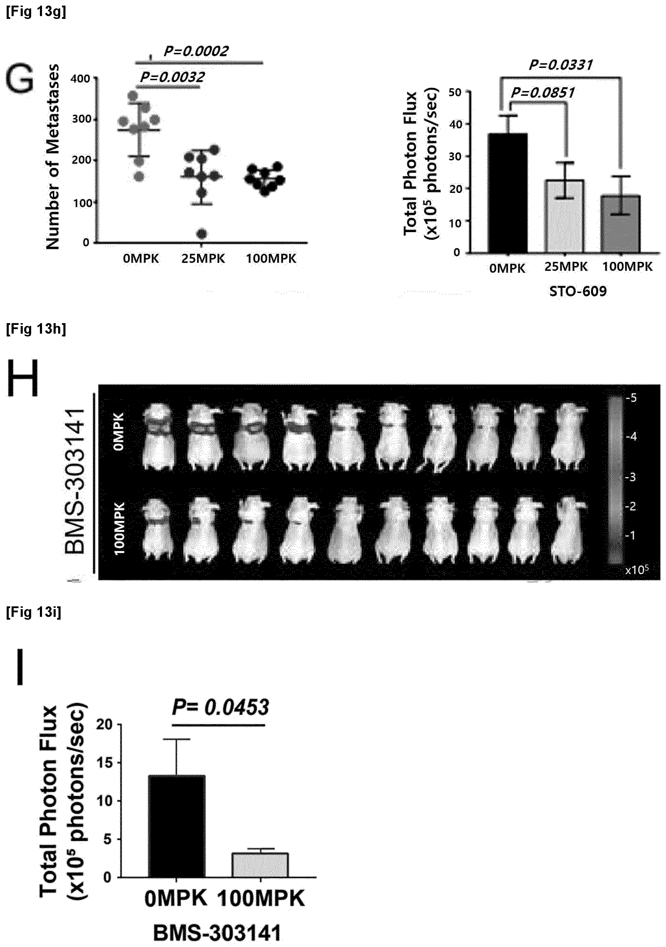

To confirm the pre-metastatic role of autophagy/acetyl-CoA/acetyl-Snail *in vivo,* a mouse model in which lung metastasis was induced by injecting A549-expressing luciferase through the mouse tail vein was used. STO-609 reduced acetylated Snail in A549 luciferase cell line *in vitro* (Fig. 13D), and also *in vivo* as it was confirmed that the total metastasis burden and total number of lung metastases were reduced in the STO-609 treatment group (25 mg/kg, 100 mg/kg, qd, p.o. 28 consecutive days) compared to the vehicle-treated group. (FIG. 13E, 13F, 13G). As a result of treatment with the ACLY inhibitor BMS303141 (100 mg/kg, qd p.o 20 consecutive days), the total degree of metastasis was reduced (Fig. 13H, 13I, 13J), suggesting that inhibition of the autophagy/acetyl-CoA axis exerts an anti-metastatic effect *in vivo.*

Through this, it was found that autophagy-induced acetyl-CoA and Snail acetylation acts as the main mechanism of metastasis and malignancy not only in KL lung cancer but also in other types of carcinomas in which autophagy is activated, such as pancreatic cancer. Furthermore, the therapeutic effect of various metastatic cancers can be expected through pharmacological inhibition of this axis.

Although the present invention has been described in detail above, it will be obvious to those skilled in the art that these specific techniques are only preferred embodiments, and the scope of the present invention is not limited thereto. Accordingly, the substantial scope of the present invention will be defined by the appended claims and equivalents thereof.

## Claims

1. A pharmaceutical composition for inhibiting metastasis of cancer comprising, as an active ingredient, an inhibitor of the activity or expression of at least one protein selected from calcium/calmodulin-dependent protein kinase kinase 2 (CAMKK2) and ATP citrate lyase (ACLY); or an inhibitor of expression of a gene encoding the protein.

2. The pharmaceutical composition according to claim 1, wherein the inhibitor of the activity or expression of the protein comprises at least one selected from the group consisting of compounds, peptides, peptide mimetics, aptamers, antibodies and natural products that specifically bind to the protein.

3. The pharmaceutical composition according to claim 1, wherein the inhibitor of activity or expression of CAMKK2 protein is at least one selected from the group consisting of STO-609, SGC-CAMKK2-1, KN62, KN93, AlP, ACS-I and berbamine.

4. The pharmaceutical composition according to claim 1, wherein the inhibitor of activity or expression of ACLY protein is at least one selected from the group consisting of BMS303141, NDI-091143, SB-204990, ETC-1002, radicicol, (-)-hydroxycitric acid, and 2-chloro-1,3,8-trihydroxy-6-methylanthrone.

5. The pharmaceutical composition according to claim 1, wherein the inhibitor of gene expression comprises at least one selected from the group consisting of antisense nucleotides, short interfering RNA (siRNA), short hairpin RNA (shRNA) and ribozyme that complementarily bind to the gene.

6. The pharmaceutical composition according to claim 1, wherein the cancer is in which autophagy is activated.

7. The pharmaceutical composition according to claim 6, wherein the cancer comprises a mutation in at least one of KRAS and LKB1.

8. The pharmaceutical composition according to claim 1, wherein the cancer is breast cancer, ovarian cancer, colon cancer, stomach cancer, liver cancer, pancreatic cancer, cervical cancer, thyroid cancer, parathyroid cancer, lung cancer, non-small cell lung cancer, prostate cancer, gallbladder cancer, biliary tract cancer, non-Hodgkin's lymphoma, Hodgkin's lymphoma, blood cancer, bladder cancer, kidney cancer, melanoma, colon cancer, bone cancer, skin cancer, head cancer, uterine cancer, rectal cancer, brain tumor, perianal cancer, fallopian tube carcinoma, endometrial carcinoma, vaginal cancer, vulvar carcinoma, esophageal cancer, small intestine cancer, endocrine adenocarcinoma, adrenal cancer, soft tissue sarcoma, urethral cancer, penile cancer, ureteral cancer, renal cell carcinoma, renal pelvic carcinoma, central nervous system (CNS) tumor, primary CNS lymphoma, spinal cord tumor, brainstem glioma or pituitary adenoma.

9. A method for screening inhibitors for cancer metastasis comprising treating a candidate substance to the separated biological sample; and
measuring the activity or expression level of at least one protein of calcium/calmodulin-dependent protein kinase kinase 2 (CAMKK2) and ATP citrate lyase (ACLY) or measuring the expression level of a gene encoding the protein in the biological sample after treating with the candidate substance.

10. The method for screening according to claim 9, wherein the biological sample comprises whole blood, leukocytes, peripheral blood mononuclear cells, buffy coat, plasma, serum, sputum, tears, mucus, nasal washes, nasal aspirate, breath, urine, semen, saliva, peritoneal washings, pelvic fluids, cystic fluid, meningeal fluid, amniotic fluid, glandular fluid, pancreatic fluid, lymph fluid, pleural fluid, nipple aspirate, bronchial aspirate, synovial fluid, joint aspirate, organ secretions, cells, cell extracts or cerebrospinal fluids.

11. The method for screening according to claim 9, wherein the candidate substance is at least one selected from the group consisting of natural compounds, synthetic compounds, RNA, DNA, polypeptides, enzymes, proteins, ligands, antibodies, antigens, bacterial or fungal metabolites and bioactive molecules.

12. The method for screening according to claim 9, wherein the agent for measuring the activity or expression level of the protein comprises at least one selected from the group consisting of antibodies, oligopeptides, ligands, peptide nucleic acids (PNAs) and aptamers that specifically bind to the protein.

13. The method for screening according to claim 9, wherein the agent for measuring the expression level of a gene comprises at least one selected from the group consisting of primers, probes and antisense nucleotides that specifically bind to the gene.

14. The method for screening according to claim 9, wherein the method of measuring the activity or expression level of a protein is protein chip analysis, immunoassay, ligand binding assay, MALDI-TOF (Matrix Assisted Laser Desorption/lonization Time of Flight Mass Spectrometry) analysis, SELDI-TOF (Surface Enhanced Laser Desorption/lonization Time of Flight Mass Spectrometry) analysis, radiation immunity assay, radioimmuno-diffusion method, Oukteroni immunodiffusion method, rocket immunoelectrophoresis, tissue immunostaining, complement fixation assay, two-dimensional electrophoretic assay, Liquid Chromatography-Mass Spectrometry (LC-MS), Liquid Chromatography-Mass Spectrometry/ Mass Spectrometry (LC-MS/MS), Western blotting or ELISA (enzyme linked immunosorbentassay).

15. The method for screening according to claim 9, wherein the analytical method for measuring gene expression levels is reverse transcription polymerase reaction (RT-PCR), competitive reverse transcription polymerase reaction (Competitive RT-PCR), real-time reverse transcription polymerase reaction (Real-time RT-PCR), RNase protection assay (RPA), Northern blotting or DNA chip.

16. The method for screening according to claim 9, wherein determining the candidate substance as a cancer metastasis inhibitor further comprises determining the candidate substance as a cancer metastasis inhibitor when the activity or expression level of at least one of CAMKK2 and ACLY measured in the biological sample after treating with the candidate substance is reduced, or the expression level of a gene encoding the protein is reduced.

17. The method for screening according to claim 9, wherein the cancer is in which autophagy is activated.

18. The method for screening according to claim 9, wherein the cancer comprises a mutation in at least one of KRAS and LKB1.

19. The method for screening according to claim 9, wherein the cancer is breast cancer, ovarian cancer, colon cancer, stomach cancer, liver cancer, pancreatic cancer, cervical cancer, thyroid cancer, parathyroid cancer, lung cancer, non-small cell lung cancer, prostate cancer, gallbladder cancer, biliary tract cancer, non-Hodgkin's lymphoma, Hodgkin's lymphoma, blood cancer, bladder cancer, kidney cancer, melanoma, colon cancer, bone cancer, skin cancer, head cancer, uterine cancer, rectal cancer, brain tumor, perianal cancer, fallopian tube carcinoma, endometrial carcinoma, vaginal cancer, vulvar carcinoma, esophageal cancer, small intestine cancer, endocrine adenocarcinoma, adrenal cancer, soft tissue sarcoma, urethral cancer, penile cancer, ureteral cancer, renal cell carcinoma, renal pelvic carcinoma, central nervous system (CNS) tumor, primary CNS lymphoma, spinal cord tumor, brainstem glioma or pituitary adenoma.

20. A method for providing information for predicting the therapeutic response of a metastasis inhibitor of cancer comprising detecting the presence or absence of a mutation in at least one gene of KRAS and LKB1 in a biological sample isolated from a target subject having cancer or having a high possibility of developing cancer,
wherein the metastasis inhibitor of cancer is an inhibitor of the activity or expression of Calcium/calmodulin-dependent protein kinase kinase 2 (CAMKK2) and ATP citrate lyase (ACLY) proteins, or an inhibitor of the expression of genes encoding the proteins.

21. The method for providing information according to claim 20, wherein the method for detecting the presence of mutations in genes comprises at least one selected from the group consisting of reverse transcription polymerase reaction (RT-PCR), competitive reverse transcription polymerase reaction (Competitive RT-PCR), real-time reverse transcription polymerase reaction (Real-time RT-PCR), RNase protection assay (RPA), Northern blotting, DNA chip and RNA sequencing.

22. The method for providing information according to claim 20, wherein the agent for detecting the presence or absence of mutations in genes comprises at least one selected from the group consisting of primers, probes and antisense nucleotides that specifically bind to the gene.

23. The method for providing information according to claim 20, wherein the method further comprises detecting the presence or degree of acetylation of Snail protein in the biological sample.

24. The method for providing information according to claim 23, wherein it is predicted that the therapeutic response of the cancer metastasis inhibitor is high when a mutation is present in at least one gene of KRAS and LKB1 in the biological sample and acetylation of Snail protein is increased compared to the control group.

25. The method for providing information according to claim 20, wherein the cancer is in which autophagy is activated.

26. The method for providing information according to claim 20, wherein the cancer is breast cancer, ovarian cancer, colon cancer, stomach cancer, liver cancer, pancreatic cancer, cervical cancer, thyroid cancer, parathyroid cancer, lung cancer, non-small cell lung cancer, prostate cancer, gallbladder cancer, biliary tract cancer, non-Hodgkin's lymphoma, Hodgkin's lymphoma, blood cancer, bladder cancer, kidney cancer, melanoma, colon cancer, bone cancer, skin cancer, head cancer, uterine cancer, rectal cancer, brain tumor, perianal cancer, fallopian tube carcinoma, endometrial carcinoma, vaginal cancer, vulvar carcinoma, esophageal cancer, small intestine cancer, endocrine adenocarcinoma, adrenal cancer, soft tissue sarcoma, urethral cancer, penile cancer, ureteral cancer, renal cell carcinoma, renal pelvic carcinoma, central nervous system (CNS) tumor, primary CNS lymphoma, spinal cord tumor, brainstem glioma or pituitary adenoma.
